# EUROPEAN PATENT SPECIFICATION

(11) **EP 2 064 211 B1**
(45) Date of publication and mention of the grant of the patent: **11.11.2015**
(21) Application number: 06784289.8
(22) Date of filing: 20.09.2006
(51) Int. Cl.: C07D 471/04, A61K 31/437, A61P 35/00

(54) **IMIDAZO[1,2-A]PYRIDINE HYDROXYMATE COMPOUNDS THAT ARE INHIBITORS OF HISTONE DEACETYLASE**
IMIDAZO[1,2-A]PYRIDINHYDROXYMATVERBINDUNGEN, BEI DENEN ES SICH UM INHIBITOREN VON HISTONDEACETYLASE HANDELT
COMPOSÉS D'HYDROXYMATE D'IMIDAZO[1,2-A]PYRIDINE QUI SONT DES INHIBITEURS D'HISTONE DÉSACÉTYLASE

(43) Date of publication of application: 03.06.2009
(73) Proprietor: MEI Pharma, Inc., San Diego, CA 92130 (US)
(72) Inventor: LEE, Ken Chi Lik, Singapore 670627 (SG); SUN, Eric, T., Singapore 269258 (SG); WANG, Haishan, Singapore 126837 (SG)
(74) Representative: Schrell, Andreas
(86) International application number: PCT/SG2006/000277
(87) International publication number: WO 2008/036046

(56) References cited:
- WO-A1-01/38322
- WO-A1-2005/028447
- WO-A1-2006/101455
- US-A1- 2005 137 234
- US-A1- 2005 239 823
- WERMUTH C G: "MOLECULAR VARIATIONS BASED ON ISOSTERIC REPLACEMENTS" PRACTICE OF MEDICINAL CHEMISTRY, XX, XX, 1 January 1996 (1996-01-01), pages 203-237, XP002190259

## Description

### FIELD OF THE INVENTION

The present invention relates to hydroxamate compounds that are inhibitors of histone deacetylase (HDAC). More particularly, the present invention relates to imidazo[1,2-a]pyridine containing compounds and methods for their preparation. These compounds may be useful as medicaments for the treatment of proliferative disorders as well as other diseases involving, relating to or associated with enzymes having histone deacetylase (HDAC) activities.

### BACKGROUND OF THE INVENTION

Local chromatin architecture is generally recognized as an important factor in the regulation of gene expression. The architecture of chromatin, a protein-DNA complex, is strongly influenced by post-translational modifications of the histones which are the protein components. Reversible acetylation of histones is a key component in the regulation of gene expression by altering the accessibility of transcription factors to DNA. In general, increased levels of histone acetylation are associated with increased transcriptional activity, whereas decreased levels of acetylation are associated with repression of gene expression [Wade P.A. Hum. Mol. Genet. 10, 693-698 (2001), De Ruijter A.J.M. et al, Biochem. J., 370, 737-749 (2003)]. In normal cells, histone deacetylases (HDACs) and histone acetyl transferase together control the level of acetylation of histones to maintain a balance. Inhibition of HDACs results in the accumulation of acetylated histones, which results in a variety of cell type dependent cellular responses, such as apoptosis, necrosis, differentiation, cell survival, inhibition of proliferation and cytostasis.

Inhibitors of HDAC have been studied for their therapeutic effects on cancer cells. For example, suberoylanilide hydroxamic acid (SAHA) is a potent inducer of differentiation and/or apoptosis in murine erythroleukemia, bladder, and myeloma cell lines [Richon V.M. et al, Proc. Natl. Acad. Sci. USA, 93: 5705-5708 (1996), Richon V.M. et al, Proc. Natl. Acad. Sci. USA, 95: 3003-3007 (1998)]. SAHA has been shown to suppress the growth of prostate cancer cells *in vitro* and *in vivo* [Butler L.M. et al, Cancer Res. 60, 5165-5170 (2000)]. Other inhibitors of HDAC that have been widely studied for their anti-cancer activities are trichostatin A (TSA) and trapoxin B [Yoshida M. et al, J. Biol. Chem., 265, 17174 (1990), Kijima M. et al, J. Biol. Chem., 268, 22429 (1993)]. Trichostatin A is a reversible inhibitor of mammalian HDAC. Trapoxin B is a cyclic tetrapeptide, which is an irreversible inhibitor of mammalian HDAC. However, due to the in vivo instability of these compounds they are less desirable as anti-cancer drugs. Recently, other small molecule HDAC inhibitors have become available for clinical evaluation [US6,552,065]. Additional HDAC inhibiting compounds have been reported in the literature [Bouchain G. et al, J. Med. Chem., 46, 820-830 (2003)] and patents [WO 03/066579A2, WO 01 /38322 A1]. The in vivo activity of such inhibitors can be directly monitored by their ability to increase the amount of acetylated histones in the biological sample. HDAC inhibitors have been reported to interfere with neurodegenerative processes, for instance, HDAC inhibitors arrest polyglutamine-dependent neurodegeneration [Nature, 413(6857): 739-43, 18 October, 2001]. In addition, HDAC inhibitors have also been known to inhibit production of cytokines such as TNF, IFN, IL-1 which are known to be implicated in inflammatory diseases and/or immune system disorders. [ J. Biol. Chem. 1990; 265(18): 10232-10237; Science, 1998; 281: 1001-1005; Dinarello C.A. and Moldawer L.L. Proinflammatory and anti-inflammatory cytokines in rheumatoid arthritis. A primer for clinicians. 3rd Edition, Amergen Inc., 2002].

WO 2005/028447 A1 discloses benzimidazole compounds bearing an N-hydroxyacrylamide group which are active as histone deacytelase inhibitors. WO 2005/028447 A1 demonstrates that the heteroaryl ring of the benzimidazole moiety can bear various substituents including alkyl and substituted aminoalkyl among a broad range of disclosed substituents.

Nevertheless, there is still a need to provide further HDAC inhibitors that would be expected to have useful, improved pharmaceutical properties in the treatment of diseases such as cancer, neurodegenerative diseases, disorders involving angiogenesis and inflammatory and/or immune system disorders. With a view to meeting this need a number of small organic moiety scaffolds have been investigated including a number of heterocyclic systems. We have now found that judicious selection of the substituents on the 5 membered ring of the imidazo[1,2-a]pyridine ring system leads to the production of a family of compounds with improved pharmacokinetic properties when compared with the compounds of the prior art. We have now found that judicious selection of the substituents on the 5 membered ring of the imidazo[1,2-a]pyridine ring system leads to the production of a family of compounds with improved enzyme and cellular activities when compared with the compounds of the prior art. The compounds within the family also show improved microsomal stability and thereby improved pharmacokinetic properties when compared to the compounds of the prior art.

### SUMMARY OF THE INVENTION

In one aspect the present invention provides a compound of the formula (I): 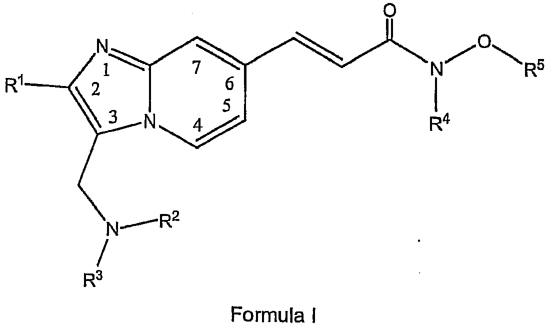 wherein:
R¹ is alkyl;
R² is alkyl;
R³ is alkyl;
wherein if the total number of carbon atoms in R² is X and the total number of carbon atoms in R³ is Y, then R² and R³ are selected such that the sum of X + Y is an integer selected from the group consisting of 5, 6, 7 and 8;
R⁴ is H;
R⁵ is H;
or a pharmaceutically acceptable salt thereof.

A group of compounds, which the group of compounds according to the present invention is based on, can be characterized by the following formula (Ia): 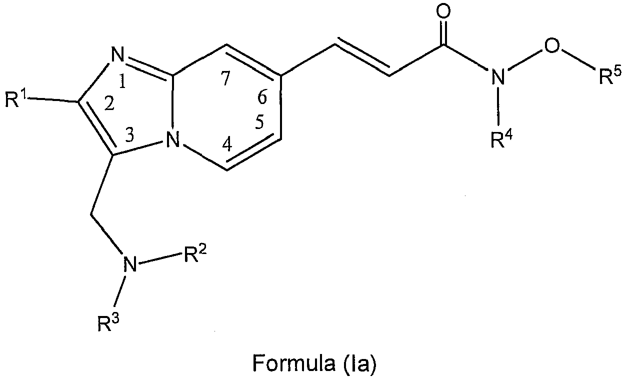 wherein:
R¹ is alkyl which may be optionally substituted with one or more substituents selected from the group consisting of F, Br, I, OH, SH, OCF₃, OR⁶, SR⁶, amino, NR⁷R⁸, cycloalkyl, and NO₂;
R² is H or alkyl which may be optionally substituted with one or more substituents selected from the group consisting of F, Br, I, OH, SH, OCF₃, OR⁶, SR⁶, amino, NR⁷R⁸, cycloalkyl, and NO₂;
R³ is alkyl which may be optionally substituted with one or more substituents selected from the group consisting of F, Br, I, OH, SH, OCF₃, OR⁶, SR⁶, amino, NR⁷R⁸, cycloalkyl, and NO₂;
R⁴ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
R⁵ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁶ is independently selected from the group consisting of: alkyl, cycloalkyl, heterocycloalkyl, aryl and heteroaryl, each of which may be optionally substituted;
each R⁷ is independently selected from the group consisting of: H, alkyl, cycloalkyl, heterocycloalkyl, aryl and heteroaryl, each of which may be optionally substituted;
each R⁸ is independently selected from the group consisting of: H, alkyl, cycloalkyl, heterocycloalkyl, aryl and heteroaryl, each of which may be optionally substituted;
or a pharmaceutically acceptable salt or prodrug thereof.
R² can be H or C₁-C₆ alkyl. Alternatively, R² can be H or C₁-C₄ alkyl. This provides non-inventive compounds of formula (II) and inventive compounds of formula (III). 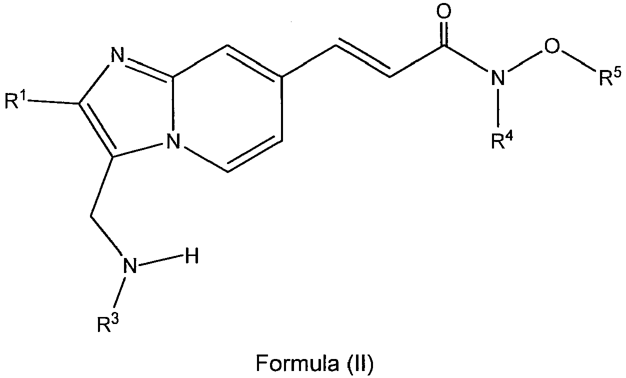
wherein R¹, R³, R⁴ and R⁵ are as defined in formula (Ia) above. 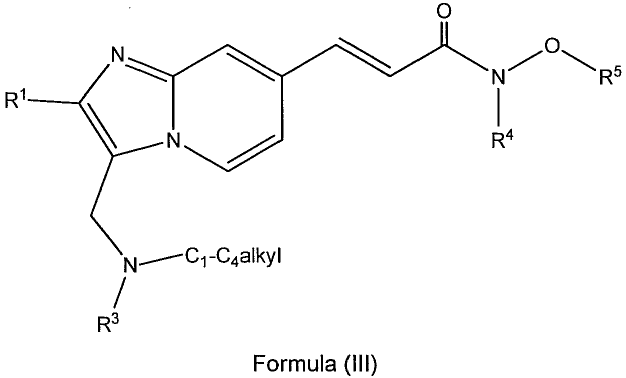
wherein R¹, R³, R⁴ and R⁵ are as defined in formula (I) above.

In one embodiment of the compounds of formula (III) R² is selected from the group consisting of methyl, ethyl, propyl and butyl. This provides compounds of formula (IIIa) - (IIId). 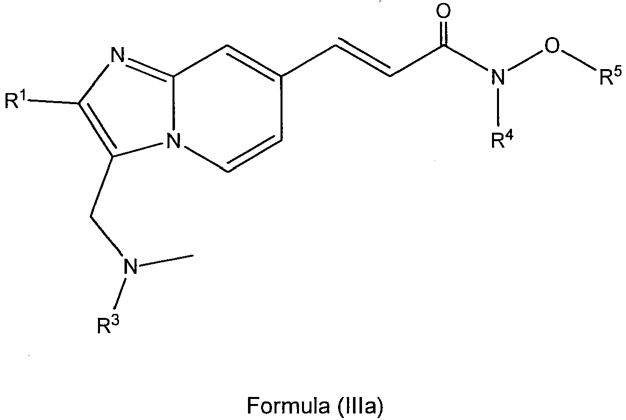 wherein R¹, R³, R⁴ and R⁵ are as defined in formula (I) above. 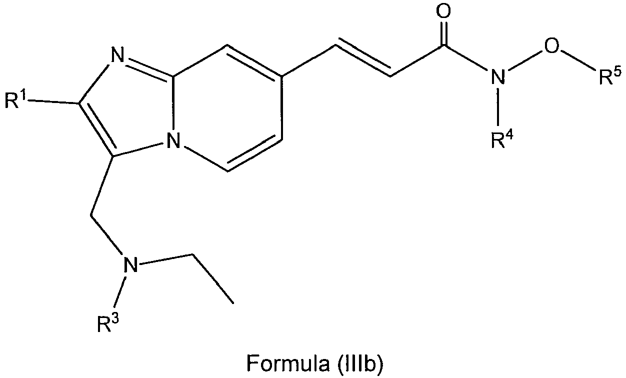 wherein R¹, R³, R⁴ and R⁵ are as defined in formula (I) above. 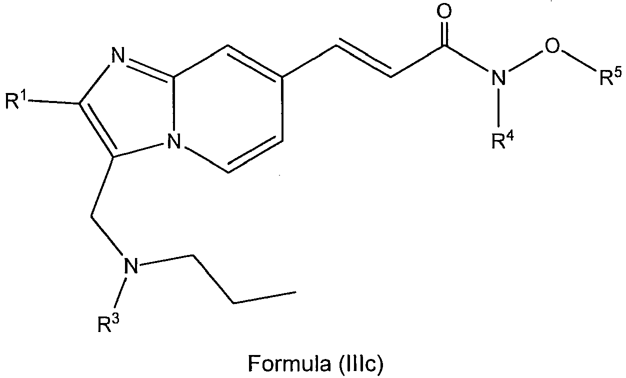 wherein R¹, R³, R⁴ and R⁵ are as defined in formula (I) above. 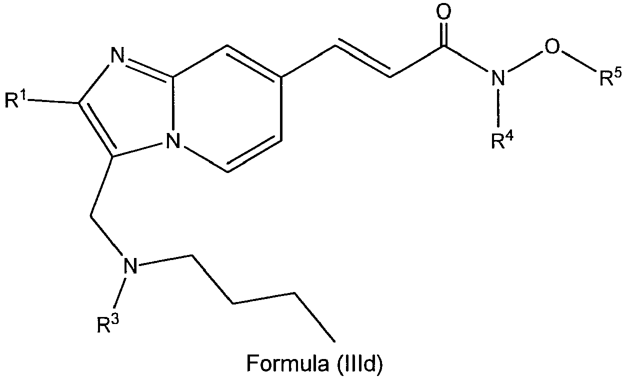 wherein R¹, R³, R⁴ and R⁵ are as defined in formula (I) above.

In one embodiment of the compounds of the invention R¹ is alkyl. In other embodiment R¹ is C₁-C₅ alkyl. In another embodiment R¹ is selected from the group consisting of methyl, ethyl, propyl, isopropyl, 2-methyl-propyl, 2,2-dimethyl-propyl, butyl, tert-butyl, 3-methyl-butyl, and pentyl.

Specific examples of R¹ include methyl, ethyl, propyl, 2-methyl-propyl, t-butyl, butyl, 3-methyl-butyl, and pentyl.

In one embodiment of the invention R³ is C₁-C₆ alkyl. In another embodiment of the invention R³ is selected from the group consisting of methyl, ethyl, propyl, isopropyl, 2-methyl-propyl, 2,2-dimethyl-propyl, butyl, tert-butyl, 2-ethyl-butyl, 3-methyl-butyl, 3,3-dimethyl-butyl, pentyl, 2-methyl-pentyl, 3-methyl-pentyl, 4-methyl-pentyl and hexyl.

According to the invention if the total number of carbon atoms in R² is X and the total number of carbon atoms in R³ is Y, then R² and R³ are selected such that the sum of X + Y is an integer greater than or equal to 5, that is 5, 6, 7 or 8. In another embodiment of the invention if the total number of carbon atoms in R² is X and the total number of carbon atoms in R³ is Y then R² and R³ are selected such that the sum of X + Y is an integer selected from the group consisting of 6, 7 and 8. In one embodiment of the invention the sum of X + Y is 6. In another embodiment of the invention the sum of X + Y is 7. In another embodiment of the invention the sum of X + Y is 8.

In a non-inventive embodiment if R² is H and the total number of carbon atoms in R¹ is Z and the total number of carbon atoms in R³ is Y, then R¹ and R³ are selected such that the sum of Z + Y is an integer greater than or equal to 5.

In another embodiment of the invention if the total number of carbon atoms in R¹ is Z and the total number of carbon atoms in R³ is Y then R¹ and R³ are selected such that the sum of Z + Y is an integer selected from the group consisting of 6, 7, 8 and 9. In one embodiment of the invention the sum of Z + Y is 6. In another embodiment of the invention the sum of Z + Y is 7. In another embodiment of the invention the sum of Z + Y is 8. In another embodiment of the invention the sum of Z + Y is 9.

In a non-inventive embodiment if R² is H and R³ is n-butyl then R¹ is not methyl.

In a non-inventive embodiment if R² is H and R³ is n-butyl then R¹ is not t-butyl.

In an embodiment of the invention if R² is methyl and R³ is propyl then R¹ is not t-butyl.

According to the invention R⁴ is H.

According to the invention R⁵ is H.

The values of R² and R³ can be selected such that the group -CH₂NR²R³ is selected from the group consisting of - resulting in inventive or non-inventive compounds:

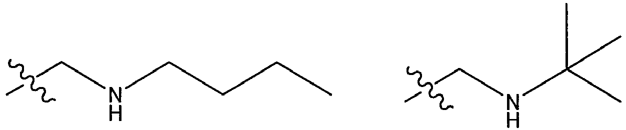

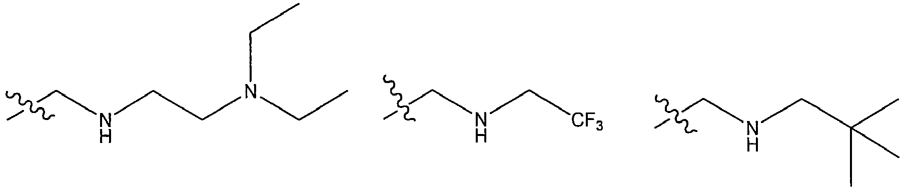

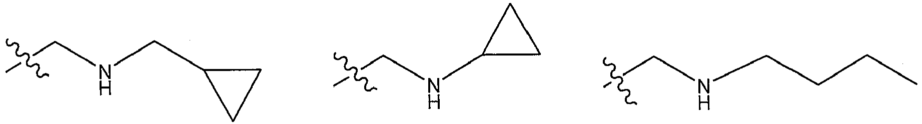

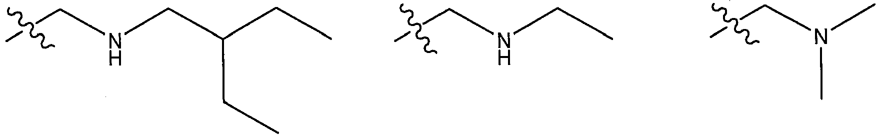

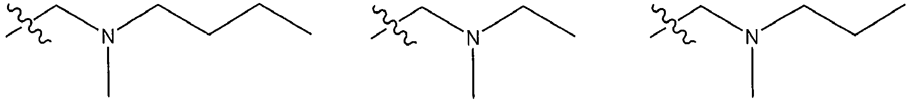

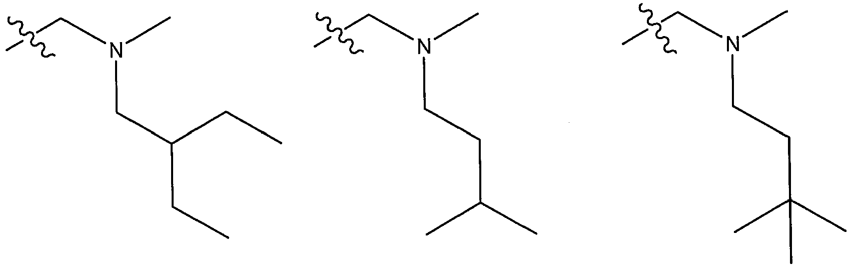

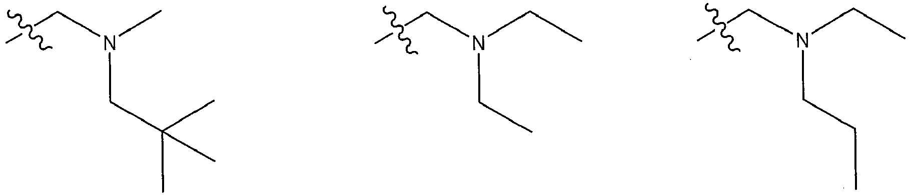

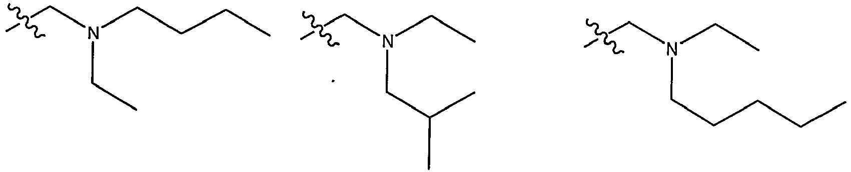

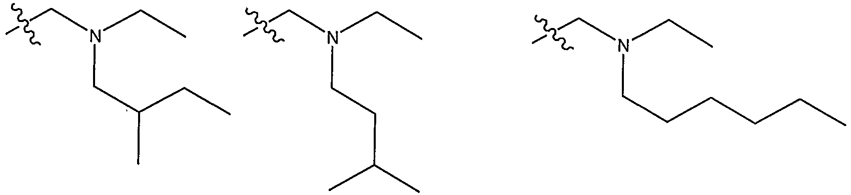

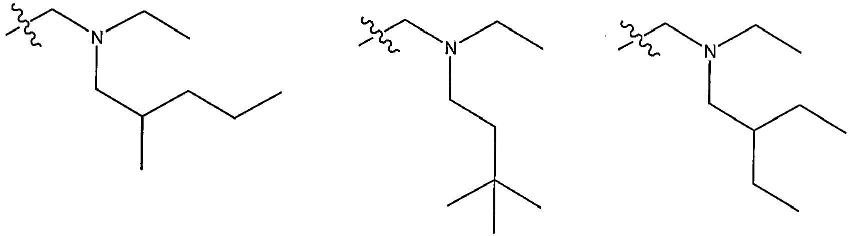

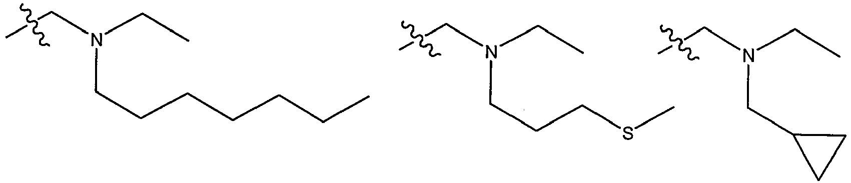

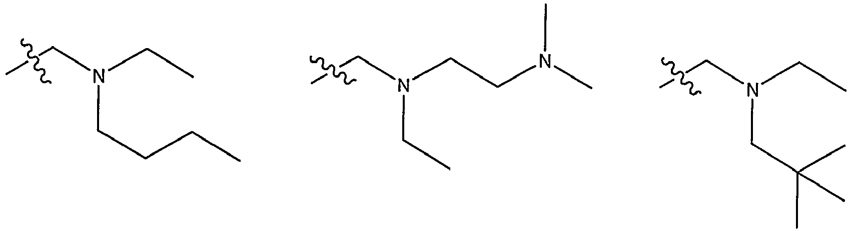

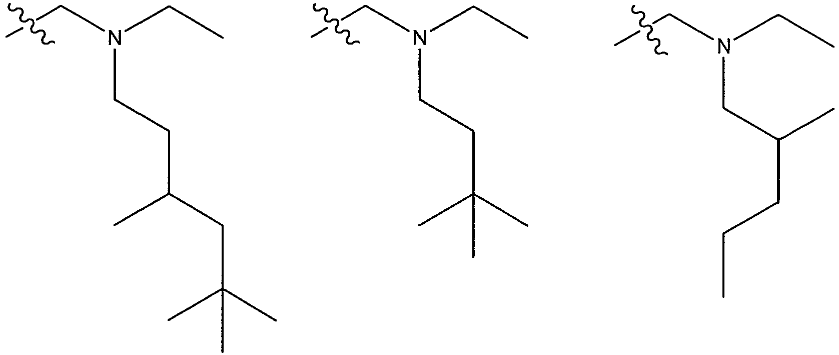

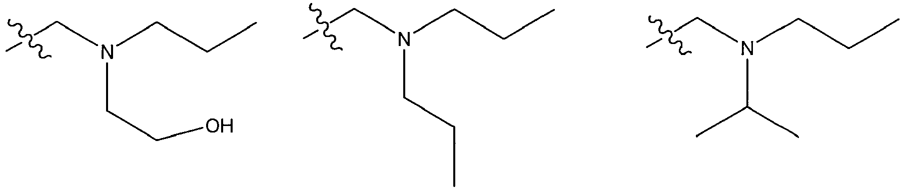

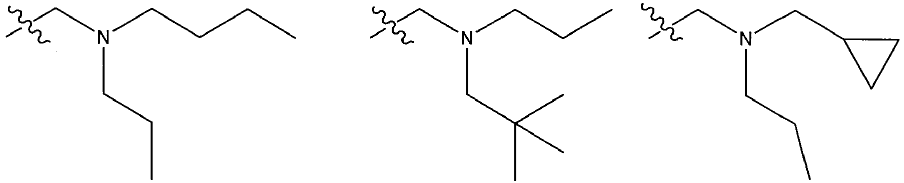

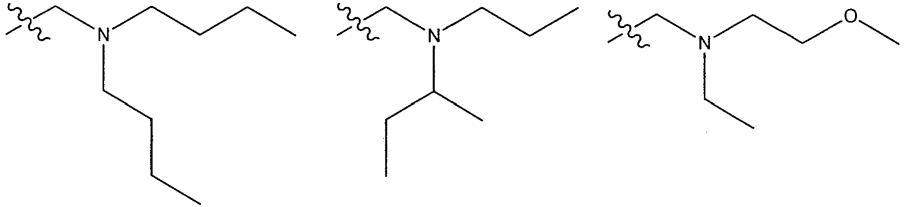

In addition to compounds of the invention as described above the embodiments disclosed are also directed to pharmaceutically acceptable salts. Such compounds and salts are at times collectively referred to herein as "HDAC inhibiting agents" or "HDAC inhibitors". In certain embodiments the compounds disclosed are used to modify deacetylase activity, in some cases histone deacetylase activity and in some cases HDAC 8, or HDAC 1 activity.

The embodiments disclosed also relate to pharmaceutical compositions each comprising a therapeutically effective amount of a HDAC inhibiting agent of the embodiments described with a pharmaceutically acceptable carrier or diluent for treating cellular proliferative ailments. The term "effective amount" as used herein indicates an amount necessary to administer to a host to achieve a therapeutic result, e.g., inhibition of proliferation of malignant cancer cells, benign tumor cells or other proliferative cells.

The invention also relates to pharmaceutical compositions including a compound of the invention with a pharmaceutically acceptable carrier, diluent or excipient.

In yet a further aspect the present invention provides a method of treatment of a disorder caused by, associated with or accompanied by disruptions of cell proliferation and/or angiogenesis including administration of a therapeutically effective amount of a compound of formula (I).

In one embodiment the disorder is selected from the group consisting of but not limited to cancer (e.g. breast cancer, colon cancer, prostate cancer, pancreatic cancer, leukemias, lymphomas, ovarian cancers, neuroblastomas, melanoma, inflammatory diseases/immune system disorders, angiofibroma, cardiovascular diseases (e.g. restenosis, arteriosclerosis), fibrotic diseases (e.g. liver fibrosis), diabetes, autoimmune diseases, chronic and acute neurodegenerative disease like disruptions of nerval tissue, Huntington's disease and infectious diseases like fungal, bacterial and viral infections. In another embodiment the disorder is a proliferative disorder. The proliferative disorder is preferably cancer. The cancer can include solid tumors or hematologic malignancies.

The invention also provides agents for the treatment of a disorder caused by, associated with or accompanied by disruptions of cell proliferation and/or angiogenesis including a compound of formula (I) as disclosed herein. In one embodiment the agent is an anti-cancer agent. In another embodiment, the agent is an anti-angiogenesis agent.

The invention also relates to the use of compounds of formula (I) in the manufacture of a medicament for the treatment of a disorder caused by, associated with or accompanied by disruptions of cell proliferation and/or angiogenesis. In one embodiment the disorder is a proliferative disorder, such as a cancer.

In yet a further aspect the invention provides the use of a compound of formula (I) to modify deacetylase activity. In one embodiment the deacetylase activity is histone deacetylase activity. In one embodiment the deacetylase activity is class I histone deacetylase activity. In a specific embodiment the histone deacetylase is HDAC1.

In yet a further embodiment the invention provides a method of treatment of a disorder, disease or condition that can be treated by the inhibition of histone deacetylase including administration of a therapeutically effective amount of a compound of formula (I).

In one embodiment the disorder is selected from the group consisting of but not limited to Proliferative disorders (e.g. cancer); Neurodegenerative diseases including Huntington's Disease, Polyglutamine diseases, Parkinson's Disease, Alzheimer's Disease, Seizures, Striatonigral degeneration, Progressive supranuclear palsy, Torsion dystonia, Spasmodic torticollis and dyskinesis, Familial tremor, Gilles de la Tourette syndrome, Diffuse Lewy body disease, Pick's disease, Intracerebral haemorrhage, Primary lateral sclerosis, Spinal muscular atrophy, Amyotrophic lateral sclerosis, Hypertrophic interstitial polyneuropathy, Retinitis pigmentosa, Hereditary optic atrophy, Hereditary spastic paraplegia, Progressive ataxia and Shy-Drager syndrome; Metabolic diseases including Type 2 diabetes; Degenerative Diseases of the Eye including Glaucoma, Age-related macular degeneration, macular myopic degeneration, Rubeotic glaucoma, Interstitial keratitis, Diabetic retinopathy, Peter's anomaly retinal degeneration, Cellophane Retinopathy; Cogan's Dystrophy; Corneal Dystrophy; Iris Neovascularization (Rubeosis); Neovascularization of the Cornea; Retinopathy of Prematurity; Macular Edema; Macular Hole; Macular Pucker; Marginal Blepharitis, Myopia, nonmalignant growth of the conjunctiva; Inflammatory diseases and/or Immune system disorders including Rheumatoid Arthritis (RA), Osteoarthritis, Juvenile chronic arthritis, Graft versus Host disease, Psoriasis, Asthma, Spondyloarthropathy, Crohn's Disease, inflammatory bowel disease, Colitis Ulcerosa, Alcoholic hepatitis, Diabetes, Sjoegrens's syndrome, Multiple Sclerosis, Ankylosing spondylitis, Membranous glomerulopathy, Discogenic pain, Systemic Lupus Erythematosus, allergic contact dermatitis; Disease involving angiogenesis including cancer, psoriasis, rheumatoid arthritis; Psychological disorders including bipolar disease, schizophrenia, depression and dementia; Cardiovascular Diseases including Heart failure, restenosis, cardiac hypertrophy and arteriosclerosis; Fibrotic diseases including liver fibrosis, lung fibrosis, cystic fibrosis and angiofibroma; Infectious diseases including Fungal infections, such as *Candida Albicans*, Bacterial infections, Viral infections, such as Herpes Simplex, Protozoal infections, such as Malaria, Leishmania infection, Trypanosoma brucei infection, Toxoplasmosis and coccidiosis, and Haematopoietic disorders including thalassemia, anemia and sickle cell anemia.

The invention also provides agents for the treatment of a disorder, disease or condition that can be treated by the inhibition of histone deacetylase including a compound of formula (I) as disclosed herein. In one embodiment the agent is an anti-cancer agent.

The invention also relates to the use of compounds of formula (I) in the manufacture of a medicament for the treatment of a disorder, disease or condition that can be treated by the inhibition of histone deacetylase.

The invention also provides a method for inhibiting cell proliferation including administration of an effective amount of a compound according to formula (I).

The invention also provides agents for inhibiting cell proliferation including a compound of formula (I) as disclosed herein.

The invention also relates to the use of compounds of formula (I) in the manufacture of a medicament for inhibiting cell proliferation.

In yet an even further aspect the invention provides a method of treatment of a neurodegenerative disorder in a patient including administration of a therapeutically effective amount of a compound of formula (I). In one embodiment the neurodegenerative disorder is Huntington's Disease.

The invention also provides agents for the treatment of a neurodegenerative disorder including a compound of formula (I) as disclosed herein. In one embodiment the agent is an anti-Huntington's disease agent.

The invention also relates to the use of compounds of formula (I) in the manufacture of a medicament for the treatment of a neurodegenerative disorder. In one embodiment the neurodegenerative disorder is Huntington's Disease.

In yet an even further aspect the invention provides a method of treatment of an inflammatory disease and/or immune system disorder in a patient including administration of a therapeutically effective amount of a compound of formula (I). In one embodiment the inflammatory disease and/or immune system disorder is rheumatoid arthritis. In another embodiment the inflammatory disease and/or immune system disorder is Systemic Lupus Erythematosus.

The invention also provides agents for the treatment of inflammatory disease and/or immune system disorder including a compound of formula (I) as disclosed herein.

The invention also relates to the use of compounds of formula (I) in the manufacture of a medicament for the treatment of inflammatory disease and/or immune system disorder. In one embodiment the inflammatory disease and/or immune system disorder is rheumatoid arthritis. In another embodiment the inflammatory disease and/or immune system disorder is Systemic Lupus Erythematosus.

In yet an even further aspect the invention provides a method of treatment of eye disease mediated by HDAC inhibition in a patient including administration of a therapeutically effective amount of a compound of formula (I). In one embodiment, the eye disease is macular degeneration. In another embodiment, the eye disease is glaucoma. In another embodiment, the eye disease is retinal degeneration.

The invention also provides agents for the treatment of eye disease mediated by HDAC inhibition including a compound of formula (I). In one embodiment, the eye disease is macular degeneration. In another embodiment, the eye disease is glaucoma. In another embodiment, the eye disease is retinal degeneration.

The invention also relates to the use of compounds of formula (I) in the preparation of a medicament for the treatment of eye disease mediated by HDAC inhibition. In one embodiment, the eye disease is macular degeneration. In another embodiment, the eye disease is glaucoma. In another embodiment, the eye disease is retinal degeneration.

The invention also relates to a method of treatment of a proliferative disorder in patient including administration of a therapeutically effective amount of a compound according to formula (I) to the patient.

The invention also relates to the use of a compound of formula (I) in the manufacture of a medicament for the treatment of a proliferative disorder.

The invention also relates to a method of treatment of cancer in patient including administration of a therapeutically effective amount of a compound of formula (I) to the patient. In one embodiment the cancer is a hematologic malignancy. In one embodiment the hematologic malignancy is selected from the group consisting of B-cell lymphoma, T-cell lymphoma and leukemia. In one embodiment the cancer is a solid tumor. In one embodiment the solid tumor is selected from the group consisting of breast cancer, lung cancer, ovarian cancer, prostate cancer, head and neck cancer, renal cancer, gastric cancer, colon cancer, pancreatic cancer and brain cancer.

The invention also provides agents for the treatment cancer including a compound of formula (I). In one embodiment the cancer is a hematologic malignancy. In one embodiment the hematologic malignancy is selected from the group consisting of B-cell lymphoma, T-cell lymphoma and leukemia. In one embodiment the cancer is a solid tumor. In one embodiment the solid tumor is selected from the group consisting of breast cancer, lung cancer, ovarian cancer, prostate cancer, head and neck cancer, renal cancer, gastric cancer, colon cancer, pancreatic cancer and brain cancer.

The invention also relates to the use of a compound of formula (I) in the manufacture of a medicament for the treatment of cancer. In one embodiment the cancer is a hematologic malignancy. In one embodiment the hematologic malignancy is selected from the group consisting of B-cell lymphoma, T-cell lymphoma and leukemia. In one embodiment the cancer is a solid tumor. In one embodiment the solid tumor is selected from the group consisting of breast cancer, lung cancer, ovarian cancer, prostate cancer, head and neck cancer, renal cancer, gastric cancer, colon cancer, pancreatic cancer and brain cancer.

### DETAILED DESCRIPTION OF THE EMBODIMENTS

There are disclosed hydroxamate compounds, for example imidazo[1,2-a]pyridine containing hydroxamic acid in one of the substituents, that may be inhibitors of deacetylases, including but not limited to inhibitors of histone deacetylases. The hydroxamate compounds may be suitable for prevention or treatment of a disorder caused by, associated with or accompanied by disruptions of cell proliferation and/or angiogenesis when used either alone or together with a pharmaceutically acceptable carrier, diluent or excipient. An example of such a disorder is cancer.

As used herein the term 'cancer' is a general term intended to encompass the vast number of conditions that are characterised by uncontrolled abnormal growth of cells.

It is anticipated that the compounds of the invention will be useful in treating various cancers including but not limited to bone cancers including Ewing's sarcoma, osteosarcoma, chondrosarcoma and the like, brain and CNS tumours including acoustic neuroma, neuroblastomas, glioma and other brain tumours, spinal cord tumours, breast cancers, colorectal cancers, advanced colorectal adenocarcinomas, colon cancers, endocrine cancers including adenocortical carcinoma, pancreatic cancer, pituitary cancer, thyroid cancer, parathyroid cancer, thymus cancer, multiple endocrine neoplasma, gastrointestinal cancers including stomach cancer, esophageal cancer, small intestine cancer, Liver cancer, extra hepatic bile duct cancer, gastrointestinal carcinoid tumour, gall bladder cancer, genitourinary cancers including testicular cancer, penile cancer, prostate cancer, gynaecological cancers including cervical cancer, ovarian cancer, vaginal cancer, uterus/endometrium cancer, vulva cancer, gestational trophoblastic cancer, fallopian tube cancer, uterine sarcoma, head and neck cancers including oral cavity cancer, lip cancer, salivary gland cancer, larynx cancer, hypopharynx cancer, orthopharynx cancer, nasal cancer, paranasal cancer, nasopharynx cancer, leukemias including childhood leukemia, acute lymphocytic leukemia, acute myeloid leukemia, chronic lymphocytic leukemia, chronic myeloid leukemia, hairy cell leukemia, acute promyelocytic leukemia, plasma cell leukemia, myelomas, haematological disorders including myelodysplastic syndromes, myeloproliferative disorders, aplastic anemia, Fanconi anemia, Waldenstroms Macroglobulinemia, lung cancers including small cell lung cancer, non-small cell lung cancer, lymphomas including Hodgkin's disease, non-Hodgkin's lymphoma, cutaneous T-cell lymphoma, peripheral T-cell lymphoma, AIDS related Lymphoma, B-cell lymphoma, Burkitt's lymphoma; eye cancers including retinoblastoma, intraocular melanoma, skin cancers including melanoma, non-melanoma skin cancer, merkel cell cancer, soft tissue sarcomas such as childhood soft tissue sarcoma, adult soft tissue sarcoma, Kaposi's sarcoma, urinary system cancers including kidney cancer, Wilms tumour, bladder cancer, urethral cancer, and transitional cell cancer.

Exemplary cancers that may be treated by the compounds of the present invention are breast cancer, lung cancer, ovarian cancer, prostate cancer, head and neck cancer, renal cancer (e.g. renal cell carcinoma), gastric cancer, colon cancer, colon cancer, colorectal cancer and brain cancer.

Exemplary cancers that may be treated by compounds of the present invention include but are not limited to B-cell lymphoma (e.g. Burkitt's lymphoma), leukemias (e.g. Acute promyelocytic leukemia), cutaneous T-cell lymphoma (CTCL) and peripheral T-cell lymphoma.

Exemplary cancers that may be treated by compounds of the present invention include solid tumors and hematologic malignancies.

The compounds may also be used in the treatment of a disorder involving, relating to or, associated with dysregulation of histone deacetylase (HDAC).

There are a number of disorders that have been implicated by or known to be mediated at least in part by HDAC activity, where HDAC activity is known to play a role in triggering disease onset, or whose symptoms are known or have been shown to be alleviated by HDAC inhibitors. Disorders of this type that would be expected to be amenable to treatment with the compounds of the invention include the following but not limited to: Proliferative disorders (e.g. cancer); Neurodegenerative diseases including Huntington's Disease, Polyglutamine diseases, Parkinson's Disease, Alzheimer's Disease, Seizures, Striatonigral degeneration, Progressive supranuclear palsy, Torsion dystonia, Spasmodic torticollis and dyskinesis, Familial tremor, Gilles de la Tourette syndrome, Diffuse Lewy body disease, Pick's disease, Intracerebral haemorrhage, Primary lateral sclerosis, Spinal muscular atrophy, Amyotrophic lateral sclerosis, Hypertrophic interstitial polyneuropathy, Retinitis pigmentosa, Hereditary optic atrophy, Hereditary spastic paraplegia, Progressive ataxia and Shy-Drager syndrome; Metabolic diseases including Type 2 diabetes; Degenerative Diseases of the Eye including Glaucoma, Age-related macular degeneration, macular myopic degeneration, Rubeotic glaucoma, Interstitial keratitis, Diabetic retinopathy, Peter's anomaly retinal degeneration, Cellophane Retinopathy; Cogan's Dystrophy; Corneal Dystrophy; Iris Neovascularization (Rubeosis); Neovascularization of the Cornea; Retinopathy of Prematurity; Macular Edema; Macular Hole; Macular Pucker; Marginal Blepharitis, Myopia, nonmalignant growth of the conjunctiva; Inflammatory diseases and/or Immune system disorders including Rheumatoid Arthritis (RA), Osteoarthritis, Juvenile chronic arthritis, Graft versus Host disease, Psoriasis, Asthma, Spondyloarthropathy, Crohn's Disease, inflammatory bowel disease, Colitis Ulcerosa, Alcoholic hepatitis, Diabetes, Sjoegrens's syndrome, Multiple Sclerosis, Ankylosing spondylitis, Membranous glomerulopathy, Discogenic pain, Systemic Lupus Erythematosus, allergic contact dermatitis; Disease involving angiogenesis including cancer, psoriasis, rheumatoid arthritis; Psychological disorders including bipolar disease, schizophrenia, depression and dementia; Cardiovascular Diseases including Heart failure, restenosis, cardiac hypertrophy and arteriosclerosis; Fibrotic diseases including liver fibrosis, lung fibrosis, cystic fibrosis and angiofibroma; Infectious diseases including Fungal infections, such as *Candida Albicans*, Bacterial infections, Viral infections, such as Herpes Simplex, Protozoal infections, such as Malaria, Leishmania infection, Trypanosoma brucei infection, Toxoplasmosis and coccidiosis, and Haematopoietic disorders including thalassemia, anemia and sickle cell anemia.

As used herein, the term unsubstituted means that there is no substituent or that the only substituents are hydrogen.

The term "optionally substituted" as used throughout the specification denotes that the group may or may not be further substituted or fused (so as to form a condensed polycyclic system), with one or more non-hydrogen substituent groups.

"Alkyl" as a group or part of a group refers to a straight or branched aliphatic hydrocarbon group, preferably a C₁-C₁₄ alkyl, more preferably C₁-C₁₀ alkyl, most preferably C₁-C₆ unless otherwise noted. Examples of suitable straight and branched C₁-C₆ alkyl substituents include methyl, ethyl, n-propyl, 2-propyl, n-butyl, sec-butyl, t-butyl, hexyl, and the like.

"Alkenyl" as group or part of a group denotes an aliphatic hydrocarbon group containing at least one carbon-carbon double bond and which may be straight or branched preferably having 2-14 carbon atoms, more preferably 2-12 carbon atoms, most preferably 2-6 carbon atoms, in the chain. The group may contain a plurality of double bonds in the normal chain and the orientation about each is independently E or Z. Exemplary alkenyl group include, but are not limited to, ethenyl and propenyl.

"Alkynyl as a group or part of a group means an aliphatic hydrocarbon group containing a carbon-carbon triple bond and which may be straight or branched preferably having from 2-14 carbon atoms, more preferably 2-12 carbon atoms in the chain, preferably 2-6 carbon atoms in the chain. Exemplary structures include, but are not limited to, ethynyl and propynyl.

"Cycloalkyl" refers to a saturated or partially saturated, monocyclic or fused or spiro polycyclic, carbocycle preferably containing from 3 to 9 carbons per ring, such as cyclopropyl, cyclobutyl, cyclopentyl, cyclohexyl and the like, unless otherwise specified. It includes monocyclic system such as cyclohexyl, bicyclic systems such as decalin, and polycyclic systems such as adamantane.

"Cycloalkylalkyl" means a cycloalkyl-alkyl- group in which the cycloalkyl and alkyl moieties are as previously described. Exemplary monocycloalkylalkyl groups include cyclopropylmethyl, cyclopentylmethyl, cyclohexylmethyl and cycloheptylmethyl.

"Heterocycloalkyl" refers to a saturated or partially saturated monocyclic, bicyclic or polycyclic ring containing at least a heteroatom selected from nitrogen, sulfur, oxygen, preferably from 1 to 3 heteroatoms in at least one ring. Each ring is preferably from 3 to 10 membered, more preferably 4 to 7 membered. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane.

"Heterocycloalkylalkyl" refers to a heterocycloalkyl-alkyl group in which the heterocycloalkyl and alkyl moieties are as previously described. Exemplary heterocycloalkylalkyl groups include (2-tetrahydrofuryl)methyl, (2-tetrahydrothiofuranyl)methyl.

"Acyl" means an alkyl-CO- group in which the alkyl group is as described herein. Examples of acyl include acetyl and benzoyl. The alkyl group is preferably a C₁-C₆ alkyl group.

"Heteroalkyl" refers to a straight- or branched-chain alkyl group preferably having from 2 to 14 carbons, more preferably 2 to 10 atoms in the chain, one or more of which has been replaced by a heteroatom selected from S, O, and N. Exemplary heteroalkyls include alkyl ethers, secondary and tertiary alkyl amines, alkyl sulfides, and the like.

"Aryl" as a group or part of a group denotes (i) an optionally substituted monocyclic, or fused polycyclic, aromatic carbocycle (ring structure having ring atoms that are all carbon) preferably having from 5 to 12 atoms per ring. Examples of aryl groups include phenyl, naphthyl, and the like; (ii) an optionally substituted partially saturated bicyclic aromatic carbocyclic moiety in which a phenyl and a C₅₋₇ cycloalkyl or C₅₋₇ cycloalkenyl group are fused together to form a cyclic structure, such as tetrahydronaphthyl, indenyl or indanyl.

"Arylalkyl" means an aryl-alkyl- group in which the aryl and alkyl moieties are as previously described. Preferred arylalkyl groups contain a C₁₋₅ alkyl moiety. Exemplary arylalkyl groups include benzyl, phenethyl and naphthelenemethyl.

The term "heteroaryl" either alone or part of another group refers to groups containing an aromatic ring (preferably a 5 or 6 membered aromatic ring) having 1 or more heteroatoms as ring atoms in the aromatic ring with the remainder of the ring atoms being carbon atoms. Suitable heteroatoms include oxygen, sulfur, and nitrogen. Examples of heteroaryl include thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, indole, isoindole, 1 H-indazole, purine, 4H-quinolidine, isoquinoline, quinoline, phthalazine, naphthyridine, quinoxaline, quinazoline, cinnoline, carbazole, phenanthridine, acridine, phenazine, thiazole, isothiazole, phenothiazine, oxazole, isoxazole, furazane, phenoxazine, 2-, 3-, or 4-pyridyl, 2-, 3-, 4-, 5-, or 8-quinolyl, 1-, 3-, 4-, or 5-isoquinolyl, 1-, 2-, or 3-indolyl, 2-benzothiazolyl, 2-benzo[b]thienyl, benzo[b]furanyl, 2- or 3-thienyl, or the like. More preferred examples include 2- or 3-thienyl, 2-, 3-, or 4-pyridyl, 2- or 3-quinolyl, 1-isoquinolyl, 1- or 2-indolyl, 2-benzothiazolyl, and the like.

"Heteroarylalkyl" means a heteroaryl-alkyl group in which the heteroaryl and alkyl moieties are as previously described. Preferred heteroarylalkyl groups contain a C₁ to C₆ alkyl moiety. Exemplary heteroarylalkyl groups include pyridylmethyl.

It is understood that included in the family of compounds of formula (I) are isomeric forms including diastereoisomers, enantiomers, and tautomers. It is also understood that some isomeric forms such as diastereomers, enantiomers, and geometrical isomers can be separated by physical and/or chemical methods and by those skilled in the art.

Some of the compounds of the disclosed embodiments may exist as single stereoisomers, racemates, and/or mixtures of enantiomers and/or diastereomers. All such single stereoisomers, racemates and mixtures thereof are intended to be within the scope of the subject matter described and claimed.

Additionally, formula (I) is intended to cover, where applicable, solvated as well as unsolvated forms of the compounds. Thus, each formula includes compounds having the indicated structure, including the hydrated as well as the non-hydrated forms.

In addition to compounds of the formula (I), the HDAC inhibiting agents of the various embodiments include pharmaceutically acceptable salts.

The term "pharmaceutically acceptable salts" refers to salts that retain the desired biological activity of the above-identified compounds, and include pharmaceutically acceptable acid addition salts and base addition salts. Suitable pharmaceutically acceptable acid addition salts of compounds of formula (I) may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, arylsulfonic. Suitable pharmaceutically acceptable base addition salts of compounds of formula (I) include metallic salts made from lithium, sodium, potassium, magnesium, calcium, aluminium, and zinc, and organic salts made from organic bases such as choline, diethanolamine, morpholine. Other examples of organic salts are: ammonium salts, quaternary salts such as tetramethylammonium salt; amino acid addition salts such as salts with glycine and arginine. Additional information on pharmaceutically acceptable salts can be found in Remington's Pharmaceutical Sciences, 19th Edition, Mack Publishing Co., Easton, PA 1995. In the case of agents that are solids, it is understood by those skilled in the art that the inventive compounds, agents and salts may exist in different crystalline or polymorphic forms, all of which are intended to be within the scope of the present invention and specified formulae.

"Prodrug" means a compound which is convertible *in vivo* by metabolic means (e.g. by hydrolysis, reduction or oxidation) to a compound of formula (I). For example an ester prodrug of a compound of formula (I) containing a hydroxyl group may be convertible by hydrolysis *in vivo* to the parent molecule. Suitable esters of compounds of formula (I) containing a hydroxyl group, are for example acetates, citrates, lactates, tartrates, malonates, oxalates, salicylates, propionates, succinates, fumarates, maleates, methylene-bis-β-hydroxynaphthoates, gestisates, isethionates, di-*p*-toluoyltartrates, methanesulphonates, ethanesulphonates, benzenesulphonates, *p*-toluenesulphonates, cyclohexylsulphamates and quinates. As another example an ester prodrug of a compound of formula I containing a carboxy group may be convertible by hydrolysis in vivo to the parent molecule. (Examples of ester prodrugs are those described by F.J. Leinweber, Drug Metab. Res.,18:379,1987).

Administration of compounds within formula (I) to humans can be by any of the accepted modes for enteral administration such as oral or rectal, or by parenteral administration such as subcutaneous, intramuscular, intravenous and intradermal routes. Injection can be bolus or via constant or intermittent infusion. The active compound is typically included in a pharmaceutically acceptable carrier or diluent and in an amount sufficient to deliver to the patient a therapeutically effective dose. In various embodiments the inhibitor compound may be selectively toxic or more toxic to rapidly proliferating cells, e.g. cancerous tumors, than to normal cells.

The term "therapeutically effective amount" or "effective amount" is an amount sufficient to effect beneficial or desired results. An effective amount can be administered in one or more administrations. An effective amount is typically sufficient to palliate, ameliorate, stabilize, reverse, slow or delay the progression of the disease state. A therapeutically effective amount can be readily determined by a skilled practitioner by the use of conventional techniques and by observing results obtained in analogous circumstances. In determining the effective amount a number of factors are considered including the species of the patient, its size, age, general health, the specific disease involved, the degree or severity of the disease, the response of the individual patient, the particular compound administered, the mode of administration, the bioavailability of the compound, the dose regimen selected, the use of other medication and other relevant circumstances.

In using the compounds of the invention they can be administered in any form or mode which makes the compound bioavailable. One skilled in the art of preparing formulations can readily select the proper form and mode of administration depending upon the particular characteristics of the compound selected, the condition to be treated, the stage of the condition to be treated and other relevant circumstances. We refer the reader to Remingtons Pharmaceutical Sciences, 19th edition, Mak Publishing Co. (1995) for further information.

The compounds of the present invention can be administered alone or in the form of a pharmaceutical composition in combination with a pharmaceutically acceptable carrier, diluent or excipient. The compounds of the invention, while effective themselves, are typically formulated and administered in the form of their pharmaceutically acceptable salts as these forms are typically more stable, more easily crystallised and have increased solubility.

The compounds are, however, typically used in the form of pharmaceutical compositions which are formulated depending on the desired mode of administration. As such in a further embodiment the present invention provides a pharmaceutical composition including a compound of formula (I) and a pharmaceutically acceptable carrier, diluent or excipient. The compositions are prepared in manners well known in the art.

The invention in other embodiments provides a pharmaceutical pack or kit comprising one or more containers filled with one or more of the ingredients of the pharmaceutical compositions of the invention. In such a pack or kit can be found a container having a unit dosage of the agent(s). The kits can include a composition comprising an effective agent either as concentrates (including lyophilized compositions), which can be diluted further prior to use or they can be provided at the concentration of use, where the vials may include one or more dosages. Conveniently, in the kits, single dosages can be provided in sterile vials so that the physician can employ the vials directly, where the vials will have the desired amount and concentration of agent(s). Associated with such container(s) can be various written materials such as instructions for use, or a notice in the form prescribed by a governmental agency regulating the manufacture, use or sale of pharmaceuticals or biological products, which notice reflects approval by the agency of manufacture, use or sale for human administration.

The compounds of the invention may be used or administered in combination with one or more additional drug (s) that include chemotherapeutic drugs or HDAC inhibitor drugs and/or procedures (e.g. surgery, radiotherapy) for the treatment of the disorder/diseases mentioned. The components can be administered in the same formulation or in separate formulations. If administered in separate formulations the compounds of the invention may be administered sequentially or simultaneously with the other drug(s).

In addition to being able to be administered in combination with one or more additional drugs that include chemotherapeutic drugs or HDAC inhibitor drugs the compounds of the invention may be used in a combination therapy. When this is done the compounds are typically administered in combination with each other. Thus one or more of the compounds of the invention may be administered either simultaneously (as a combined preparation) or sequentially in order to achieve a desired effect. This is especially desirable where the therapeutic profile of each compound is different such that the combined effect of the two drugs provides an improved therapeutic result.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of micro-organisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents that delay absorption such as aluminium monostearate and gelatin.

If desired, and for more effective distribution, the compounds can be incorporated into slow release or targeted delivery systems such as polymer matrices, liposomes, and microspheres.

The injectable formulations can be sterilized, for example, by filtration through a bacterial-retaining filter, or by incorporating sterilizing agents in the form of sterile solid compositions that can be dissolved or dispersed in sterile water or other sterile injectable medium just prior to use.

Solid dosage forms for oral administration include capsules, dragees, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

If desired, and for more effective distribution, the compounds can be incorporated into slow release or targeted delivery systems such as polymer matrices, liposomes, and microspheres.

The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminium metahydroxide, bentonite, agar-agar, and tragacanth, and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, patches, sprays, ointments and inhalants. The active compound is mixed under sterile conditions with a pharmaceutically acceptable carrier and any needed preservatives, buffers, or propellants which may be required.

A preferred dosage will be a range from about 0.01 to 300 mg per kilogram of body weight per day. A more preferred dosage will be in the range from 0.1 to 100 mg per kilogram of body weight per day, more preferably from 0.2 to 80 mg per kilogram of body weight per day, even more preferably 0.2 to 50 mg per kilogram of body weight per day. A suitable dose can be administered in multiple sub-doses per day.

As discussed above, the compounds of the embodiments disclosed inhibit histone deacetylases. The enzymatic activity of a histone deacetylase can be measured using known methodologies [Yoshida M. et al, J. Biol. Chem., 265, 17174 (1990), J. Taunton et al, Science 1996 272: 408]. In certain embodiments, the histone deacetylase inhibitor interacts with and/or reduces the activity of more than one known histone deacetylase in the cell, which can either be from the same class of histone deacetylase or different class of histone deacetylase. In some other embodiments, the histone deacetylase inhibitor interacts and reduces the activity of predominantly one histone deacetylase, for example HDAC-1, HDAC-2, HDAC-3 or HDAC-8, which belongs to Class I HDAC enzymes [De Ruijter A.J.M. et al, Biochem. J., 370, 737-749 (2003)]. HDACs can also target non-histone substrates to regulate a variety of biological functions implicated in disease pathogenesis. These non-histone substrates include Hsp90, α-tubulin, p53, NFkb and HIF1a [Drummond et al., Annu. Rev. Pharmacol. Toxicol. 45:495 (2004)]. Certain preferred histone deacetylase inhibitors are those that interact with, and/or reduce the activity of a histone deacetylase which is involved in tumorigenesis, and these compounds may be useful for treating proliferative diseases. Examples of such cell proliferative diseases or conditions include cancer (include any metastases), psoriasis, and smooth muscle cell proliferative disorders such as restenosis. The inventive compounds may be particularly useful for treating tumors such as breast cancer, colon cancer, lung cancer, ovarian cancer, prostate cancer, head and/or neck cancer, or renal, gastric, pancreatic cancer and brain cancer as well as hematologic malignancies such as lymphoma and leukemias. In addition, the inventive compounds may be useful for treating a proliferative disease that is refractory to the treatment with other chemotherapeutics; and for treating hyperproliferative condition such as leukemias, psoriasis and restenosis. In other embodiments, compounds of this invention can be used to treat pre-cancer conditions or hyperplasia including familial adenomatous polyposis, colonic adenomatous polyps, myeloid dysplasia, endometrial dysplasia, endometrial hyperplasia with atypia, cervical dysplasia, vaginal intraepithelial neoplasia, benign prostatic hyperplasia, papillomas of the larynx, actinic and solar keratosis, seborrheic keratosis and keratoacanthoma.

Additionally compounds of the various embodiments disclosed herein may be useful for treating neurodegenerative diseases, and inflammatory diseases and/or immune system disorders.

The disorder is preferably selected from the group consisting of cancer, inflammatory diseases and/or immune system disorders (e.g. rheumatoid arthritis, systemic lupus erythematosus), angiofibroma, cardiovascular diseases, fibrotic diseases, diabetes, autoimmune diseases, chronic and acute neurodegenerative disease like Huntington's disease, Parkinson's disease, disruptions of nerval tissue and infectious diseases like fungal, bacterial and viral infections. In another embodiment the disorder is a proliferative disorder.

The histone deacetylase inhibitors of the invention have significant antiproliferative effects and promote differentiation, cell cycle arrest in the G1 or G2 phase, and induce apoptosis.

### SYNTHESIS OF DEACETYLASE INHIBITORS

The agents of the various embodiments may be prepared using the reaction routes and synthesis schemes as described below, employing the techniques available in the art using starting materials that are readily available. The preparation of particular compounds of the embodiments is described in detail in the following examples, but the artisan will recognize that the chemical reactions described may be readily adapted to prepare a number of other agents of the various embodiments. For example, the synthesis of non-exemplified compounds may be successfully performed by modifications apparent to those skilled in the art, e.g. by appropriately protecting interfering groups, by changing to other suitable reagents known in the art, or by making routine modifications of reaction conditions. A list of suitable protecting groups in organic synthesis can be found in T.W. Greene and P. G. M. Wuts' Protective Groups in Organic Synthesis, 3rd Edition, Wiley-InterScience, 1999. Alternatively, other reactions disclosed herein or known in the art will be recognized as having applicability for preparing other compounds of the various embodiments.

Reagents useful for synthesizing compounds may be obtained or prepared according to techniques known in the art.

In the examples described below, unless otherwise indicated, all temperatures in the following description are in degrees Celsius and all parts and percentages are by weight, unless indicated otherwise.

Various starting materials and other reagents were purchased from commercial suppliers, such as Aldrich Chemical Company or Lancaster Synthesis Ltd., and used without further purification, unless otherwise indicated. For moisture sensitive reactions, anhydrous solvents like dichloromethane (DCM), dimethylsulfoxide (DMSO), tetrahydrofuran (THF) and N,N-dimethylformamide (DMF) were purchased from Aldrich in SureSeal bottles and used as received. All other solvents were purified by using standard methods in the art, unless otherwise indicated.

The reactions set forth below were performed under a positive pressure of nitrogen, argon or with a drying tube, at ambient temperature (unless otherwise stated), in anhydrous solvents, and the reaction flasks are fitted with rubber septa for the introduction of substrates and reagents via syringe. Glassware was oven-dried and/or heat-dried. Analytical thin-layer chromatography was performed on glass-backed silica gel 60 F 254 plates (E Merck (0.25 mm)) and eluted with the appropriate solvent ratios (v/v). The reactions were assayed by TLC and terminated as judged by the consumption of starting material.

The TLC plates were visualized by UV absorption or with a p-anisaldehyde spray reagent or a phosphomolybdic acid reagent (Aldrich Chemical, 20wt% in ethanol) which was activated with heat, or by staining in iodine chamber. Work-ups were typically done by doubling the reaction volume with the reaction solvent or extraction solvent and then washing with the indicated aqueous solutions using 25% by volume of the extraction volume (unless otherwise indicated). Product solutions were dried over anhydrous sodium sulfate prior to filtration, and evaporation of the solvents was under reduced pressure on a rotary evaporator and noted as solvents removed in vacuo. Flash column chromatography [Still et al, J. Org. Chem., 43, 2923 (1978)] was conducted using E Merck-grade flash silica gel (47-61 mm) and a silica gel:crude material ratio of about 20:1 to 50:1, unless otherwise stated. Hydrogenolysis was done at the pressure indicated or at ambient pressure.

¹H NMR spectra was recorded on a Bruker instrument operating at 400 MHz, and ¹³C-NMR spectra was recorded operating at 100 MHz. NMR spectra are obtained as CDCl₃ solutions (reported in ppm), using chloroform as the reference standard (7.25 ppm and 77.00 ppm) or CD₃OD (3.4 and 4.8 ppm and 49.3 ppm), or an internal tetramethylsilane standard (0.00 ppm) when appropriate. Other NMR solvents were used as needed. When peak multiplicities are reported, the following abbreviations are used: s = singlet, d = doublet, t = triplet, m = multiplet, br = broadened, dd = doublet of doublets, dt = doublet of triplets. Coupling constants, when given, are reported in Hertz.

Mass spectra were obtained using LC/MS either in ESI or APCI. All melting points are uncorrected.

All final products had greater than 90% purity (LC/PDA: Xterra 1 S column, 4.6 x 20mm 3.5µ column; 2.0 ml/min, gradient 5-95% B over 6 min, Solvent A: H₂O with 0.1% TFA; Solvent B: acetonitrile with 0.1 TFA; UV 254)).

The following examples are intended to illustrate the embodiments disclosed and are not to be construed as being limitations thereto. Additional compounds, other than those described below, may be prepared using the following described reaction scheme or appropriate variations or modifications thereof.

The compounds 1 to 5, 15, 26, 53, 58, 62 and 65 are not belonging to the group of inventive compounds.

### SYNTHESIS

Scheme 1 illustrates one method of preparing the compounds of the invention. The imidazopyridine core structure was constructed by a condensation reaction using 4-bromo-2-aminopyridine **I** as one of the starting materials [J. Med. Chem. 1998, 41, 5108]. The hydroxymethyl group was introduced at the 3 position by reacting with formaldehyde to give the intermediate **IV.** This intermediate **IV** was then subjected to Heck reaction condition in which the alkenyl ester **VI** was produced. The alcohol group in **VI** was then oxidized to the aldehyde **VII** which was further converted to an aminoalkyl group under reductive amination conditions using sodium acetoxyborohydride. The hydroxamic acid **VIII** was formed as described in the previous schemes.

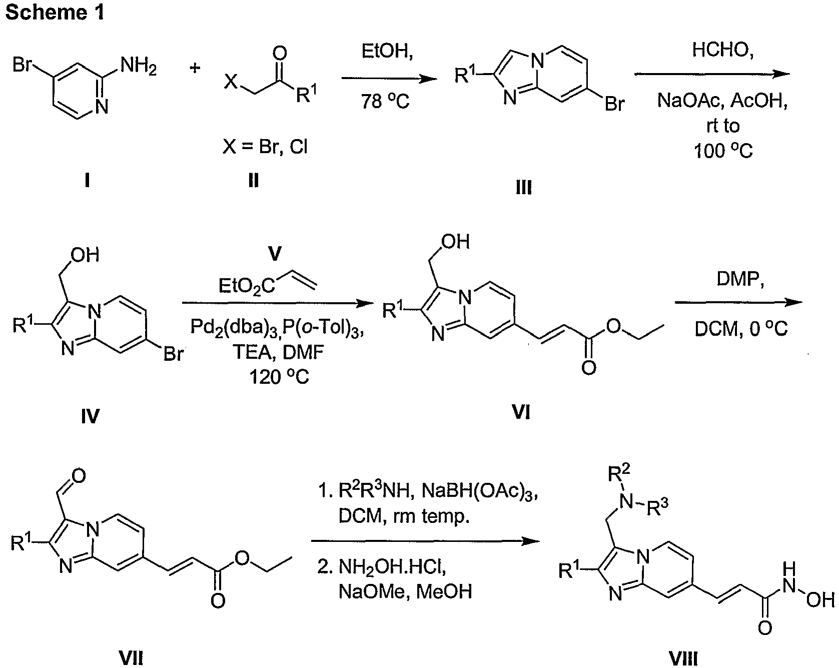

Scheme 2 illustrates yet another method in preparing compounds of the invention. The imidazopyridine core structure was constructed by the condensation reaction using 4-bromo-2-aminopyridine **I** as one of the starting material. The alkenyl ester group was introduced at the 6-position by the Heck reaction. This intermediate **X** was then subjected to a Mannich reaction in which the aminoalkyl group was introduced [J. Org. Chem. 1965, 30, 2403]. Without further workups and purifications, the crude material was converted into the hydroxamic acid **VIII** as described in the previous schemes.

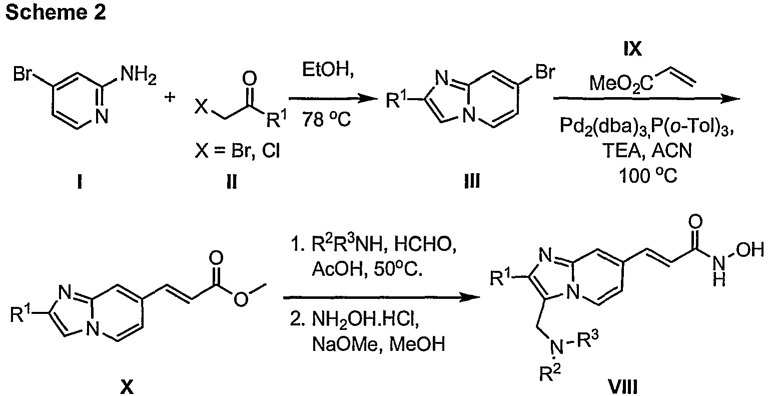

Scheme 3 illustrates yet another method of preparing compounds of the invention. The imidazopyridine core structure was constructed by the condensation reaction using 4-cyano-2-aminopyridine **XI** as one of the starting material. The alkenyl ester group was introduced at the 6-position by a series of common organic transformations (basic hydrolysis; esterification; DIBAL-H reduction; DMP oxidation and the Wittig reaction). The intermediate **XVII** was then subjected to a Mannich reaction in which the aminoalkyl group was introduced [J. Org. Chem. 1965, 30, 2403]. Without further workups and purifications, the crude material was converted into the hydroxamic acid **VIII** as described in the previous schemes.

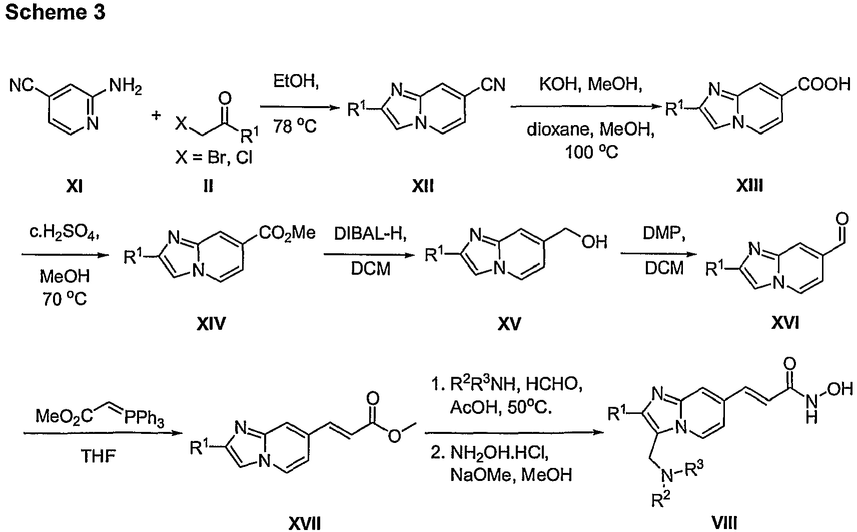

Scheme 4 illustrates yet another method in preparing compounds of the invention. The imidazopyridine core structure was constructed by the condensation reaction using 4-bromo-2-aminopyridine **II** as one of the starting material. The alkenyl ester group was introduced at the 6-position by the Heck reaction. This intermediate **XVII** was then subjected to a Mannich reaction in which the aminoalkyl group was introduced *[*J. Org. Chem. 1965, 30, 2403]. Without further workups and purifications, the crude material was converted into the hydroxamic acid **XVIII** as described in the previous schemes. After purification by reversed-phase prep-HPLC, the intermediate **VIII** was subjected to reductive amination with the appropriate aldehydes to furnish the desired product.

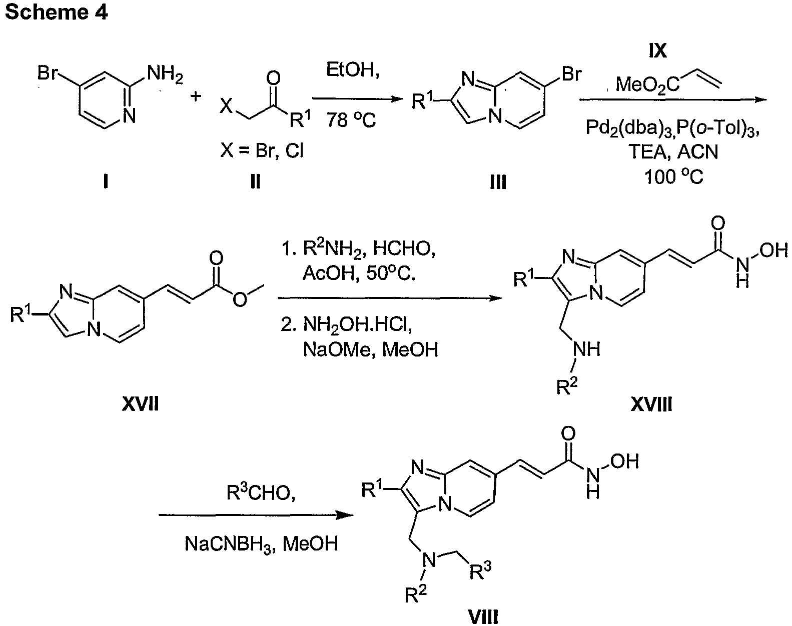

The following preparation and examples are given to enable those skilled in the art to more clearly understand and to practice the subject matter hereof. They should not be considered as limiting the scope of the disclosure, but merely as being illustrative and representative thereof.

### General Synthetic Procedure

### Step 1: Condensation reaction

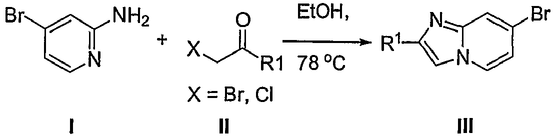

To a stirred solution of the amino-pyridine **I** (0.58 mmol) and EtOH (1.4 mL) was added an appropriately substituted ketone **II** (0.69 mmol) and the mixture was then stirred at 78 °C for 4 h. When the reaction has completed, the contents were evaporated. Saturated sodium carbonate solution was added and ethyl acetate was used to extract the aqueous layer. The combined organic extracts were then washed with water and followed by brine, before drying in anhydrous sodium sulfate. The contents were then filtered and concentrated. The crude product was used immediately without further purification (Journal of Medicinal Chemistry, 1998, 41(25), 5108).

### Step 2: Heck reaction

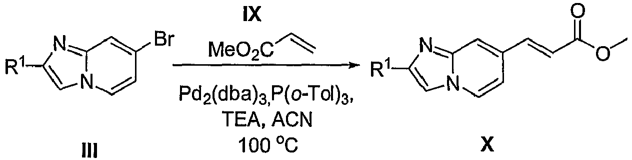

Methyl acrylate (1.5 equiv) was added into a stirred suspension of the imidazopyridine **III** (1 equiv), Pd₂(dba)₃ (0.02 equiv), P(*o*-Tol)₃ (0.05 equiv), Et₃N (2.0 equiv) and CH₃CN (0.3 M) at room temperature. The reaction was heated to reflux at ~ 100 °C. When the starting material had fully depleted (monitored by LCMS), the reaction mixture was diluted with ethyl acetate. The organic layer was then washed with NaHCO₃ and brine. The organic layer was dried in Na₂SO₄ before being filtered and concentrated in vacuo. The crude product was purified by flash column chromatography.

### Step 3: Mannich reaction and Hydroxamic acid formation

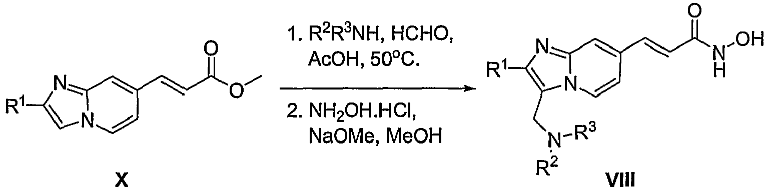

The amine (3.0 equiv) was added slowly into a stirred solution of the imidazo[1,2-α]pyridinyl methyl ester **X** (1.0 equiv), formaldehyde solution (3.0 equiv) and AcOH (20 equiv) and the mixture was heated up to 50 °C. When the starting material has fully depleted (monitored by LCMS), the crude product was used immediately for the next step.

To a stirred solution of the crude material from the Mannich reaction and NH₂OH.HCl (20 equiv) was added NaOMe (40 equiv) at - 78 °C. The reaction mixture was allowed to warm up slowly to room temperature. When the reaction is completed, the mixture was cooled to 0 °C before using 1 M HCl to quench the reaction. Small amounts of MeOH and H₂O were added to solubilize the mixture. The crude product was purified by reverse-phase prep-HPLC.

### Example 1

### 3-{2-tert-Butyl-3-[(2,2-dimethyl-propylamino)-methyl]-imidazo[1,2-a]pyridin-7-yl}-N-hydroxy-acrylamide (Compound 1)

The titled compound was prepared according to the procedures described in the general synthetic procedure by using appropriate starting materials. HPLC: 85.65 %; t_{R} = 0.821 min; LCMS (ESI) m/z 359 [MH]⁺.

### Example 2

### 3-{2-tert-Butyl-3-[(1-ethyl-propylamino)-methyl]-imidazo[1,2-a]pyridin-7-yl}-N-hydroxy-acrylamide (Compound 2)

The titled compound was prepared according to the procedures described in the general synthetic procedure by using appropriate starting materials. HPLC: 86.52 %; t_{R} = 0.654 min; LCMS (ESI) m/z 359 [MH]⁺; ¹H NMR (CD₃OD): δ 8.86 (d, *J* = 7.2 Hz, 1H), 7.91 (s, 1H), 7.76 (d, *J* = 7.0 Hz, 1 H), 7.65 (d, *J* = 15.8 Hz, 1 H), 6.81 (d, *J* = 15.7 Hz, 1 H), 4.88 (s, 2H), 1.91 - 1.87 (m, 4H), 1.58 (s, 9H) 1.05 (t, *J* = 7.5 Hz, 6H).

### Example 3

### 3-[2-tert-Butyl-3-(tert-butylamino-methyl)-imidazo[1,2-a]pyridin-7-yl]-N-hydroxy-acrylamide (Compound 3)

The titled compound was prepared according to the procedures described in the general synthetic procedure by using appropriate starting materials. HPLC: 94.13 %; t_{R} = 0.541 min; LCMS (ESI) m/z 345.07 [MH]⁺; ¹H NMR (CD₃OD): δ 8.71 (d, *J* = 7.2 Hz, 1H), 7.89 (s, 1H), 7.71 (d, *J* = 6.0 Hz, 1H), 7.64 (d, *J* = 6.0 Hz, 1H), 6.79 (d, *J* = 15.7 Hz, 1H), 4.88 - 4.73 (m, 2H), 1.59 (s, 9H), 1.57 (s, 9H).

### Example 4

### 3-(2-Butyl-3-butylaminomethyl-imidazo[1,2-a]pyridin-7-yl)-N-hydroxy-acrylamide (Compound 4)

The titled compound was prepared according to the procedures described in the general synthetic procedure by using appropriate starting materials. HPLC: 99.9 %; t_{R} = 0.891 min; LCMS (ESI) m/z 345 [MH]⁺.

### Example 5

### 3-[2-Butyl-3-(tert-butylamino-methyl)-imidazo[1,2-a]pyridin-7-yl]-N-hydroxy-acrylamide (Compound 5)

The titled compound was prepared according to the procedures described in the general synthetic procedure by using appropriate starting materials. HPLC: 98.53 %; t_{R} = 0.673 min; LCMS (ESI) m/z 345 [MH]⁺; ¹H NMR (CD3OD): δ 8.86 (d, *J* = 6.8 Hz, 1H), 7.99 (s, 1H), 7.78 (d, *J* = 6.8 Hz, 1H), 7.56 (d, *J* = 15.7 Hz, 1H), 6.76 (d, *J* = 15.7 Hz, 1H), 4.87 (masked peaks, 2H), 3.05 (t, *J* = 7.9 Hz, 2H), 1.80 - 1.88 (m, 2H), 1.49 - 1.67 (m, 11 H), 1.04 (t, *J* = 7.3 Hz, 3H).

### Example 6

### 3-(2-Butyl-3-dipropylaminomethyl-imidazo[1,2-a]pyridin-7-yl)-N-hydroxy-acrylamide (compound 6)

The titled compound was prepared according to the procedures described in the general synthetic procedure by using appropriate starting materials. HPLC: 99 %; t_{R} = 1.169 min; LCMS (ESI) m/z 373 [MH]⁺.

### Example 7

### 3-(2-Butyl-3-diethylaminomethyl-imidazo[1,2-a]pyridin-7-yl)-N-hydroxy-acrylamide (compound 7)

The titled compound was prepared according to the procedures described in the general synthetic procedure by using appropriate starting materials. HPLC: 99 %; t_{R} = 0.537 min; LCMS (ESI) m/z 345 [MH]⁺.

### Example 8

### 3-{2-Butyl-3-[(butyl-methyl-amino)-methyl]-imidazo[1,2-a]pyridin-7-yl}-N-hydroxy-acrylamide (compound 8)

The titled compound was prepared according to the procedures described in the general synthetic procedure by using appropriate starting materials. HPLC: 99 %; t_{R} = 1.154 min; LCMS (ESI) m/z 359 [MH]⁺.

### Example 9

### 3-{2-Butyl-3-[(butyl-ethyl-amino)-methyl]-imidazo[1,2-a]pyridin-7-yl}-N-hydroxy-acrylamide (compound 9)

The titled compound was prepared according to the procedures described in the general synthetic procedure by using appropriate starting materials. HPLC: 99 %; t_{R} = 1.248 min; LCMS (ESI) m/z 373 [MH]⁺.

### Example 10

### 3-{2-Butyl-3-[(butyl-propyl-amino)-methyl]-imidazo[1,2-a]pyridin-7-yl}-N-hydroxy-acrylamide (compound 10)

The titled compound was prepared according to the procedures described in the general synthetic procedure by using appropriate starting materials. HPLC: 99 %; t_{R} = 1.429 min; LCMS (ESI) m/z 387 [MH]⁺.

### Example 11

### 3-(2-tert-Butyl-3-diethylaminomethyl-imidazo[1,2-a]pyridin-7-yl)-N-hydroxy-acrylamide (compound 11)

The titled compound was prepared according to the procedures described in the general synthetic procedure by using appropriate starting materials. HPLC: 99 %; t_{R} = 0.652 min; LCMS (ESI) m/z 345 [MH]⁺.

### Example 12

### 3-(3-Dibutylaminomethyl-2-methyl-imidazo[1,2-a]pyridin-7-yl)-N-hyroxy-acrylamide (compound 12)

The titled compound was prepared according to the procedures described in the general synthetic procedure by using appropriate starting materials. HPLC: 98.00 %; t_{R} = 0.964 min; LCMS (ESI) m/z 359 [MH]⁺; ¹H NMR (CD₃OD): δ 8.73 (s, 1H), 7.96 (s, 1H), 7.67 - 7.62 (m, 2H), 6.79 (d, *J* = 15.8 Hz, 1 H), 4.91 (masked peaks, 2H), 3.26 (t, *J* = 1.6 Hz, 4H), 2.65 (s, 3H), 1.83 -1.77 (m, 4H), 1.46 -1.38 (m, 4H), 1.04 (t, *J* = 8.0 Hz, 6H).

### Example 13

### 3-{2-tert-Butyl-3-[(ethyl-propyl-amino)-methyl]-imidazo[1,2-a]pyridin-7-yl}-N-hydroxy-acrylamide (compound 13)

The titled compound was prepared according to the procedures described in the general synthetic procedure by using appropriate starting materials. HPLC: 99 %; t_{R} = 0.631 min; LCMS (ESI) m/z 359 [MH]⁺.

### Example 14

### 3-(2-Butyl-3-dimethylaminomethyl-imidazo[1,2-a]pyridin-7-yl)-N-hydroxy-acrylamide (compound 14)

The titled compound was prepared according to the procedures described in the general synthetic procedure by using appropriate starting materials. HPLC: 99 %; t_{R} = 0.477 min; LCMS (ESI) m/z 317 [MH]⁺.

### Example 15

### 3-(2-tert-Butyl-3-ethylaminomethyl-imidazo[1,2-a]pyridin-7-yl)-N-hydroxy-acrylamide (compound 15)

The titled compound was prepared according to the procedures described in the general synthetic procedure by using appropriate starting materials. HPLC: 99 %; t_{R} = 0.379 min; LCMS (ESI) m/z 317 [MH]⁺.

### Example 16

### Preparation of 3-{2-tert-Butyl-3-[(butyl-ethyl-amino)-methyl]-imidazo[1,2-a]pyridin-7-yl}-N-hydroxy-acrylamide (Compound 16)

The intermediate **XVII** was prepared according to the 3-steps procedure described in the general synthetic procedure by using appropriate starting materials. **XVII** was then subjected to reductive amination described below.

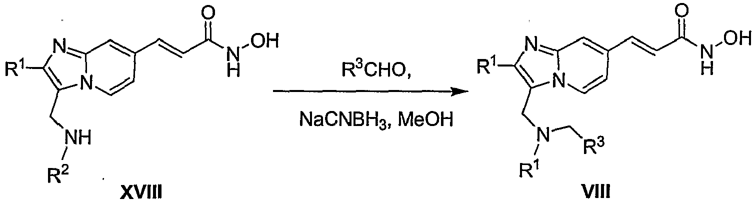

To a stirred solution of the amine **XVII** (1.0 equiv.), **R³CHO** (2.0 equiv.) and MeOH was added NaCNBH₃ (3.0 equiv.) and the mixture was then stirred at room temperature for 2 h. When the reaction has completed, the contents were purified by reversed-phase prep-HPLC immediately.

### 3-{2-tert-Butyl-3-[(butyl-ethyl-amino)-methyl]-imidazo[1,2-a]pyridin-7-yl}-N-hydroxy-acrylamide (Compound 16)

HPLC: 99 %; t_{R} = 0.976 min; LCMS (ESI) m/z 373 [MH]⁺.

### Example 17

### 3-{2-tert-Butyl-3-[(ethyl-isobutyl-amino)-methyl]-imidazo[1,2-a]pyridin-7-yl}-N-hydroxy-acrylamide (compound 17)

The titled compound was prepared according to the procedures described in the general synthetic procedure by using appropriate starting materials. HPLC: 99 %; t_{R} = 1.159 min; LCMS (ESI) m/z 373 [MH]⁺.

### Example 18

### 3-{2-tert-Butyl-3-[(ethyl-pentyl-amino)-methyl]-imidazo[1,2-a]pyridin-7-yl}-N-hydroxy-acrylamide (compound 18)

The titled compound was prepared according to the procedures described in the general synthetic procedure by using appropriate starting materials. HPLC: 99 %; t_{R} = 1.337 min; LCMS (ESI) m/z 387 [MH]⁺.

### Example 19

### 3-(2-tert-Butyl-3-{[ethyl-(2-methyl-butyl)-amino]-methyl}-imidazo[1,2-a]pyridin-7-yl)-N-hydroxy-acrylamide (compound 19)

The titled compound was prepared according to the procedures described in the general synthetic procedure by using appropriate starting materials. HPLC: 99 %; t_{R} = 1.446 min; LCMS (ESI) m/z 387 [MH]⁺.

### Example 20

### 3-(2-tert-Butyl-3-{[ethyl-(3-methyl-butyl)-amino]-methyl}-imidazo[1,2-a]pyridin-7-yl)-N-hydroxy-acrylamide (compound 20)

The titled compound was prepared according to the procedures described in the general synthetic procedure by using appropriate starting materials. HPLC: 99 %; t_{R} = 0.275 min; LCMS (ESI) m/z 387 [MH]⁺.

### Example 21

### 3-{2-tert-Butyl-3-[(ethyl-hexyl-amino)-methyl]-imidazo[1,2-a]pyridin-7-yl}-N-hydroxy-acrylamide (compound 21)

The titled compound was prepared according to the procedures described in the general synthetic procedure by using appropriate starting materials. HPLC: 99 %; t_{R} = 1.647 min; LCMS (ESI) m/z 401 [MH]⁺.

### Example 22

### 3-(2-tert-Butyl-3-{[ethyl-(2-methyl-pentyl)-amino]-methyl}-imidazo[1,2-a]pyridin-7-yl)-N-hydroxy-acrylamide (compound 22)

The titled compound was prepared according to the procedures described in the general synthetic procedure by using appropriate starting materials. HPLC: 99 %; t_{R} = 1.752 min; LCMS (ESI) m/z 401 [MH]⁺.

### Example 23

### 3-(2-tert-Butyl-3-{[(3,3-dimethyl-butyl)-ethyl-amino]-methyl}-imidazo[1,2-a]pyridin-7-yl)-N-hydroxy-acrylamide (compound 23)

The titled compound was prepared according to the procedures described in the general synthetic procedure by using appropriate starting materials. HPLC: 99 %; t_{R} = 1.503 min; LCMS (ESI) m/z 400 [MH]⁺.

### Example 24

### 3-(2-tert-Butyl-3-{[ethyl-(2-ethyl-butyl)-amino]-methyl}-imidazo[1,2-a]pyridin-7-yl)-N-hydroxy-acrylamide (compound 24)

The titled compound was prepared according to the procedures described in the general synthetic procedure by using appropriate starting materials. HPLC: 99 %; t_{R} = 1.692 min; LCMS (ESI) m/z 401 [MH]⁺.

### Example 25

### 3-(2-tert-Butyl-3-[(ethyl-heptyl-amino)-methyl-imidazo[1,2-a]pyridin-7-yl}-N-hydroxy-acrylamide (compound 25)

The titled compound was prepared according to the procedures described in the general synthetic procedure by using appropriate starting materials. HPLC: 99 %; t_{R} = 1.943 min; LCMS (ESI) m/z 415 [MH]⁺.

### Example 26

### 3-(2-tert-Butyl-3-{[ethyl-(3-methylsulfanyl-propyl)-amino]-methyl}-imidazo[1,2-a]pyridin-7-yl)-N-hydroxy-acrylamide (compound 26)

The titled compound was prepared according to the procedures described in the general synthetic procedure by using appropriate starting materials. HPLC: 99 %; t_{R} = 1.133 min; LCMS (ESI) m/z 405 [MH]⁺.

### Example 27

### 3-{2-tert-Butyl-3-[(cyclopropylmethyl-ethyl-amino)-methyl]-imidazo[1,2-a]pyridin-7-yl}-N-hydroxy-acrylamide (compound 27)

The titled compound was prepared according to the procedures described in the general synthetic procedure by using appropriate starting materials. HPLC: 99 %; t_{R} = 0.695 min; LCMS (ESI) m/z 371 [MH]⁺.

### Example 28

### 3-{2-tert-Butyl-3-[(cyclohexylmethyl-ethyl-amino)-methyl]-imidazo[1,2-a]pyridin-7-yl}-N-hydroxy-acrylamide (compound 28)

The titled compound was prepared according to the procedures described in the general synthetic procedure by using appropriate starting materials. HPLC: 99 %; t_{R} = 1.699 min; LCMS (ESI) m/z 413 [MH]⁺.

### Example 29

### 3-{3-[(Butyl-ethyl-amino)-methyl]-2-methyl-imidazo[1,2-a]pyridin-7-yl}-N-hydroxy-acrylamide (compound 29)

The titled compound was prepared according to the procedures described in the general synthetic procedure by using appropriate starting materials. HPLC: 99 %; t_{R} = 0.412 min; LCMS (ESI) m/z 331 [MH]⁺; ¹H NMR (CD₃OD): δ 8.67 (d, *J* = 7.0 Hz, 1 H), 7.91 (s, 1 H), 7.65 (d, *J* = 15.6 Hz, 1 H), 7.60 (d, *J* = 7.2 Hz, 1 H), 6.76 (d, *J* = 16.0 Hz, 1 H), 4.95 (masked peaks, 2H), 3.36 - 3.37 (m, 2H), 3.25 (masked peaks, 2H), 2.63 (s, 3H), 1.83 - 1.75 (m, 2H), 1.47 -1.38 (m, 5H), 1.01 (t, *J* = 7.4 Hz, 3H).

### Example 30

### 3-{3-[(Ethyl-propyl-amino)-methyl]-2-methyl-imidazo[1,2-a]pyridin-7-yl}-N-hydroxy-acrylamide (compound 30)

The titled compound was prepared according to the procedures described in the general synthetic procedure by using appropriate starting materials. HPLC: 97.28 %; t_{R} = 0.316 min; LCMS (ESI) m/z 317 [MH]⁺; ¹H NMR (CD₃OD): δ 8.72 (d, *J* = 6.8 Hz, 1H), 7.94 (s, 1H), 7.68 - 7.61 (m, 2H), 6.80 (d, *J* = 15.6 Hz, 1H), 4.95 (masked peaks, 2H), 3.64 - 3.60 (m, 2H), 3.27 - 3.25 (m, 2H), 2.64 (s, 3H), 1.85 - 1.82 (m, 2H), 1.38 (t, *J* = 7.4 Hz, 3H), 1.03 (t, *J* = 7.3 Hz, 3H).

### Example 31

### 3-(3-{[Ethyl-(2-ethyl-butyl)-amino]-methyl}-2-methyl-imidazo[1,2-a]pyridin-7-yl)-N-hydroxy-acrylamide (compound 31)

The titled compound was prepared according to the procedures described in the general synthetic procedure by using appropriate starting materials. HPLC: 99 %; t_{R} = 0.963 min; LCMS (ESI) m/z 359 [MH]⁺; ¹H NMR (CD₃OD): δ 8.70 (d, *J* = 7.2 Hz, 1H), 7.92 (s, 1H), 7.69 - 7.64 (m, 2H), 6.78 (d, *J* = 15.8 Hz, 1 H), 4.61 (brs, 2H), 3.14 - 3.12 (m, 2H), 2.91 - 2.86 (m, 2H), 2.60 (s, 3H), 1.68 (brs, 1 H), 1.42 -1.35 (m, 7H), 0.86 (t, *J* = 7.2 Hz, 3H).

### Example 32

### 3-{2-tert-Butyl-3-[(isopropyl-propyl-amino)-methyl]-imidazo[1,2-a]pyridin-7-yl}-N-hydroxy-acrylamide (compound 32)

The titled compound was prepared according to the procedures described in the general synthetic procedure by using appropriate starting materials. HPLC: 95.11 %; t_{R} = 0.756 min; LCMS (ESI) m/z 373 [MH]⁺; ¹H NMR (CD₃OD) δ 8.81 (s, 1 H), 8.02 (s, 1 H), 7.91 (s, 1H), 7.59 (d, *J* = 15.9 Hz, 1 H), 6.79 (d, *J* = 15.7 Hz, 1 H), 5.04 - 4.76 (m, 2H), 3.91 (s, 1 H), 1.86 -1.28 (m, 19H), 0.83 (s, 3H).

### Example 33

### 3-(-tert-Butyl-3-{[(2,2-dimethyl-propyl)-methyl-amino]-methyl}-imidazo[1,2-a]pyridin-7-yl)-N-hydroxy-acrylamide (compound 33)

The titled compound was prepared according to the procedures described in the general synthetic procedure by using appropriate starting materials. HPLC: 99 %; t_{R} = 1.318 min; LCMS (ESI) m/z 373 [MH]⁺; ¹H NMR (CD₃OD): δ 8.82 (d, *J* = 6.9 Hz, 1 H), 7.83 (s, 1 H), 7.65 (d, *J* = 6.9 Hz, 1 H), 7.59 (d, *J* = 15.8 Hz, 1 H), 6.75 (d, *J* = 15.7 Hz, 1 H), 4.85 - 4.70 (m, 2H), 4.26 (s, 2H), 2.56-2.46 (m, 3H), 1.49 (s, 9H), 0.71 (s, 9H).

### Example 34

### 3-{2-tert-Butyl-3-[(butyl-methyl-amino)-methyl]-imidazo[1,2-a]pyridin-7-yl}-N-hydroxy-acrylamide (compound 34)

The titled compound was prepared according to the procedures described in the general synthetic procedure by using appropriate starting materials. HPLC: 99 %; t_{R} = 0.841 min; LCMS (ESI) m/z 359 [MH]⁺; ¹H NMR (CD₃OD): δ 8.78 (d, *J* = 7.2 Hz, 1H), 7.87 (s, 1H), 7.65 - 7.61 (m, 3H), 6.76 (d, *J* = 15.9 Hz, 1 H), 4.89 - 4.74 (m, 2H), 2.74 (s, 3H), 2.60 (s, 2H), 1.54 (s, 9H), 1.34 (m, 2H), 0.93 (t, *J* = 7.5 Hz, 3H).

### Example 35

### 3-(2-tert-Butyl-3-{[(2-ethyl-butyl)-methyl-amino]-methyl}-imidazo[1,2-a]pyridin-7-yl)-N-hydroxy-acrylamide (compound 35)

The titled compound was prepared according to the procedures described in the general synthetic procedure by using appropriate starting materials. HPLC: 99 %; t_{R} = 1.454 min; LCMS (ESI) m/z 387 [MH]⁺; ¹H NMR (CD₃OD): δ 8.78 (d, *J* = 7.3 Hz, 1H), 7.86 (s, 1H), 7.66 - 7.62 (m, 2H), 6.77 (d, *J =* 15.7 Hz, 1 H), 4.89 - 4.74 (m, 2H), 4.41 (s, 2H), 2.60 (s, 2H), 2.49 (s, 3H) 1.53 (s, 9H), 1.32 -1.20 (m, 4H), 0.75 (t, *J =* 7.5 Hz, 6H).

### Example 36

### 3-(2-tert-Butyl-3-{[methyl-(3-methyl-butyl)-amino]-methyl}-imidazo[1,2-a]pyridin-7-yl)-N-hydroxy-acrylamide (compound 36)

The titled compound was prepared according to the procedures described in the general synthetic procedure by using appropriate starting materials. HPLC: 99 %; t_{R} = 1.141 min; LCMS (ESI) m/z 373 [MH]⁺.

### Example 37

### 3-(2-tert-Butyl-3-{[(3,3-dimethyl-butyl)-methyl-amino]-methyl}-imidazo[1,2-a]pyridin 7-yl)-N-hydroxy-acrylamide (compound 37)

The titled compound was prepared according to the procedures described in the general synthetic procedure by using appropriate starting materials. HPLC: 99 %; t_{R} = 1.360 min; LCMS (ESI) m/z 387 [MH]⁺.

### Example 38

### 3-(3-{[(2,2-Dimethyl-propyl)-propyl-amino]-methyl}-2-methyl-imidazo[1,2-a]pyridin-7-yl)-N-hydroxy-acrylamide (compound 38)

The titled compound was prepared according to the procedures described in the general synthetic procedure by using appropriate starting materials. HPLC: 99 %; t_{R} = 0.816 min; LCMS (ESI) m/z 359 [MH]⁺; ¹H NMR (CD₃OD): δ 8.71 (d, *J* = 7.2 Hz, 1H), 7.99 (s, 1H), 7.72 - 7.65 (m, 2H), 6.80 (d, *J* = 15.7 Hz, 1 H), 4.90 (masked peaks, 2H), 2.73 (brs, 2H), 2.59 (s, 3H), 2.43 (brs, 2H), 1.76 -1.74 (m, 2H), 0.95 (t, *J* = 7.2 Hz, 3H), 0.72 (s, 9H).

### Example 39

### 3-(3-{[(2,2-Dimethyl-propyl)-ethyl-amino]-methyl}-2-methyl-imidazo[1,2-a]pyridin-7-yl)-N-hydroxy-acrylamide (compound 39)

The titled compound was prepared according to the procedures described in the general synthetic procedure by using appropriate starting materials. HPLC: 99 %; t_{R} = 0.623 min; LCMS (ESI) m/z 345 [MH]⁺; ¹H NMR (CD₃OD): δ 8.72 (d, *J* = 7.2 Hz, 1 H), 7.94 (s, 1 H), 7.66 - 7.71 (m, 2H), 6.80 (d, *J* = 15.7 Hz, 1H), 4.84 (masked peaks, 2H), 4.28 (brs, 2H), 2.96 (brs, 2H), 2.61 (s, 3H), 1.30 (t, J = 4 Hz, 3H), 0.79 (s, 9H).

### Example 40

### 3-(2-Butyl-3-[(ethyl-isobutyl-amino)-methyl]-imidazo[1,2-a]pyridin-7-yl}-N-hydroxy-acrylamide (compound 40)

The titled compound was prepared according to the procedures described in the general synthetic procedure by using appropriate starting materials. HPLC: 99 %; t_{R} = 1.078 min; LCMS (ESI) m/z 373 [MH]⁺; ¹H NMR (CD₃OD): δ 8.64 (d, *J* = 7.2 Hz, 1 H), 7.85 (s, 1 H), 7.63 - 7.57 (m, 2H), 6.72 (d, *J* = 15.7 Hz, 1 H), 4.47 (brs, 2H), 3.00 (brs, 2H), 2.89 (t, *J =* 8.1 Hz, 2H), 1.95 (brs, 1 H) 1.74 (q, *J =* 7.6 Hz, 2H), 1.41 (q, *J =* 7.6 Hz, 2H), 1.25 - 1.23 (m, 3H), 0.96 - 0.87 (m, 9H).

### Example 41

### 3-(2-Butyl-3-{(ethyl-(2-methyl-pentyl)-amino]-methyl}-imidazo[1,2-a]pyridin-7-yl)-N-hydroxy-acrylamide (compound 41)

The titled compound was prepared according to the procedures described in the general synthetic procedure by using appropriate starting materials. HPLC: 99 %; t_{R} = 1.338 min; LCMS (ESI) m/z 387 [MH]⁺; ¹H NMR (CD₃OD): δ 8.61 (d, *J* = 7.1 Hz, 1H), 7.83 (s, 1H), 7.63 - 7.55 (m, 2H), 6.72 (d, *J =* 15.8 Hz, 1H), 4.91 - 4.66 (masked peaks, 2H), 2.95 (brs, 2H), 2.88 (t, *J* = 8.1 Hz, 2H), 1.73 (q, *J* = 7.5 Hz, 4H), 1.43 - 1.36 (m, 4H), 1.23 (masked peaks, 3H), 1.10-1.00 (m, 1 H), 0.92 (t, *J =* 7.3 Hz, 3H), 0.82 - 0.77 (m, 6H).

### Example 42

### 3-(2-Butyl-3-{[ethyl-(3-methyl-butyl)-amino]-methyl}-imidazo[1,2-a]pyridin-7-yl)-N-hydroxy-acrylamide (compound 42)

The titled compound was prepared according to the procedures described in the general synthetic procedure by using appropriate starting materials. HPLC: 99 %; t_{R} = 1.428 min; LCMS (ESI) m/z 387 [MH]⁺; ¹H NMR (CD₃OD): δ 8.58 (d, *J* = 7.1 Hz, 1H), 7.82 (s, 1H), 7.60 (d, *J =* 15.7 Hz, 1H), 7.51(d, *J*= 6.0 Hz, 1H), 6.69 (d, *J* = 15.7 Hz, 1H), 4.83 - 4.71 (masked peaks, 2H), 3.30 - 3.19 (m, 4H), 3.20 - 3.00 (m, 2H), 2.89 (t, *J* = 7.8 Hz, 1 H) 1.90 -1.70 (m, 2H), 1.61 -1.50 (m, 3H), 1.43 (q, *J* = 7.6 Hz, 2H), 1.32 (t, *J* = 7.2 Hz, 3H), 0.97 - 0.90 (m, 9H).

### Example 43

### 3-(2-Butyl-3-{[(2,2-dimethyl-propyl)-ethyl-amino]-methyl}-imidazo[1,2-a]pyridin-7-yl)-N-hydroxy-acrylamide (compound 43)

The titled compound was prepared according to the procedures described in the general synthetic procedure by using appropriate starting materials. HPLC: 90.41 %; t_{R} = 1.344 min; LCMS (ESI) m/z 387 [MH]⁺; ¹H NMR (CD₃OD): δ 8.80 - 8.60 (m, 1 H), 7.90 (s, 1 H), 7.80 - 7.65 (m, 2H), 6.73(d, *J* = 15.7 Hz, 1H), 4.88 - 4.73 (masked peaks, 2H), 3.98 (brs, 2H), 2.90 (t, *J* = 7.8 Hz, 2H), 2.75 (brs, 2H), 1.73 (q, *J* = 7.4 Hz, 2H), 1.41 (q, *J* = 7.6 Hz, 2H), 1.20 (m, 3H), 0.95 (t, *J* = 7.4 Hz, 3H), 0.61 (brs, 9H).

### Example 44

### 3-(2-Butyl-3-{[ethyl-(2-methyl-pentyl)-amino]-methyl}-imidazo[1,2-a]pyridin-7-yl)-N-hydroxy-acrylamide (compound 44)

The titled compound was prepared according to the procedures described in the general synthetic procedure by using appropriate starting materials. HPLC: 99 %; t_{R} = 1.638 min; LCMS (ESI) m/z 401 [MH]⁺; ¹H NMR (CD₃OD): δ 8.70 (d, *J* = 7.3 Hz, 1 H), 7.89 (s, 1 H), 7.68 - 7.57 (m, 2H), 6.72 (d, *J* = 15.7 Hz, 1H), 4.40 (s, 2H), 3.10 - 3.00 (m, 2H), 2.89 (t, *J* = 7.5 Hz, 2H), 2.40 - 2.80 (m, 2H), 1.73 (q, *J* = 7.8 Hz, 2H), 1.42 (q, *J* = 7.6 Hz, 2H), 1.27 - 1.23 (m, 7H), 0.93 (t, *J* = 7.4 Hz, 4H), 0.84 - 0.77 (m, 6H).

### Example 45

### 3-(2-Butyl-3-{[(3,3-dimethyl-butyl)-ethyl-amino]-methyl)-imidazo[1,2-a]pyridin-7-yl)-N-hydroxy-acrylamide (compound 45)

The titled compound was prepared according to the procedures described in the general synthetic procedure by using appropriate starting materials. HPLC: 99 %; t_{R} = 1.630 min; LCMS (ESI) m/z 401 [MH]⁺; ¹H NMR (CD₃OD) δ 8.71 (d, *J* = 7.8 Hz, 1 H), 7.91 (s, 1 H), 7.67 - 7.58 (m, 2H), 6.73 (d, *J* = 15.7 Hz, 1 H), 4.89 - 4.74 (masked peaks, 2H), 2.93 (t, *J* = 7.7 Hz, 2H), 1.77 (m, 2H), 1.68 (m, 2H), 1.44 (q, *J* = 7.6 Hz, 2H), 1.33 (t, *J* = 7.2 Hz, 2H), 0.99 - 0.89 (m, 12H).

### Example 46

### 3-(2-Butyl-3-{[ethyl-(2-ethyl-butyl)-amino]-methyl}-imidazo[1,2-a]pyridin-7-yl)-N-hydroxy-acrylamide (compound 46)

The titled compound was prepared according to the procedures described in the general synthetic procedure by using appropriate starting materials. HPLC: 96.23 %; t_{R} = 1.586 min; LCMS (ESI) m/z 401 [MH]⁺; ¹H NMR (CD₃OD): δ 8.64 (d, *J* = 7.2 Hz, 1H), 7.90 (s, 1 H), 7.64 - 7.55 (m, 2H), 6.73 (d, *J* = 15.7 Hz, 1 H), 4.36 (brs, 2H), 2.89 (t, *J* = 7.5 Hz, 2H), 1.73 (q, *J =* 7.8 Hz, 2H), 1.41 (q, *J* = 7.6 Hz, 2H), 1.30 -1.25 (m, 4H), 0.95 (t, *J* = 7.4 Hz, 3H), 0.75 - 0.72 (m, 6H).

### Example 47

### 3-(2-Butyl-3-{[ethyl-(3,5,5-trimethyl-hexyl)-amino]-methyl}-imidazo[1,2-a]pyridin-7-yl)-N-hydroxy-acrylamide (compound 47)

The titled compound was prepared according to the procedures described in the general synthetic procedure by using appropriate starting materials. HPLC: 96.82 %; t_{R} = 2.298 min; LCMS (ESI) m/z 443 [MH]⁺; ¹H NMR (CD₃OD): δ 8.72 (d, *J* = 7.6 Hz, 1H), 7.90 (s, 1 H), 7.62 - 7.53 (m, 2H), 6.72 (d, *J* = 15.7 Hz, 1 H), 4.84 - 4.74 (masked peaks, 2H), 2.92 (m, 2H), 1.80 - 1.60 (m, 4H), 1.60 -1.50 (m, 2H), 1.44 (q, *J* = 7.6 Hz, 2H), 1.36 (t, *J* = 7.2 Hz, 3H), 0.96 - 0.88 (m, 5H), 0.84 (s, 9H).

### Example 48

### 3-[3-(tert-Butylamino-methyl)-2-propyl-imidazo[1,2-a]pyridin-7-yl]-N-hydroxy-acrylamide (compound 48)

The titled compound was prepared according to the procedures described in the general synthetic procedure by using appropriate starting materials. HPLC: 99 %; LC-MS (ESI) m/z 331 [MH]⁺; ¹H NMR (CD₃OD) δ 8.71 (d, *J* = 7*.*1 Hz, 1 H), 7.97 (s, 1H), 7.73 (d, *J* = 6.8 Hz, 1H), 7.63 (d, *J* = 15.7 Hz, 1H), 6.78 (d, *J* = 15.7 Hz, 1H), 4.78 (s, 2H), 2.98 (t, 2H), 1.90 - 1.84 (m, 2H), 1.59 (brs, 9H), 1.10 (t, 3H).

### Example 49

### 3-(3-{[Ethyl-(3-methyl-butyl)-amino]-methyl}-2-methyl-imidazo[1,2-a]pyridin-7-yl)-N-hydroxy-acrylamide (compound 49)

The titled compound was prepared according to the procedures described in the general synthetic procedure by using appropriate starting materials. HPLC: 99 %; t_{R} = 0.622 min; LCMS (ESI) m/z 345 [MH]⁺; ¹H NMR (CD₃OD): δ 8.72 (brs, 1H), 7.92 (s, 1H), 7.70 - 7.60 (m, 2H), 6.78 (d, *J =* 14.5 Hz, 1 H), 4.84 (masked peaks, 2H), 3.30 - 3.24 (m, 4H), 2.61 (s, 3H), 1.66 -1.59 (m, 3H), 1.37 -1.24 (m, 3H), 0.95 - 0.90 (m, 6H).

### Example 50

### 3-(3-{[(3,3-Dimethyl-butyl)-ethyl-amino]-methyl}-2-methyl-imidazo[1,2-a]pyridin-7-yl)-N-hydroxy-acrylamide (compound 50)

The titled compound was prepared according to the procedures described in the general synthetic procedure by using appropriate starting materials. HPLC: 94.31 %; t_{R} = 1.176 min; LCMS (ESI) m/z 359 [MH]⁺; ¹H NMR (CD₃OD): δ 8.66 (brs, 1H), 7.90 (s, 1H), 7.69 - 7.62 (m, 2H), 6.77 (d, *J* = 15.7 Hz, 1 H), 4.75 (masked peaks, 2H), 3.27 - 3.20 (m, 4H), 2.56 (d, *J* = 1.0 Hz, 3H), 1.63 -1.62 (m, 2H), 1.32 -1.24 (m, 3H), 0.91 (s, 9H).

### Example 51

### 3-(3-{[Ethyl-(3-methyl-butyl)-amino]-methyl}-2-propyl-imidazo[1,2-a]pyridin-7-yl)-N-hydroxy-acrylamide compound 51)

The titled compound was prepared according to the procedures described in the general synthetic procedure by using appropriate starting materials. HPLC: 95.68 %; LCMS (ESI) m/z 373 [MHt; ¹H NMR (CD₃OD δ 8.70 (d, *J* = 7.3 Hz, 1 H), 7.92 (s, 1 H), 7.68 (d, *J* = 15.7 Hz, 1H), 7.63 (dd, *J =* 1.4, 7.2 Hz, 1H), 6.78 (d, *J* = 15.7 Hz, 1H), 2.95 (t, 2H), 1.94 - 1.82 (m, 2H), 1.70 - 1.62 (brs, 3H), 1.39 (t, 3H), 1.08 (t, 3H), 0.98 (t, 9H).

### Example 52

### 3-(3-{[(3,3-Dimethyl-butyl)-ethyl-amino]-methyl}-2-propyl-imidazo[1,2-a]pyridin-7-yl)-N-hydroxy-acrylamide (compound 52)

The titled compound was prepared according to the procedures described in the general synthetic procedure by using appropriate starting materials. HPLC: 95.13 %; LCMS (ESI) m/z 387 [MH]⁺; ¹H NMR (CD₃OD) δ 8.71 (d, *J* = 7.1 Hz, 1 H), 7.92 (s, 1 H), 7.68 (d, *J =* 15.7 Hz, 1 H), 7.63 (dd, *J =7.3* Hz, 1 H), 6.78 (d, *J* = 15.7 Hz, 1 H), 2.96 (t, 2H), 1.91 - 1.85 (m, 2H), 1.73 -1.69 (m, 2H), 1.38 (t, 3H), 1.08 (t, 3H), 0.99 (t, 10H).

### Example 53

### 3-[3-(tert-Butylamino-methyl)-2-pentyl-imidazo[1,2-a]pyridin-7-yl]-N-hydroxy-acrylamide (compound 53)

The titled compound was prepared according to the procedures described in the general synthetic procedure by using appropriate starting materials. HPLC: 89.81 %; t_{R} = 1.192 min; LCMS (ESI) m/z 359 [MH]⁺; ¹H NMR (CD₃OD): δ 8.56 (brs, 1H), 7.86 (s, 1H), 7.61 - 7.57 (m, 2H), 6.69 (d, *J* = 15.8 Hz, 1H), 4.70 (s, 2H), 2.90 (t, *J* = 8.1 Hz, 2H), 1.77 (brs, 2H), 1.51 (s, 9H), 1.42 -1.35 (m, 4H), 0.90 (t, *J* = 7.2 Hz, 3H).

### Example 54

### 3-{3-[(Butyl-ethyl-amino)-methyl]-2-pentyl-imidazo[1,2-a]pyridin-7-yl}-N-hydroxy-acrylamide(compound 54)

The titled compound was prepared according to the procedures described in the general synthetic procedure by using appropriate starting materials. HPLC: 95.63 %; t_{R} = 1.451 min; LCMS (ESI) m/z 387 [MH]⁺; ¹H NMR (CDₛOD): 8 8.60 (d, *J* = 7.2 Hz, 1H), 7.83 (s, 1 H), 7.65 - 7.53 (m, 2H), 6.69 (d, *J =* 15.7 Hz, 1 H), 4.87 - 4.72 (masked peaks, 2H), 3.18 - 3.00 (m, 2H), 2.88 (t, *J* = 8.0 Hz, 2H), 1.76 - 1.60 (m, 5H), 1.37 - 1.29 (m, 10H), 0.94 - 0.86 (m, 6H).

### Example 55

### 3-(3-{(Ethyl-(3-methyl-butyl)-amino]-methyl}-2-pentyl-imidazo[1,2-a]pyridin-7-yl)-N-hydroxy-acrylamide (compound 55)

The titled compound was prepared according to the procedures described in the general synthetic procedure by using appropriate starting materials. HPLC: 94.84 %; t_{R} = 1.681 min; LCMS (ESI) m/z 401 [MH]⁺; ¹H NMR (CD₃OD): δ 8.61 (d, *J* = 7.3 Hz, 1H), 7.84 (s, 1H), 7.62 (d, *J* = 8.2 Hz, 2H), 6.72 (d, *J* = 15.7 Hz, 1H), 3.20 - 3.19 (m, 2H), 2.90 (t, *J* = 8.0 Hz, 2H), 1.80 - 1.77 (m, 2H), 1.63 -1.55 (m, 2H), 1.39 - 1.37 (m, 4H), 1.33 (t, *J* = 7.2 Hz, 4H), 0.95 - 0.88 (m, 9H).

### Example 56

### 3-(3-{[(3,3-Dimethyl-butyl)-ethyl-amino]-methyl}-2-pentyl-imidazo[1,2-a]pyridin-7-yl)-N-hydroxy-acrylamide (compound 56)

The titled compound was prepared according to the procedures described in the general synthetic procedure by using appropriate starting materials. HPLC: 94.41 %; t_{R} = 1.871 min; LCMS (ESI) m/z 415 [MH]⁺; ¹H NMR (CD₃OD): δ 8.62 (d, *J* = 7.2 Hz, 1H), 7.87 (s, 1H), 7.61 (d, *J* = 15.8 Hz, 1 H), 7.55 (d, *J* = 7.1 Hz, 1 H), 6.74 (d, *J* = 15.9 Hz, 1 H), 4.84 - 4.74 (masked peaks, 2H), 3.21 - 3.19 (m, 2H), 2.91 (t, *J* = 7.8 Hz, 2H), 1.88 - 1.77 (m, 2H), 1.66 - 1.62 (m, 4H), 1.49 -1.30 (m, 7H), 1.03 - 0.85 (m, 12H).

### Example 57

### 3-{3-[(Butyl-ethyl-amino)-methyl]-2-propyl-imidazo[1,2-a]pyridin-7-yl}-N-hydroxy-acrylamide (compound 57)

The titled compound was prepared according to the procedures described in the general synthetic procedure by using appropriate starting materials. HPLC: 96.93 %; LCMS (ESI) m/z 359 [MH]⁺; ¹H NMR (CD₃OD) δ 8.71 (d, *J* = 7.2 Hz, 1 H), 7.93 (s, 1 H), 7.67 (d, *J* = 15.7 Hz, 1H), 7.63 (brs, 1H), 6.79 (d, *J =* 15.7 Hz, 1H), 3.24 - 3.22 (m, 2H), 2.96 (t, 2H), 1.90 - 1.84 (m, 2H), 1.82 -1.72 (m, 3H), 1.55 -1.30 (m, 8H), 1.08 (t, 3H), 1.02 - 0.96 (m, 5H).

### Example 58

### 3-[3-(tert-Butylamino-methyl)-2-isobutyl-imidazo[1,2-a]pyridin-7-yl]-N-hydroxy-acrylamide (compound 58)

The titled compound was prepared according to the procedures described in the general synthetic procedure by using appropriate starting materials. HPLC: 99 %; t_{R} = 0.716 min; LCMS (ESI) m/z 345 [MH]⁺; ¹H NMR (CD₃OD): δ 8.64 (d, *J* = 7.0 Hz, 1 H), 7.93 (s, 1 H), 7.58 - 7.67 (m, 2H), 6.73 (d, J = 15.8 Hz, 1H), 4.74 (s, 2H), 2.86 (d, *J =* 7.3 Hz, 2H), 2.17 - 2.21 (m, 1 H), 1.58 (s, 9H), 1.04 (d, *J* = 6.6 Hz, 6H).

### Example 59

### 3-{3-[(Butyl-ethyl-amino)-methyl]-2-isobutyl-imidazo[1,2-a]pyridin-7-yl}-N-hydroxy-acrylamide (compound 59)

The titled compound was prepared according to the procedures described in the general synthetic procedure by using appropriate starting materials. HPLC: 99 %; t_{R} = 0.934 min; LCMS (ESI) m/z 373 [MH]⁺; ¹H NMR (CD₃OD): δ 8.73 (s, 1H), 7.96 (s, 1H), 7.63 - 7.68 (m, 2H), 6.80 (d, *J* = 15.7 Hz, 1H), 4.87 (masked peaks, 2H), 3.31 (masked peaks, 4H), 2.88 (d, *J* = 7.1 Hz, 2H), 2.19 - 2.28 (m, 1 H), 1.79 (brs, 2H), 1.34 - 1.51 (m, 5H), 0.98 - 1.10 (m, 9H).

### Example 60

### 3-(3-{[Ethyl-(3-methyl-butyl)-amino]-methyl}-2-isobutyl-imidazo[1,2-a]pyridin-7-yl)-N-hydroxy-acrylamide (compound 60)

The titled compound was prepared according to the procedures described in the general synthetic procedure by using appropriate starting materials. HPLC: 99 %; t_{R} = 1.264 min; LCMS (ESI) m/z 387 [MH]⁺; ¹H NMR (CD₃OD): δ 8.74 (d, *J* = 7.1 Hz, 1H), 7.95 (s, 1H), 7.63 - 7.67 (m, 2H), 6.80 (d, *J* = 15.7 Hz, 1 H), 4.87 (masked peaks, 2H), 3.31 (masked peaks, 4H), 2.88 (d, *J* = 7.4 Hz, 2H), 2.19 - 2.26 (m, 1 H), 1.65 - 1.72 (m, 3H), 1.39 (t, *J* = 7.2 Hz, 3H), 0.93 - 0.97 (m, 12H).

### Example 61

### 3-(3-{[(3,3-Dimethyl-butyl)-ethyl-amino]-methyl}-2-isobutyl-imidazor[1,2-a]pyridin-7-yll-N-hydroxy-acrylamide (compound 61)

The titled compound was prepared according to the procedures described in the general synthetic procedure by using appropriate starting materials. HPLC: 99 %; t_{R} = 1.479 min; LCMS (ESI) m/z 401 [MH]⁺; ¹H NMR (CD₃OD): δ 8.74 (d, *J* = 5.0 Hz, 1 H), 7.95 (s, 1 H), 7.64 - 7.68 (m, 2H), 6.79 (d, *J* = 15.8 Hz, 1 H), 4.87 (masked peaks, 2H), 3.31 (masked peaks, 4H), 2.88 (d, *J* = 7.2 Hz, 2H), 2.21 - 2.24 (m, 1 H), 1.74 (brs, 2H), 1.38 (t, *J =* 6.9 Hz, 3H), 0.95 -1.10 (m, 15H).

### Example 62

### 3-[3-(tert-Butylamino-methyl)-2-ethyl-imidazo[1,2-a]pyridin-7-yl]-N-hydroxy-acrylamide (compound 62)

The titled compound was prepared according to the procedures described in the general synthetic procedure by using appropriate starting materials. HPLC: 99 %; t_{R} = 1.264 min; LCMS (ESI) m/z 317 [MH]⁺; ¹H NMR (CD₃OD): δ 8.64 (d, *J* = 6.9 Hz, 1H), 7.94 (s, 1H), 7.66 (d, *J* = 6.8 Hz, 1 H), 7.61 (d, *J =* 15.7 Hz, 1 H), 6.73 (d, *J =* 15.2 Hz, 1 H), 4.78 (s, 2H), 3.00 (q, *J* = 7.6 Hz, 2H), 1.58 (s, 9H), 1.44 (t, *J* = 7.5 Hz, 3H).

### Example 63

### 3-{3-[(Butyl-ethyl-amino)-methyl]-2-ethyl-imidazo[1,2-a]pyridin-7-yl}-N-hydroxy-acrylamide (compound 63)

The titled compound was prepared according to the procedures described in the general synthetic procedure by using appropriate starting materials. HPLC: 99 %; t_{R} = 0.405min; LCMS (ESI) m/z 345 [MH]⁺; ¹H NMR (CD₃OD): δ 8.64 (d, *J* = 7.2 Hz, 1 H), 7.88 (s, 1 H), 7.67 (d, *J* = 15.7 Hz, 1H), 7.57 (d, *J* = 7.0 Hz, 1H), 6.76 (d, *J* = 16.2 Hz, 1H), 4.87 (masked peaks, 2H), 3.31 (masked peaks, 4H), 2.99 (q, *J* = 7.5 Hz, 2H), 1.70 - 1.82 (m, 2H), 1.34 - 1.47 (m, 8H), 1.00 (t, *J* = 7.3 Hz, 3H).

### Example 64

### 3-(2-Ethyl-3-{[ethyl-(3-methyl-butyl)-amino]-methyl}-imidazo[1,2-a]pyridin-7-yl)-N-hydroxy-acrylamide (compound 64)

The titled compound was prepared according to the procedures described in the general synthetic procedure by using appropriate starting materials. HPLC: 99 %; t_{R} = 0.728min; LCMS (ESI) m/z 359 [MH]⁺; ¹H NMR (CD₃OD): δ 8.70 (d, *J* = 6.0 Hz, 1 H), 7.92 (s, 1 H), 7.61 - 7.67 (m, 2H), 6.78 (d, *J* = 14.0 Hz, 1H), 4.87 (masked peaks, 2H), 3.31 (masked peaks, 4H), 3.01 (q, *J* = 7.4 Hz, 2H), 1.60 - 1.70 (m, 3H), 1.38 - 1.46 (m, 6H), 0.98 (d, *J* = 5.8 Hz, 6H).

### Example 65

### 3-[3-(tert-Butylamino-methyl)-2-(3-methyl-butyl)-imidazo[1,2-a]pyridin-7-yl]-N-hydroxy-acrylamide (compound 65)

The titled compound was prepared according to the procedures described in the general synthetic procedure by using appropriate starting materials. HPLC: 99 %; t_{R} = 1.075min; LCMS (ESI) m/z 359 [MH]^{+ 1}H NMR (CD₃OD): δ 8.65 (brs, 1H), 7.92 (s, 1H), 7.60 - 7.64 (m, 2H), 6.75 (d, *J* = 14.0 Hz, 1 H), 4.87 (masked peaks, 2H), 2.97 (brs, 2H), 1.73 (brs, 3H), 1.58 (s, 9H), 1.03 (d, *J* = 5.8 Hz, 6H).

### Example 66

### 3-[3-[(Butyl-ethyl-amino)-methyl]-2-(3-methyl-butyl)-imidazo[1,2-a]pyridin-7-yl]-N-hydroxy-acrylamide (compound 66)

The titled compound was prepared according to the procedures described in the general synthetic procedure by using appropriate starting materials. HPLC: 99 %; t_{R} = 1.315 min; LCMS (ESI) m/z 387 [MH]⁺; ¹H NMR (CD₃OD): δ 8.69 (d, *J* = 5.4 Hz, 1 H), 7.91 (s, 1 H), 7.61 - 7.65 (m, 2H), 6.77 (d, *J* = 15.8 Hz, 1H), 4.87 (masked peaks, 2H), 3.31 (masked peaks, 4H), 2.97 (t, *J* = 7.6 Hz, 2H), 1.70 - 1.83 (m, 5H), 1.36 - 1.48 (m, 5H), 0.96 - 1.09 (m, 9H).

### Example 67

### 3-[3-{[Ethyl-(3-methyl-butyl)-amino]-methy}-2-(3-methyl-butyl)-imidazo[1,2-a]pyridin-7-yl]-N-hydroxy-acrylamide (compound 67)

The titled compound was prepared according to the procedures described in the general synthetic procedure by using appropriate starting materials. HPLC: 99 %; t_{R} = 1.551 min; LCMS (ESI) m/z 401 [MH]⁺; ¹H NMR (CD₃OD): δ 8.68 (d, *J* = 5.9 Hz, 1 H), 7.90 (s, 1 H), 7.59 - 7.63 (m, 2H), 6.78 (d, *J* = 15.7 Hz, 1 H), 4.87 (masked peaks, 2H), 3.31 (masked peaks, 4H), 2.97 (t, *J* = 7.5 Hz, 2H), 1.69 - 1.78 (m, 6H), 1.40 (t, *J* = 7.0 Hz, 3H), 0.93 - 1.04 (m, 12H).

### Example 68

### 3-[3-{[(3,3-Dimethyl-butyl)-ethyl-amino]-methyl}-2-(3-methyl-butyl)-imidazo[1,2-a]pyridin-7-yl]-N-hydroxy-acrylamide (compound 68)

The titled compound was prepared according to the procedures described in the general synthetic procedure by using appropriate starting materials. HPLC: 99 %; t_{R} = 1.719 min; LCMS (ESI) m/z 415 [MH]⁺; ¹H NMR (CD₃OD): δ 8.70 (d, *J* = 7.1 Hz, 1 H), 7.91 (s, 1 H), 7.60 - 7.65 (m, 2H), 6.77 (d, *J* = 15.8 Hz, 1 H), 4.87 (masked peaks, 2H), 3.31 (masked peaks, 4H), 2.98 (t, *J* = 7.8 Hz, 2H), 1.70 - 1.78 (m, 5H), 1.40 (t, *J* = 7.2 Hz, 3H), 1.00 - 1.05 (m, 15H).

Following the procedures outlined above, Table 1 shows the compounds that have been synthesized.

**Table 1**

| **Compound No.** | **Structure** | **m/z [MH]⁺** |
|---|---|---|
| **1** | 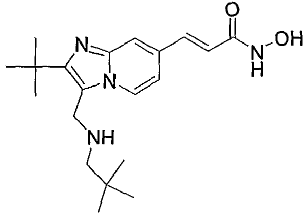 | 358 |
| **2** | 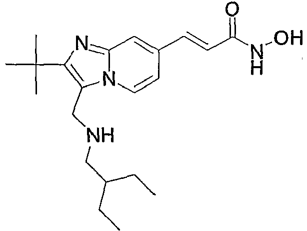 | 358 |
| **3** | 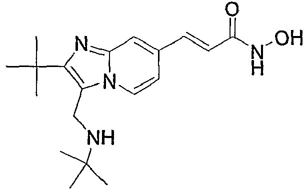 | 344 |
| **4** | 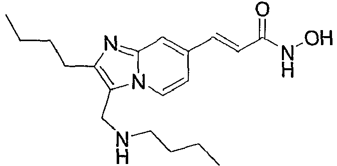 | 344 |
| **5** | 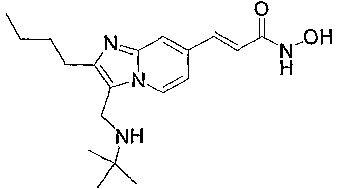 | 344 |
| **6** | 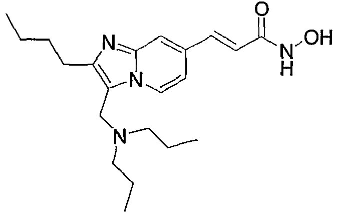 | 372 |
| **7** | 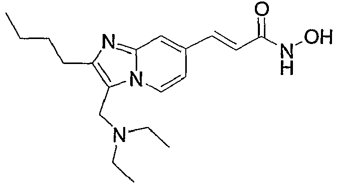 | 344 |
| **8** | 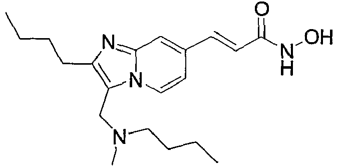 | 358 |
| **9** | 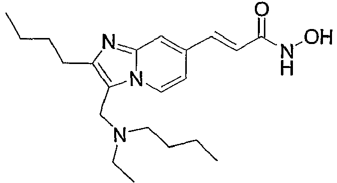 | 372 |
| **10** | 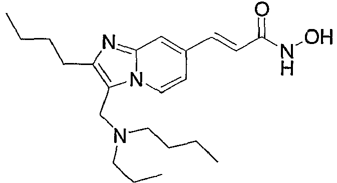 | 386 |
| **11** | 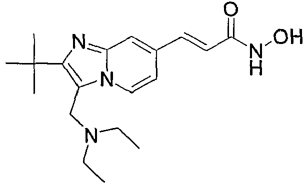 | 344 |
| **12** | 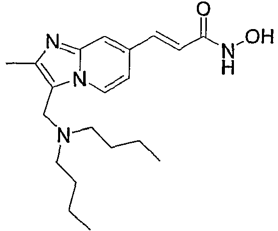 | 358 |
| **13** | 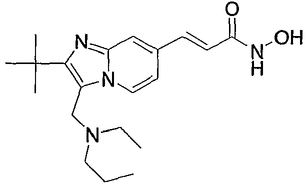 | 358 |
| **14** | 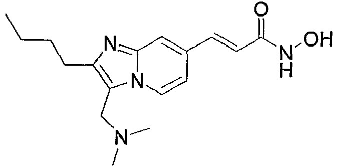 | 316 |
| **15** | 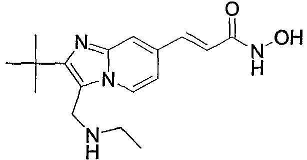 | 316 |
| **16** | 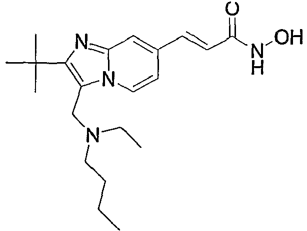 | 373 |
| **17** | 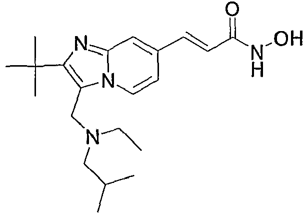 | 373 |
| **18** | 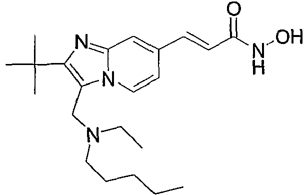 | 387 |
| **19** | 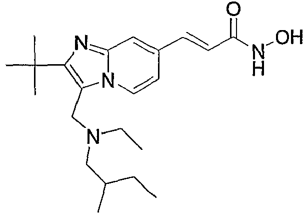 | 387 |
| **20** | 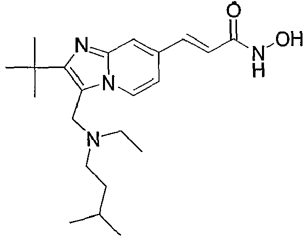 | 387 |
| **21** | 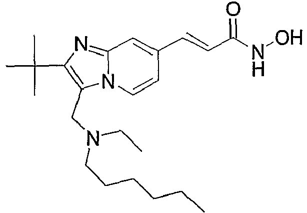 | 401 |
| **22** | 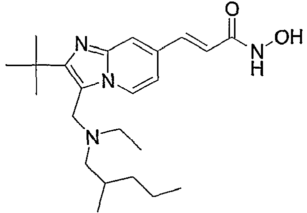 | 401 |
| **23** | 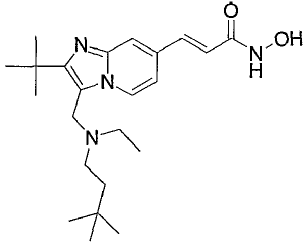 | 401 |
| **24** | 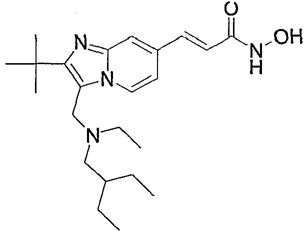 | 401 |
| **25** | 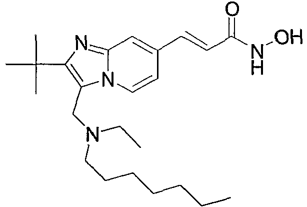 | 415 |
| **26** | 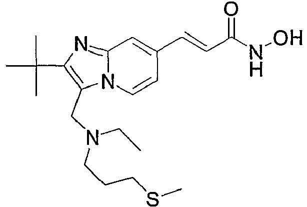 | 405 |
| **27** | 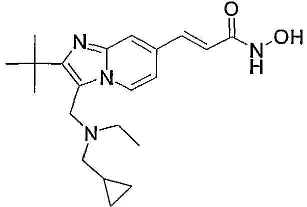 | 370 |
| **28** | 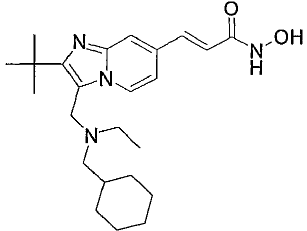 | 413 |
| **29** | 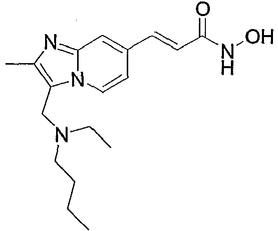 | 330 |
| **30** | 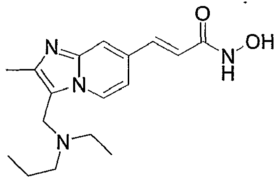 | 316 |
| **31** | 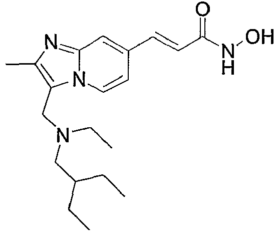 | 358 |
| **32** | 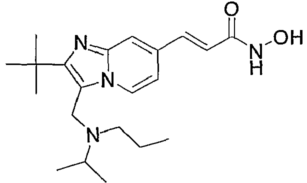 | 373 |
| **33** | 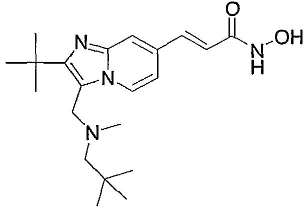 | 373 |
| **34** | 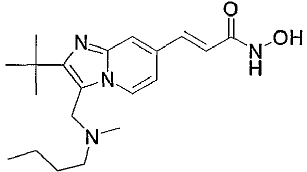 | 358 |
| **35** | 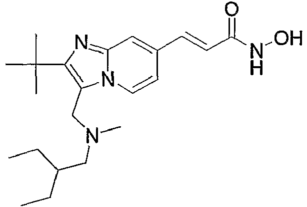 | 387 |
| **36** | 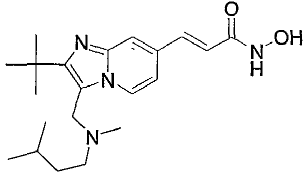 | 373 |
| **37** | 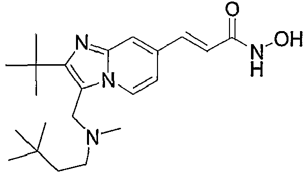 | 387 |
| **38** | 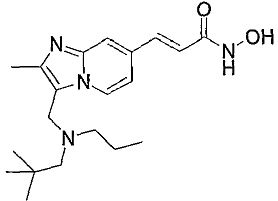 | 358 |
| **39** | 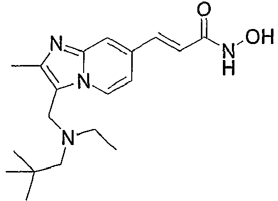 | 344 |
| **40** | 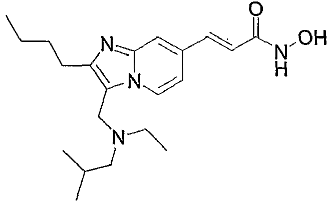 | 373 |
| **41** | 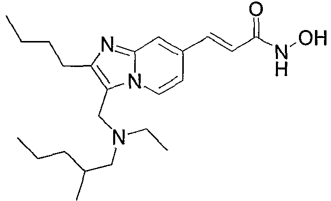 | 387 |
| **42** | 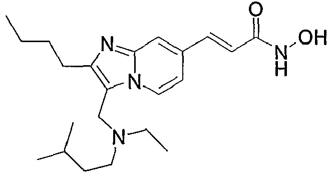 | 387 |
| **43** | 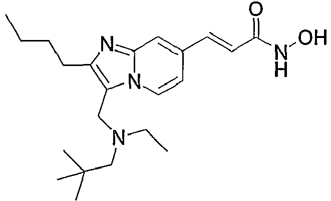 | 387 |
| **44** | 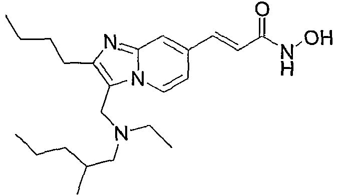 | 401 |
| **45** | 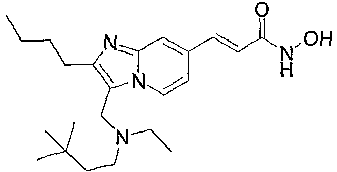 | 401 |
| **46** | 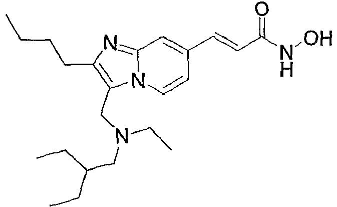 | 401 |
| **47** | 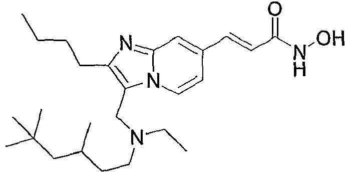 | 443 |
| **48** | 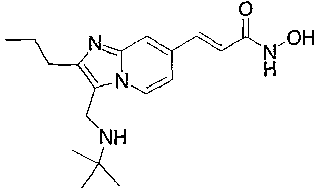 | 330 |
| **49** | 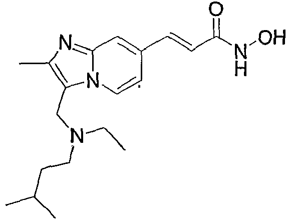 | 344 |
| **50** | 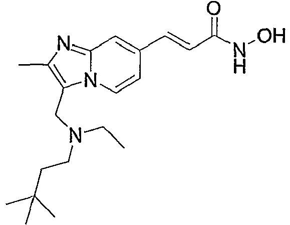 | 358 |
| **51** | 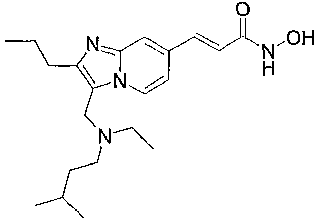 | 373 |
| **52** | 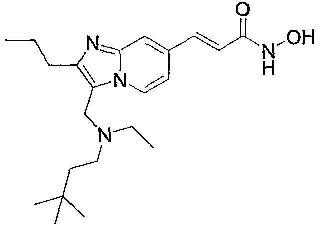 | 387 |
| **53** | 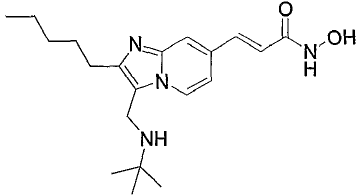 | 358 |
| **54** | 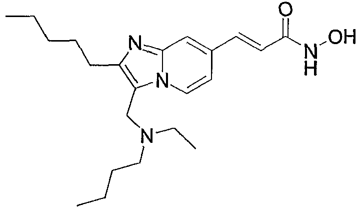 | 387 |
| **55** | 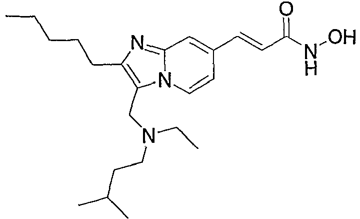 | 401 |
| **56** | 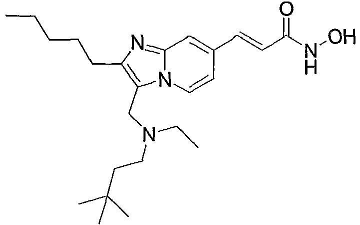 | 415 |
| **57** | 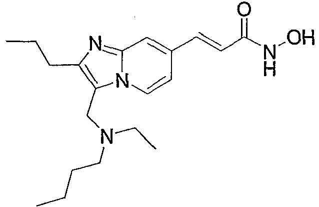 | 358 |
| **58** | 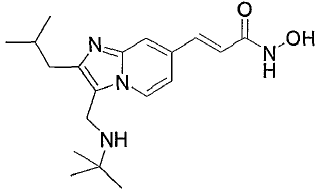 | 344 |
| **59** | 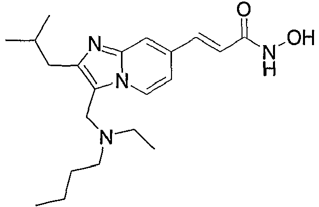 | 373 |
| **60** | 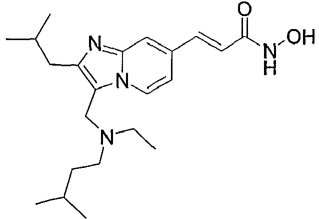 | 387 |
| **61** | 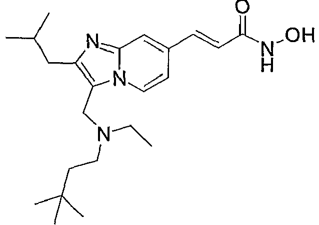 | 401 |
| **62** | 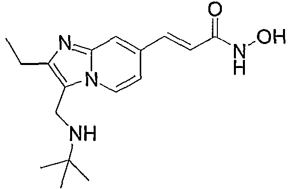 | 316 |
| **63** | 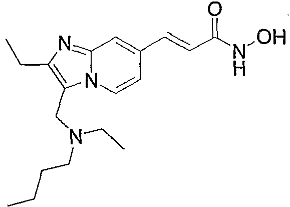 | 344 |
| **64** | 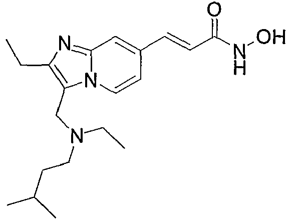 | 358 |
| **65** | 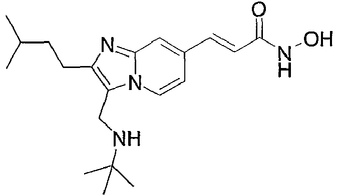 | 358 |
| **66** | 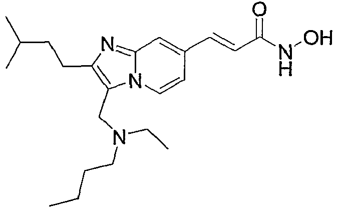 | 387 |
| **67** | 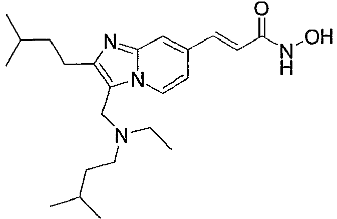 | 401 |
| **68** | 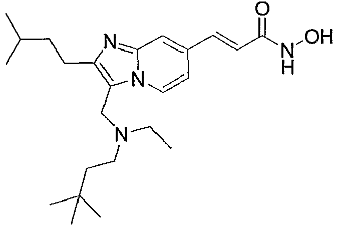 | 415 |

### BIOLOGICAL TESTING AND ENZYME ASSAYS

### Recombinant GST-HDAC1 Protein expression and purification

Human cDNA library was prepared using cultured SW620 cells. Amplification of human HDAC1 coding region from this cDNA library was cloned separately into the baculovirus expression pDEST20 vector (GATEWAY Cloning Technology, Invitrogen Pte Ltd). The pDEST20-HDAC1 construct was confirmed by DNA sequencing. Recombinant baculovirus was prepared using the Bac-To-Bac method following the manufacturer's instruction (Invitrogen Pte Ltd). Baculovirus titer was determined by plaque assay to be about 10⁸ PFU/ml.

Expression of GST-HDAC1 was done by infecting SF9 cells (Invitrogen Pte Ltd) with pDEST20-HDAC1 baculovirus at MOI=1 for 48 h. Soluble cell lysate was incubated with pre-equilibrated Glutathione Sepharose 4B beads (Amersham) at 4°C for 2 h. The beads were washed with PBS buffer for 3 times. The GST-HDAC1 protein was eluted by elution buffer containing 50 mM Tris, pH8.0, 150mM NaCl, 1% Triton X-100 and 10mM or 20mM reduced Glutathione. The purified GST-HDAC1 protein was dialyzed with HDAC storage buffer containing 10mM Tris, pH7.5, 100mM NaCl and 3mM MgCl₂. 20% Glycerol was added to purified GST-HDAC1 protein before storage at -80°C.

### In vitro HDAC assay for determination of IC₅₀ values

The assay has been carried out in 96 well format and the BIOMOL fluorescent-based HDAC activity assay has been applied. The reaction composed of assay buffer, containing 25 mM Tris pH 7.5, 137 mM NaCl, 2.7 mM KCl, 1 mM MgCl₂, 1 mg/ml BSA, tested compounds, an appropriate concentration of HDAC1 enzyme, 500 uM *Flur de lys* generic substrate for HDAC1 enzyme and subsequently was incubated at room temperature for 2 h. *Flur de lys* Developer was added and the reaction was incubated for 10 min. Briefly, deacetylation of the substrate sensitizes it to the developer, which then generates a fluorophore. The fluorophore is excited with 360 nm light and the emitted light (460 nm) is detected on a fluorometric plate reader (Tecan Ultra Microplate detection system, Tecan Group Ltd.).

The analytical software, Prism 4.0 (GraphPad Software Inc) has been used to generate IC₅₀ from a series of data. IC₅₀ is defined as the concentration of compound required for 50% inhibition of HDAC enzyme activity.

The HDAC enzyme inhibition results of representative compounds are shown in Table 2 (unit in the table is micromolar).

**Table 2**

| **Compound No.** | **IC₅₀ (µM) (HDAC 1)** | **Compound No.** | **IC₅₀ (µM) (HDAC 1)** | **Compound No.** | **IC₅₀ (µM) (HDAC 1)** |
|---|---|---|---|---|---|
| **1** | 0.19 | **24** | 0.19 | **47** | 0.20 |
| **2** | 0.16 | **25** | 0.54 | **48** | 0.04 |
| **3** | 0.13 | **26** | 0.09 | **49** | 0.02 |
| **4** | 0.02 | **27** | 0.33 | **50** | 0.05 |
| **5** | 0.02 | **28** | 0.19 | **51** | 0.03 |
| **6** | 0.20 | **29** | 0.03 | **52** | 0.02 |
| **7** | 0.18 | **30** | 0.54 | **53** | 0.02 |
| **8** | 0.07 | **31** | 0.01 | **54** | 0.04 |
| **9** | 0.06 | **32** | 0.30 | **55** | 0.03 |
| **10** | 0.09 | **33** | 0.78 | **56** | 0.03 |
| **11** | 1.2 | **34** | 0.22 | **57** | 0.04 |
| **12** | 0.08 | **35** | 0.41 | **58** | 0.05 |
| **13** | 0.27 | **36** | 0.35 | **59** | 0.04 |
| **14** | 0.04 | **37** | 0.27 | **60** | 0.02 |
| **15** | 0.06 | **38** | 0.13 | **61** | 0.03 |
| **16** | 0.06 | **39** | 0.05 | **62** | 0.03 |
| **17** | 0.57 | **40** | 0.09 | **63** | 0.05 |
| **18** | 0.13 | **41** | 0.06 | **64** | 0.02 |
| **19** | 0.26 | **42** | 0.02 | **65** | 0.02 |
| **20** | 0.06 | **43** | 0.16 | **66** | 0.04 |
| **21** | 0.24 | **44** | 0.08 | **67** | 0.03 |
| **22** | 0.47 | **45** | 0.03 | **68** | 0.04 |
| **23** | 0.07 | **46** | 0.15 | | |

### Cell-based proliferation assay for determination of GI₅₀ values

Human cancer cell lines (e.g. Colo205) were obtained from ATCC. Colo205 cells were cultivated in RPMI 1640 containing 2 mM L-Glutamine, 5% FBS, 1.0 mM Na Pyruvate. Colo205 cells were seeded in 96-wells plate at 5000 cells per well. The plates were incubated at 37°C, 5% CO₂, for 24 h. Cells were treated with compounds at various concentrations for 96 h. Cell growth was then monitored using CyQUANT® cell proliferation assay (Invitrogen Pte Ltd). Dose response curves were plotted to determine Gl₅₀ values for the compounds using XL-fit (ID Business Solution, Emeryville, CA). Gl₅₀ is defined as the concentration of compound required for 50% inhibition of cell growth.

The cellular or growth inhibition activity results of representative compounds are shown in Table 3 below (unit in the table is micromolar). The data indicated that the compounds of this invention are active in the inhibition of tumor cell growth.

**Table 3**

| **Compound No.** | **Gl₅₀ (µM) (Colo205)** | **Compound No.** | **Gl₅₀ (µM) (Colo205)** | **Compound No.** | **Gl₅₀ (µM) (Colo205)** |
|---|---|---|---|---|---|
| **1** | 1.5 | **24** | 0.97 | **47** | 0.53 |
| **2** | 1.5 | **25** | 2.70 | **48** | 0.18 |
| **3** | 0.74 | **26** | 0.60 | **49** | 0.11 |
| **4** | 0.18 | **27** | 2.10 | **50** | 0.16 |
| **5** | 0.14 | **28** | 1.10 | **51** | 0.08 |
| **6** | 0.61 | **29** | 0.18 | **52** | 0.07 |
| **7** | 0.68 | **30** | 4.60 | **53** | 0.07 |
| **8** | 0.17 | **31** | 0.05 | **54** | 0.15 |
| **9** | 0.16 | **32** | 2.50 | **55** | 0.14 |
| **10** | 0.58 | **33** | 6.30 | **56** | 0.15 |
| **11** | 4.4 | **34** | 1.70 | **57** | 0.16 |
| **12** | 0.80 | **35** | 2.30 | **58** | 0.21 |
| **13** | 1.8 | **36** | 0.90 | **59** | 0.15 |
| **14** | 0.23 | **37** | 0.73 | **60** | 0.15 |
| **15** | 0.47 | **38** | 0.70 | **61** | 0.17 |
| **16** | 0.68 | **39** | 0.60 | **62** | 0.26 |
| **17** | 3.90 | **40** | 0.57 | **63** | 0.18 |
| **18** | 0.75 | **41** | 0.37 | **64** | 0.10 |
| **19** | 2.20 | **42** | 0.09 | **65** | 0.10 |
| **20** | 0.35 | **43** | 0.95 | **66** | 0.16 |
| **21** | 2.00 | **44** | 0.49 | **67** | 0.13 |
| **22** | 2.10 | **45** | 0.14 | **68** | 0.13 |
| **23** | 0.48 | **46** | 0.39 | | |

### Measurement of Microsomal stability

Metabolic stability measurements *in vitro* using liver microsomes aids in the prediction of *in vivo* hepatic clearance and compound stability towards phase I biotransformation reactions mediated by P450 isozymes.

Pooled human liver microsome (HLM) preparation was purchased from BD Gentest (BD BioSciences). The incubations consisted of test compound (5 µM) or control compound (Verapamil), NADPH-generating system solution A (25 mM NADP⁺, 66 mM glucose-6-phosphate, 66 mM MgCl₂ in H₂O), NADPH-generating system solution B (40 U/ml glucose-6-phosphate dehydrogenase in 5 mM sodium citrate) and 1.0 mg/ml microsomal protein, respectively, in 100 mM potassium phosphate buffer (pH 7.4). Samples are incubated for 0, 5, 15, 30, 45, 60min. Reaction is terminated with ice-cold 80% acetonitrile and 20% DMSO. Samples are subsequently centrifuged at 4°C for 15 min at 2,000 rpm. 100 µL of the supernatant is transferred to the LC-MS plate for analysis. Before quantitative analysis, the compound is tuned in the LC/MS machine to get the optimized MS condition. Liquid chromatography is performed on a Luna C18 column (Phenomenex U.S.A, Torrance, CA) (2x50mm, 5 µM). Percentage (%) remaining of compound (by area) of each time point is calculated with respect to time 0. Plot %remaining against time (min) to obtain the curve and use the Prism software to obtain the T_{1/2}. These are demonstrated in Table 4.

**Table 4.**

| **Compound No.** | **T_{1/2} (min)** |
|---|---|
| **5** | **>60** |
| **29** | **59** |
| **48** | **>60** |
| **49** | **>60** |
| **50** | **45** |
| **63** | **>60** |
| **64** | **>60** |
| **65** | **>60** |

The measured *in vitro* T_{1/2} >60 mins for the above compounds signifies that the contribution towards the in vivo clearance of the compound due to metabolism is expected to be low. This may result in a longer half-life and increased in vivo exposure of the compounds.

The above results show the compounds of this invention are metabolically stable in human liver microsomes. Together with the appropriate physicochemical properties, e.g., molecular weight, logP and high solubility, the above compounds are likely to reach therapeutic concentrations in target organs when administrated intravenously or orally.

### High Throughput Solubility Profiling (HTSP)

Determining compound aqueous solubility has become an essential early measurement in the drug discovery process. Poor aqueous solubility can cause problems in many in vitro testing techniques, leading to unreliable results. The standard way to determine the solubility of a compound is to use the shake-flask solubility method. However, this method is inherently low-throughput and labour intensive. A high-throughput kinetic solubility profiling method in which experiments were carried out in 96-well format using compounds in 2.5% DMSO/aqueous media was developed to determine the solubilities of the compounds from this invention.

10mM compound stock in DMSO was dissolved in phosphate buffer (pH 7) to give a final concentration of 250µM in 96-well plates in triplicate. The plate was shaken for 1.5 hours at 450 rpm and left to stand for 2 hours to allow the compound to reach its saturation point. The plate was then centrifuged at 2000g for 15 minutes. The supernatant was transferred and mixed with 20µL of DMSO in another 96-well plate. A calibration curve at 100µM and 250µM was prepared from the 10mM stock by dissolving in 60% methanol/phosphate buffer solution. LC/MS was used in the analysis and the area counts were obtained by using the Quantify Method Editor. Using the three point standard curve including zero, the results for solubility were reported. For compounds with calculated concentration of 250 ± 30µM, their solubility was reported as >250µM. These are demonstrated in Table 5.

**Table 5.**

| **Compound No.** | **Solubility (µM)** |
|---|---|
| **5** | **200** |
| **48** | **168** |
| **49** | **>250** |

### In vivo antineoplastic (or anti-tumor) effect of HDAC inhibiting agents:

The efficacy of the compounds of the invention can then be determined using *in vivo* animal xenograft studies. The animal xenograft model is one of the most commonly used *in vivo* cancer models.

In these studies Female athymic nude mice (Harlan), 12-14 weeks of age would be implanted subcutaneously in the flank with 5 x 10⁶ cells of HCT116 human colon tumor cells, or with 5 x 10⁶ cells of A2780 human ovarian tumor cells, or with 5 x 10⁶ cells of PC3 prostate cancer cells. When the tumor reaches the size 100 mm³, the xenograft nude mice would be paired-match into various treatment groups. The selected HDAC inhibitors would be dissolved in appropriate vehicles and administered to xenograft nude mice intraperitoneally, intravenously or orally daily for 14-21 days. The dosing volume will be 0.01 ml/g body weight. Paclitaxol, used as positive control, will be prepared for intravenous administration in an appropriate vehicle. The dosing volume for Paclitaxol will be 0.01 ml/g body weight. Tumor volume will be calculated every second day or twice-a-week of post injection using the formula: Volume (mm³) = (w² x l)/2, where w = width and l = length in mm of an HCT116, or A2780, or PC3 tumor. Compounds of this invention that are tested would show significant reduction in tumor volume relative to controls treated with vehicle only. Acetylated histone relative to vehicle treated control group when measured shall be accumulated. The result will therefore indicate that compounds of this invention are efficacious in treating a proliferative disease such as cancer.

## Claims

1. A compound of the formula (I): 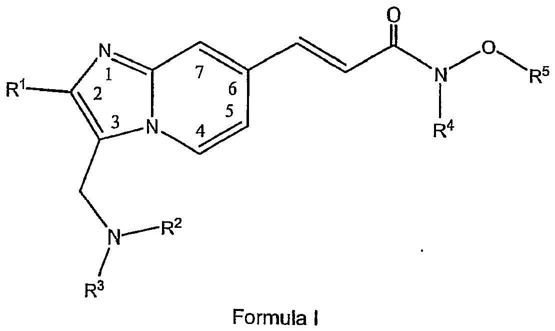 wherein:
R¹ is alkyl;
R² is alkyl;
R³ is alkyl;
wherein if the total number of carbon atoms in R² is X and the total number of carbon atoms in R³ is Y, then R² and R³ are selected such that the sum of X + Y is an integer selected from the group consisting of 5, 6, 7 and 8;
R⁴ is H;
R⁵ is H;
or a pharmaceutically acceptable salt thereof.

2. A compound of claim 1 wherein R¹ is C₁-C₅ alkyl.

3. A compound according to any one of claims 1 to 2 wherein R¹ is selected from the group consisting of methyl, ethyl, propyl, isopropyl, 2-methyl-propyl, 2,2-dimethyl-propyl, butyl, tert-butyl, 3-methyl-butyl, and pentyl.

4. A compound according to any one of claims 1 to 3 wherein R² is C₁-C₆ alkyl.

5. A compound according to any one of claims 1 to 4 wherein R² is selected from the group consisting of methyl, ethyl, propyl and butyl.

6. A compound according to any one of claims 1 to 5 wherein R³ is C₁-C₆ alkyl.

7. A compound according to any one of claims 1 to 6 wherein R³ is selected from the group consisting of methyl, ethyl, propyl, isopropyl, 2-methyl-propyl, 2,2-dimethyl- propyl, butyl, isobutyl, tert-butyl, 2-ethyl-butyl, 3-methyl-butyl, 3,3-dimethyl-butyl, pentyl, 2- methyl-pentyl, 3-methyl-pentyl, 4-methyl-pentyl, and hexyl.

8. A compound according to any one of claims 1 to 7 wherein the sum of X + Y is 6, 7 or 8.

9. A compound according to any one of claims 1 to 8 wherein the sum of X + Y is 7 or 8.

10. A compound according to any one of claims 1 to 9 wherein the compound is selected from the group consisting of:
| | |
|---|---|
| 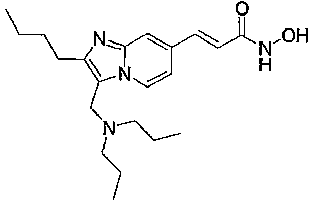 | 3-(2-Butyl-3-dipropylaminomethyl-imidazo[1,2-a]pyridin-7-yl)-N-hydroxy-acrylamide |
| 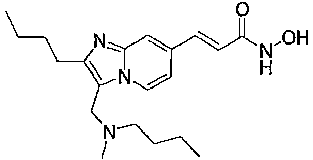 | 3-{2-Butyl-3-[(butyl-methyl-amino)-methyl]-imidazo[1,2-a]pyridin-7-yl}-N-hydroxy-acrylamide |
| 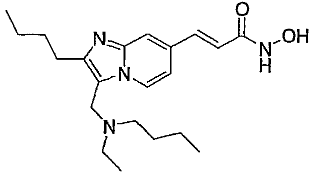 | 3-{2-Butyl-3-[(butyl-ethyl-amino)-methyl]-imidazo[1,2-a]pyridin-7-yl}-N-hydroxy-acrylamide |
| 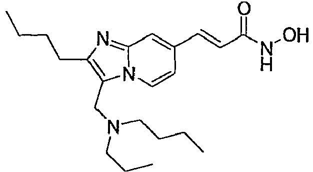 | 3-{2-Butyl-3-[(butyl-propyl-amino)-methyl]-imidazo[1,2-a]pyridin-7-yl}-N-hydroxy-acrylamide |
| 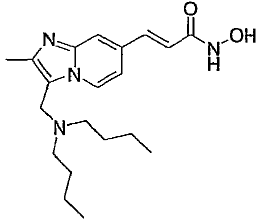 | 3-(3-Dibutylaminomethyl-2-methyl-imidazo[1,2-a]pyridin-7-yl)-N-hydroxy-acrylamide |
| 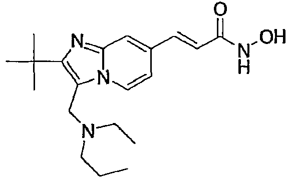 | 3-{2-tert-Butyl-3-[(ethyl-propyl-amino)-methyl]-imidazo[1,2-a]pyridin-7-yl}-N-hydroxy-acrylamide |
| 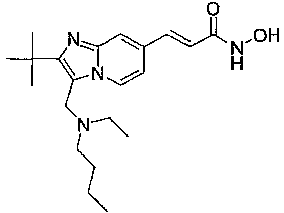 | 3-{2-tert-Butyl-3-[(butyl-ethyl-amino)-methyl]-imidazo[1,2-a]pyridin-7-yl}-N-hydroxy-acrylamide |
| 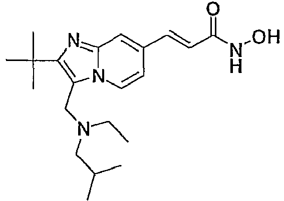 | 3-{2-tert-Butyl-3-[(ethyl-isobutyl-amino)-methyl]-imidazo[1,2-a]pyridin-7-yl}-N-hydroxy-acrylamide |
| 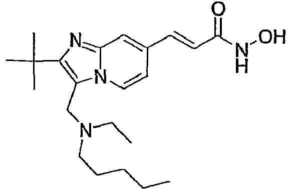 | 3-{2-tert-Butyl-3-[(ethyl-pentyl-amino)-methyl]-imidazo[1,2-a]pyridin-7-yl}-N-hydroxy-acrylamide |
| 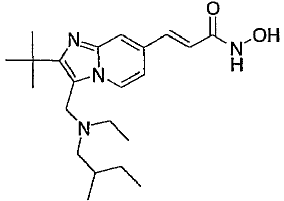 | 3-(2-tert-Butyl-3-{[ethyl-(2-methyl-butyl)-amino]-methyl}-imidazo[1,2-a]pyridin-7-yl)-N-hydroxy-acrylamide |
| 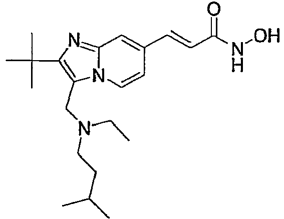 | 3-(2-tert-Butyl-3-{[ethyl-(3-methyl-butyl)-amino]-methyl}-imidazo[1,2-a]pyridin-7-yl)-N-hydroxy-acrylamide |
| 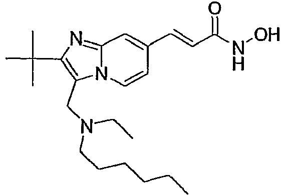 | 3-{2-tert-Butyl-3-[(ethyl-hexyl-amino)-methyl]-imidazo[1,2-a]pyridin-7-yl}-N-hydroxy-acrylamide |
| 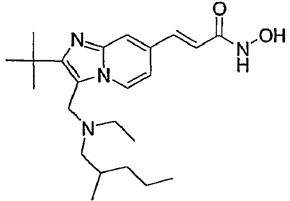 | 3-(2-tert-Butyl-3-{[ethyl-(2-methyl-pentyl)-amino]-methyl}-imidazo[1,2-a]pyridin-7-yl)-N-hydroxy-acrylamide |
| 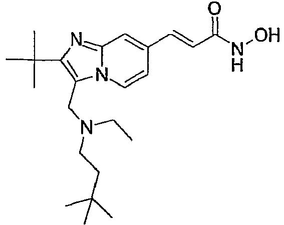 | 3-(2-tert-Butyl-3-{[(3,3-dimethyl-butyl)-ethyl-amino]-methyl}-imidazo[1,2-a]pyridin-7-yl)-N-hydroxy-acrylamide |
| 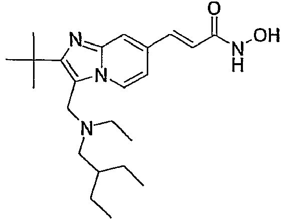 | 3-(2-tert-Butyl-3-{[ethyl-(2-ethyl-butyl)-amino]-methyl}-imidazo[1,2-a]pyridin-7-yl)-N-hydroxy-acrylamide |
| 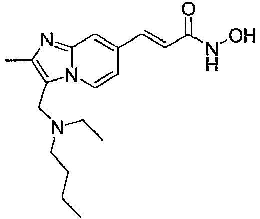 | 3-{3-[(Butyl-ethyl-amino)-methyl]-2-methyl-imidazo[1,2-a]pyridin-7-yl}-N-hydroxy-acrylamide |
| 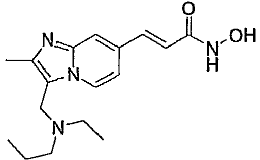 | 3-{3-[(Ethyl-propyl-amino)-methyl]-2-methyl-imidazo[1,2-a]pyridin-7-yl}-N-hydroxy-acrylamide |
| 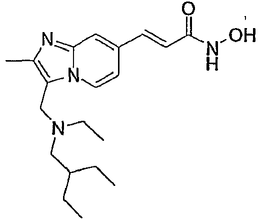 | 3-(3-{[Ethyl-(2-ethyl-butyl)-amino]-methyl}-2-methyl-imidazo[1,2-a]pyridin-7-yl)-N-hydroxy-acrylamide |
| 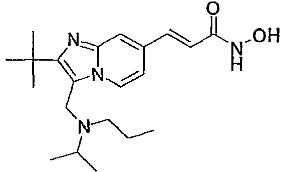 | 3-{2-tert-Butyl-3-[(isopropyl-propyl-amino)-methyl]-imidazo[1,2-a]pyridin-7-yl}-N-hydroxy-acrylamide |
| 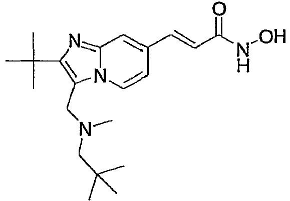 | 3-(2-tert-Butyl-3-{[(2,2-dimethyl-propyl)-methyl-amino]-methyl}-imidazo[1,2-a]pyridin-7-yl)-N-hydroxy-acrylamide |
| 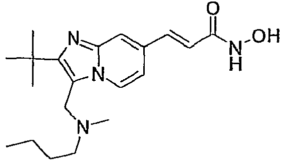 | 3-{2-tert-Butyl-3-[(butyl-methyl-amino)-methyl]-imidazo[1,2-a]pyridin-7-yl}-N-hydroxy-acrylamide |
| 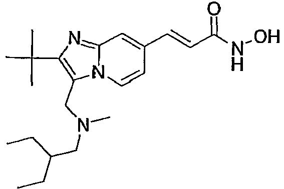 | 3-(2-tert-Butyl-3-{[(2-ethyl-butyl)-methyl-amino]-methyl}-imidazo[1,2-a]pyridin-7-yl)-N-hydroxy-acrylamide |
| 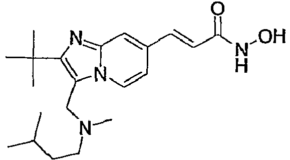 | 3-(2-tert-Butyl-3-{[methyl-(3-methyl-butyl)-amino]-methyl}-imidazo[1,2-a]pyridin-7-yl)-N-hydroxy-acrylamide |
| 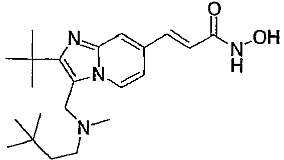 | 3-(2-tert-Butyl-3-{[(3,3-dimethyl-butyl)-methyl-amino]-methyl}-imidazo[1,2-a]pyridin-7-yl)-N-hydroxy-acrylamide |
| 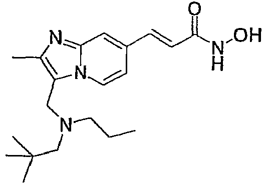 | 3-(3-{[(2,2-Dimethyl-propyl)-propyl-amino]-methyl}-2-methyl-imidazo[1,2-a]pyridin-7-yl)-N- hydroxy-acrylamide |
| 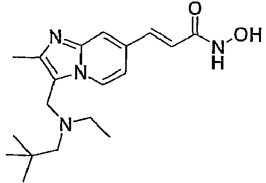 | 3-(3-{[(2,2-Dimethyl-propyl)-ethyl-amino]-methyl}-2-methyl-imidazo[1,2-a]pyridin-7-yl)-N-hydroxy-acrylamide |
| 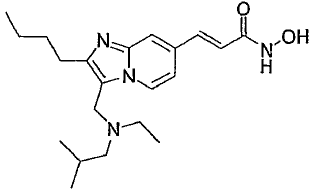 | 3-{2-Butyl-3-[(ethyl-isobutyl-amino)-methyl]-imidazo[1,2-a]pyridin-7-yl}-N-hydroxy-acrylamide |
| 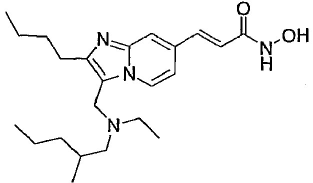 | 3-(2-Butyl-3-{[ethyl-(2-methyl-pentyl)-amino]-methyl}-imidazo[1,2-a]pyridin-7-yl)-N-hydroxy-acrylamide |
| 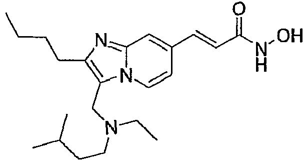 | 3-(2-Butyl-3-{[ethyl-(3-methyl-butyl)-amino]-methyl}-imidazo[1,2-a]pyridin-7-yl)-N-hydroxy-acrylamide |
| 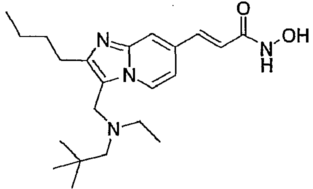 | 3-(2-Butyl-3-{[(2,2-dimethyl-propyl)-ethyl-amino]-methyl}-imidazo[1,2-a]pyridin-7-yl)-N-hydroxy-acrylamide |
| 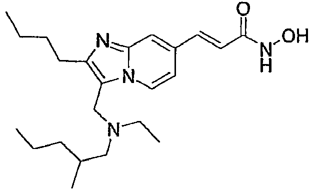 | 3-(2-Butyl-3-{[ethyl-(2-methyl-pentyl)-amino]-methyl}-imidazo[1,2-a]pyridin-7-yl)-N-hydroxy-acrylamide |
| 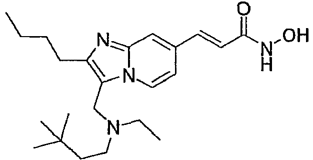 | 3-(2-Butyl-3-{[(3,3-dimethyl-butyl)-ethyl-amino]-methyl}-imidazo[1,2-a]pyridin-7-yl)-N-hydroxy-acrylamide |
| 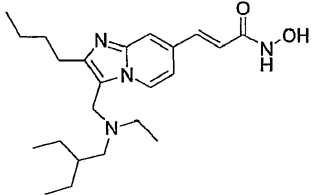 | 3-(2-Butyl-3-{[ethyl-(2-ethyl-butyl)-amino]-methyl}-imidazo[1,2-a]pyridin-7-yl)-N-hydroxy-acrylamide |
| 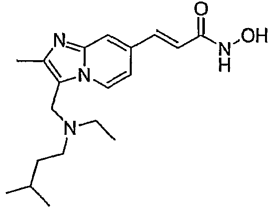 | 3-(3-{[Ethyl-(3-methyl-butyl)-amino]-methyl}-2- methyl-imidazo[1,2-a]pyridin-7-yl)-N-hydroxy-acrylamide |
| 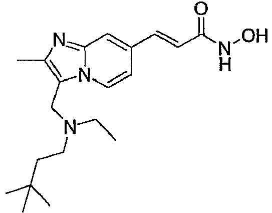 | 3-(3-{[Ethyl-(3,3-dimethyl-butyl)-amino]-methyl}-2-methyl-imidazo[1,2-a]pyridin-7-yl)-N-hydroxy-acrylamide |
| 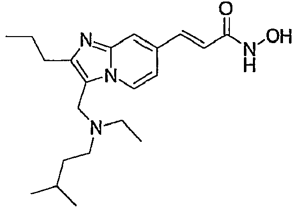 | 3-(3-{[Ethyl-(3-methyl-butyl)-amino]-methyl}-2-propyl-imidazo[1,2-a]pyridin-7-yl)-N-hydroxy-acrylamide |
| 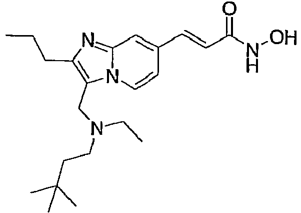 | 3-(3-{[(3,3-Dimethyl-butyl)-ethyl-amino]-methyl}-2-propyl-imidazo[1,2-a]pyridin-7-yl)-N-hydroxy-acrylamide |
| 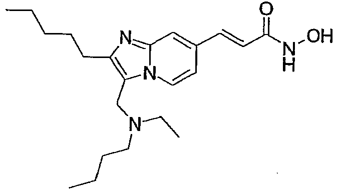 | 3-{3-[(Butyl-ethyl-amino)-methyl]-2-pentyl-imidazo[1,2-a]pyridin-7-yl}-N-hydroxy-acrylamide |
| 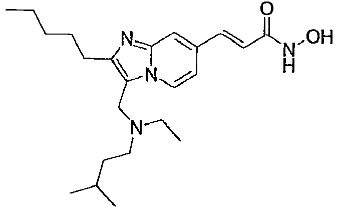 | 3-(3-{[Ethyl-(3-methyl-butyl)-amino]-methyl}-2-pentyl-imidazo[1,2-a]pyridin-7-yl)-N-hydroxy-acrylamide |
| 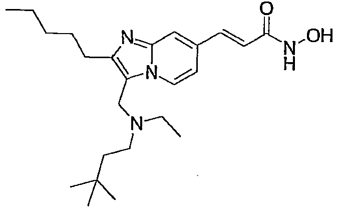 | 3-(3-{[(3,3-Dimethyl-butyl)-ethyl-amino]-methyl}-2-pentyl-imidazo[1,2-a]pyridin-7-yl)-N-hydroxy-acrylamide |
| 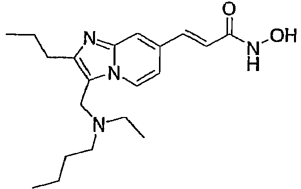 | 3-{3-[(Butyl-ethyl-amino)-methyl]-2-propyl-imidazo[1,2-a]pyridin-7-yl}-N-hydroxy-acrylamide |
| 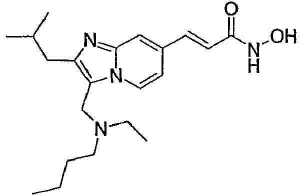 | 3-{3-[(Butyl-ethyl-amino)-methyl]-2-isobutyl-imidazo[1,2-a]pyridin-7-yl}-N-hydroxy-acrylamide |
| 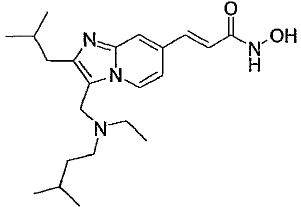 | 3-(3-{[Ethyl-(3-methyl-butyl)-amino]-methyl}-2-isobutyl-imidazo[1,2-a]pyridin-7-yl)-N-hydroxy-acrylamide |
| 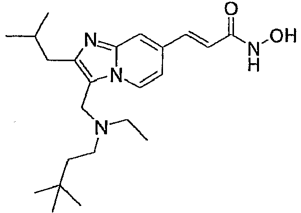 | 3-(3-{[(3,3-Dimethyl-butyl)-ethyl-amino]-methyl}-2-isobutyl-imidazo[1,2-a]pyridin-7-yl)-N-hydroxy-acrylamide |
| 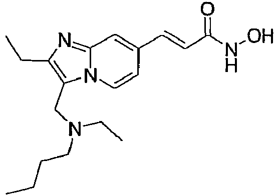 | 3-{3-[(Butyl-ethyl-amino)-methyl]-2-ethyl-imidazo[1,2-a]pyridin-7-yl}-N-hydroxy-acrylamide |
| 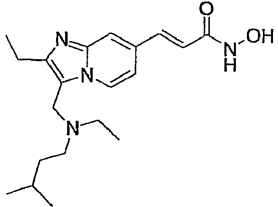 | 3-(2-Ethyl-3-{[ethyl-(3-methyl-butyl)-amino]-methyl}-imidazo[1,2-a]pyridin-7-yl)-N-hydroxy-acrylamide |
| 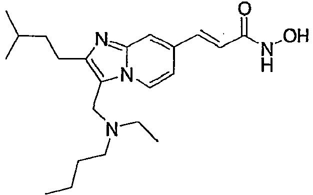 | 3-[3-[(Butyl-ethyl-amino)-methyl]-2-(3-methyl-butyl)-imidazo[1,2-a]pyridin-7-yl]-N-hydroxy-acrylamide |
| 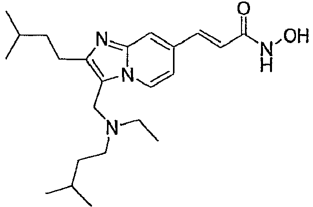 | 3-[3-{[Ethyl-(3-methyl-butyl)-amino]-methyl}-2-(3-methyl-butyl)-imidazo[1,2-a]pyridin-7-yl]-N-hydroxy-acrylamide |
| 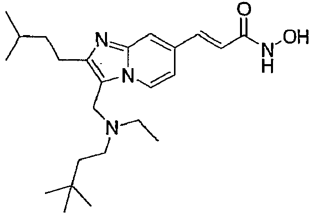 | 3-[3-{[(3,3-Dimethyl-butyl)-ethyl-amino]-methyl}-2-(3-methyl-butyl)-imidazo[1,2-a]pyridin-7-yl]-N-hydroxy-acrylamide . |

11. A pharmaceutical composition including a compound according to any one of claims 1 to 10 and a pharmaceutically acceptable diluent, excipient or carrier.

12. A compound according to any one of claims 1 to 10 for use in the treatment of a disorder caused by, associated with or accompanied by disruptions of cell proliferation and/or angiogenesis.

13. A compound for use according to claim 12 wherein the disorder is cancer.

14. A compound according to any one of claims 1 to 10 for use in the treatment of a disorder that can be treated by the inhibition of histone deacetylase.

15. A compound for use according to claim 14 wherein the disorder is selected from the group consisting of Proliferative disorders (e.g. cancer); Neurodegenerative diseases including Huntington's Disease, Polyglutamine diseases, Parkinson's Disease, Alzheimer's Disease, Seizures, Striatonigral degeneration, Progressive supranuclear palsy, Torsion dystonia, Spasmodic torticollis and dyskinesis, Familial tremor, Gilles de la Tourette syndrome, Diffuse Lewy body disease, Pick's disease, Intracerebral haemorrhage, Primary lateral sclerosis, Spinal muscular atrophy, Amyotrophic lateral sclerosis, Hypertrophic interstitial polyneuropathy, Retinitis pigmentosa, Hereditary optic atrophy, Hereditary spastic paraplegia, Progressive ataxia and Shy-Drager syndrome; Metabolic diseases including Type 2 diabetes; Degenerative Diseases of the Eye including Glaucoma, Age-related macular degeneration, macular myopic degeneration, Rubeotic glaucoma, Interstitial keratitis, Diabetic retinopathy, Peter's anomaly, retinal degeneration, Cellophane Retinopathy; Cogan's Dystrophy; Corneal Dystrophy; Iris Neovascularization (Rubeosis); Neovascularization of the Cornea; Retinopathy of Prematurity; Macular Edema; Macular Hole; Macular Pucker; Marginal Blepharitis, Myopia, nonmalignant growth of the conjunctiva; Inflammatory diseases and/or Immune system disorders including Rheumatoid Arthritis (RA), Osteoarthritis, Juvenile chronic arthritis, Graft versus Host disease, Psoriasis, Asthma, Spondyloarthropathy, Crohn's Disease, inflammatory bowel disease, Colitis Ulcerosa, Alcoholic hepatitis, Diabetes, Sjoegrens's syndrome, Multiple Sclerosis, Ankylosing spondylitis, Membranous glomerulopathy, Discogenic pain, Systemic Lupus Erythematosus, allergic contact dermatitis; Disease involving angiogenesis including cancer, psoriasis, rheumatoid arthritis; Psychological disorders including bipolar disease, schizophrenia, depression and dementia; Cardiovascular Diseases including Heart failure, restenosis, cardiac hypertrophy and arteriosclerosis; Fibrotic diseases including liver fibrosis, lung fibrosis, cystic fibrosis and angiofibroma; Infectious diseases including Fungal infections, such as Candida Albicans, Bacterial infections, Viral infections, such as Herpes Simplex, Protozoal infections, such as Malaria, Leishmania infection, Trypanosoma brucei infection, Toxoplasmosis and coccidiosis and Haematopoietic disorders including thalassemia, anemia and sickle cell anemia.

16. A compound for use according to claim 13 wherein the cancer is selected from the group consisting of a hematologic malignancy and a solid tumor.

17. A compound for use according to claim 13 wherein the cancer is selected from a group consisting of B-cell lymphoma, T-cell lymphoma, leukemia, breast cancer, lung cancer, ovarian cancer, prostate cancer, head and neck cancer, renal cancer, gastric cancer, colon cancer, pancreatic cancer and brain cancer.

## Patentansprüche

1. Verbindung mit der Formel (I): 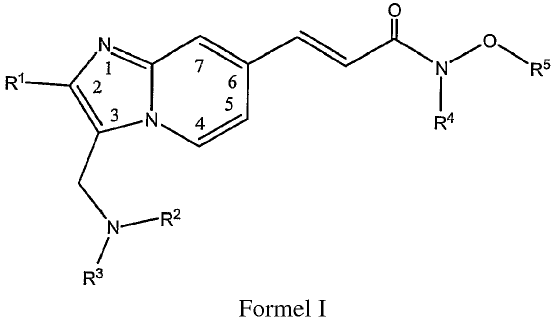 wobei:
R¹ ein Alkyl darstellt;
R² ein Alkyl darstellt;
R³ ein Alkyl darstellt;
wobei, wenn die Gesamtzahl an Kohlenstoffatomen in R² gleich X ist und die Gesamtzahl an Kohlenstoffatomen in R³ gleich Y ist, dann werden R² und R³ so ausgewählt, dass die Summe aus X + Y eine ganze Zahl ist, die aus der Gruppe ausgewählt ist, die aus 5, 6, 7 und 8 besteht;
R⁴ ein H darstellt;
R⁵ ein H darstellt;
oder ein pharmazeutisch verträgliches Salz derselben.

2. Verbindung nach Anspruch 1, wobei R¹ ein C₁-C₅-Alkyl darstellt.

3. Verbindung nach einem der Ansprüche 1 bis 2, wobei R¹ aus der Gruppe ausgewählt ist, die aus Methyl, Ethyl, Propyl, Isopropyl, 2-Methyl-propyl, 2,2-Dimethyl-propyl, Butyl, tert-Butyl, 3-Methyl-butyl und Pentyl, die jeweils als Option substituiert sein können, besteht.

4. Verbindung nach einem der Ansprüche 1 bis 3, wobei R² ein C₁-C₆-Alkyl darstellt.

5. Verbindung nach einem der Ansprüche 1 bis 4, wobei R² aus der Gruppe ausgewählt ist, die aus Methyl, Ethyl, Propyl und Butyl besteht.

6. Verbindung nach einem der Ansprüche 1 bis 5, wobei R³ ein C₁-C₆-Alkyl darstellt.

7. Verbindung nach einem der Ansprüche 1 bis 6, wobei R³ aus der Gruppe ausgewählt ist, die aus Methyl, Ethyl, Propyl, Isopropyl, 2-Methyl-propyl, 2,2-Dimethyl-propyl, Butyl, Isobutyl, tert-Butyl, 2-Ethyl-butyl, 3-Methyl-butyl, 3,3-Dimethyl-butyl, Pentyl, 2-Methyl-pentyl, 3-Methyl-pentyl, 4-Methyl-pentyl und Hexyl besteht.

8. Verbindung nach einem der Ansprüche 1 bis 7, wobei die Summe aus X + Y gleich 6, 7 oder 8 ist.

9. Verbindung nach einem der Ansprüche 1 bis 8, wobei die Summe aus X + Y gleich 7 oder 8 ist.

10. Verbindung nach einem der Ansprüche 1 bis 9, wobei die Verbindung aus der Gruppe ausgewählt ist, die aus:
| | |
|---|---|
| 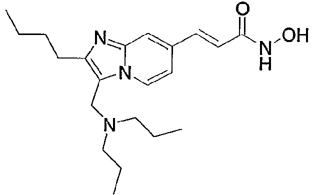 | 3-(2-Butyl-3-dipropylaminomethyl-imidazo[1,2-a]pyridin-7-yl)-N-hydroxy-acrylamid |
| 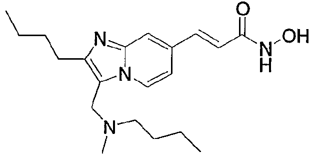 | 3-{2-Butyl-3-[(butyl-methyl-amino)-methyl]-imidazo[1,2-a]pyridin-7-yl}-N-hydroxy-acrylamid |
| 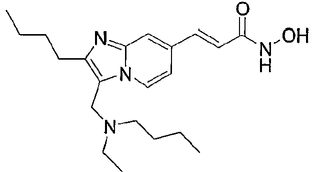 | 3-{2-Butyl-3-[(butyl-ethyl-amino)-methyl]-imidazo[1,2-a]pyridin-7-yl}-N-hydroxy-acrylamid |
| 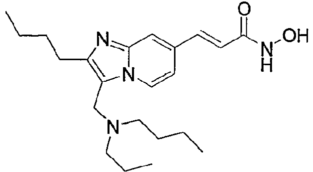 | 3-{2-Butyl-3-[(butyI-propyI-amino)-methyl]-imidazo[1,2-a]pyridin-7-yl}-N-hydroxy-acrylamid |
| 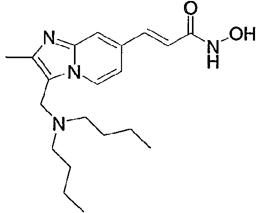 | 3-(3-Dibutylaminomethyl-2-methyl-imidazo[1,2-a]pyridin-7-yI)-N-hydroxy-acrylamid |
| 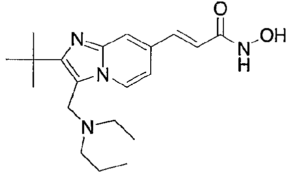 | 3-{2-tert-Butyl-3-[(ethyl-propyl-amino)-methyl]-imidazo[1,2-a]pyridin-7-yl}-N-hydroxy-acrylamid |
| 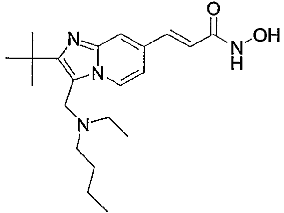 | 3-{2-tert-Butyl-3-[(butyl-ethyl-amino)-methyl]-imidazo[1,2-a]pyridin-7-yl}-N-hydroxy-acrylamid |
| 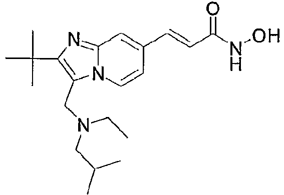 | 3-{2-tert-Butyl-3-[(ethyl-isobutyl-amino)-methyl]-imidazo[1,2-a]pyridin-7-yl}-N-hydroxy-acrylamid |
| 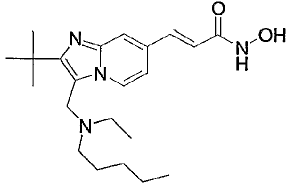 | 3-{2-tert-Butyl-3-[(ethyl-pentyl-amino)-methyl]-imidazo[1,2-a]pyridin-7-yl}-N-hydroxy-acrylamid |
| 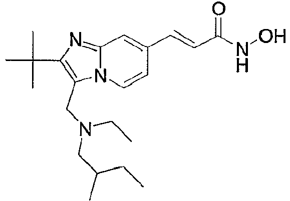 | 3-(2-tert-Butyl-3-{[ethyl-(2-methyl-butyl)-amino]-methyl}-imidazo[1,2-a]pyridin-7-yl)-N-hydroxy-acrylamid |
| 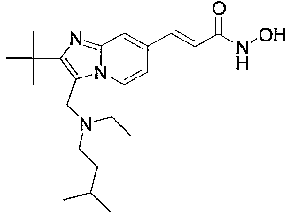 | 3-(2-tert-Butyl-3-{[ethyl-(3-methyl-butyl)-amino]-methyl}-imidazo[1,2-a]pyridin-7-yl)-N-hydroxy-acrylamid |
| 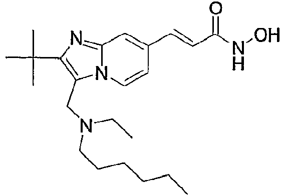 | 3-{2-tert-Butyl-3-[(ethyl-hexyl-amino)-methyl]-imidazo[1,2-a]pyridin-7-yl}-N-hydroxy-acrylamid |
| 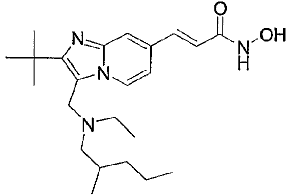 | 3-(2-tert-Butyl-3-{[ethyl-(2-methyl-pentyl)-amino]-methyl}-imidazo[1,2-a]pyridin-7-yl)-N-hydroxy-acrylamid |
| 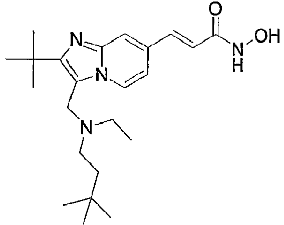 | 3-(2-tert-Butyl-3-{[(3,3-dimethyl-butyl)-ethyl-amino]-methyl}-imidazo[1,2-a]pyridin-7-yl)-N-hydroxy-acrylamid |
| 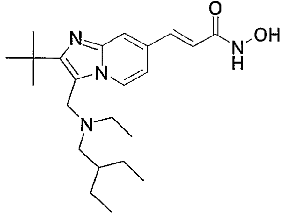 | 3-(2-tert-Butyl-3-{[ethyl-(2-ethyl-butyl)-amino]-methyl}-imidazo[1,2-a]pyridin-7-yl)-N-hydroxy-acrylamid |
| 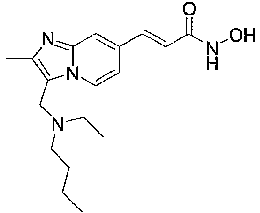 | 3-{3-[(Butyl-ethyl-amino)-methyl]-2-methyl-imidazo[1,2-a]pyridin-7-yl}-N-hydroxy-acrylamid |
| 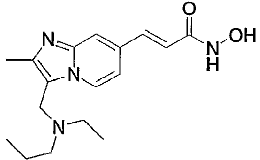 | 3-{3-[(Ethyl-propyl-amino)-methyl]-2-methyl-imidazo[1,2-a]pyridin-7-yl}-N-hydroxy-acrylamid |
| 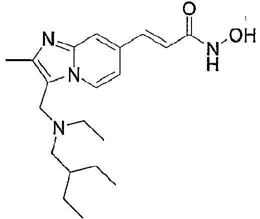 | 3-(3-{[Ethyl-(2-ethyl-butyl)-amino]-methyl}-2-methyl-imidazo[1,2-a]pyridin-7-yl)-N-hydroxy-acrylamid |
| 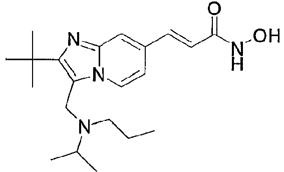 | 3-{2-tert-ButyI-3-[(isopropyl-propyl-amino)-methyl]-imidazo[1,2-a]pyridin-7-yl}-N-hydroxy-acrylamid |
| 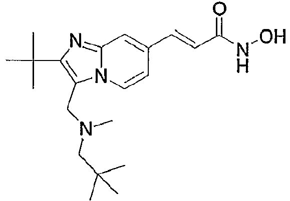 | 3-(2-tert-Butyl-3-{[(2,2-dimethyl-propyl)-methyl-amino]-methyl}-imidazo[1,2-a]pyridin-7-yl)-N-hydroxy-acrylamid |
| 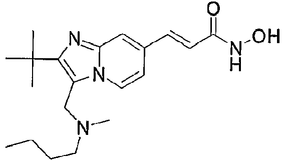 | 3-{2-tert-Butyl-3-[(butyl-methyl-amino)-methyl]-imidazo[1,2-a]pyridin-7-yl}-N-hydroxy-acrylamid |
| 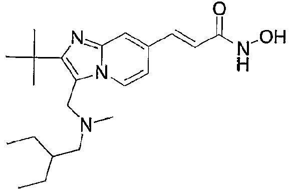 | 3-(2-tert-Butyl-3-{[(2-ethyl-butyl)-methyl-amino]-methyl}-imidazo[[1,2-a]pyridin-7-yl)-N-hydroxy-acrylamid |
| 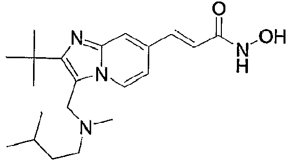 | 3-(2-tert-Butyl-3-{[methyl-(3-methyl-butyl)-amino]-methyl}-imidazo[1,2-a]pyridin-7-yl)-N-hydroxy-acrylamid |
| 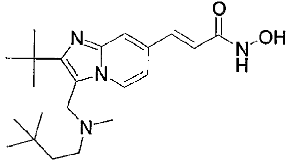 | 3-(2-tert-Butyl-3-{[(3,3-dimethyl-butyl)-methyl-amino] -methyl}-imidazo[[1,2-a]pyridin-7-yl)-N-hydroxy-acrylamid |
| 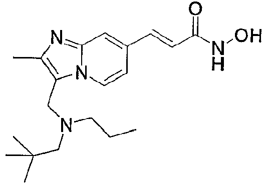 | 3-(3-{[(2,2-Dimethyl-propyl)-propyl-amino]-methyl}-2-methyl-imidazo[1,2-a]pyridin-7-yl)-N-hydroxy-acrylamid |
| 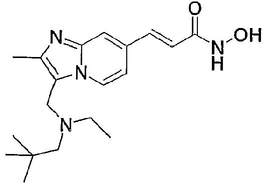 | 3-(3-{[(2,2-Dimethyl-propyl)-ethyl-amino]-methyl}-2-methyl-imidazo[1,2-a]pyridin-7-yl)-N-hydroxy-acrylamid |
| 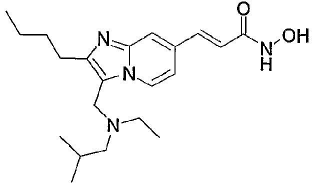 | 3-{2-Butyl-3-[(ethyl-isobutyl-amino)-methyl]-imidazo[1,2-a]pyridin-7-yl}-N-hydroxy-acrylamid |
| 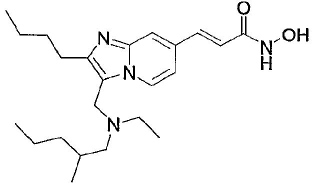 | 3-(2-Butyl-3-{[ethyl-(2-methyl-pentyl)-amino]-methyl}-imidazo[1,2-a]pyridin-7-yl)-N-hydroxy-acrylamid |
| 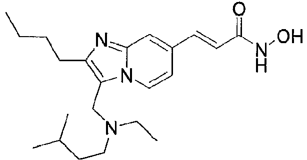 | 3-(2-Butyl-3-{[ethyl-(3-methyl-butyl)-amino]-methyl}-imidazo[1,2-a]pyridin-7-yl)-N-hydroxy-acrylamid |
| 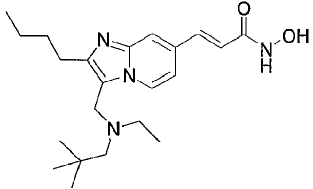 | 3-(2-Butyl-3-{[(2,2-dimethyl-propyl)-ethyl-amino]-methyl}-imidazo[1,2-a]pyridin-7-yl)-N-hydroxy-acrylamid |
| 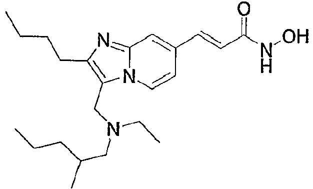 | 3-(2-Butyl-3-{[ethyl-(2-methyl-pentyl)-amino]-methyl-imidazo[1,2-a]pyridin-7-yl)-N-hydroxy-acrylamid |
| 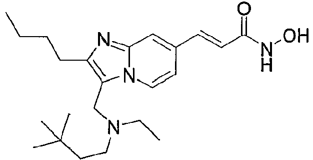 | 3-(2-Butyl-3-{[(3,3-dimethyl-butyl)-ethyl-amino]-methyl}-imidazo[1,2-a]pyridin-7-yl)-N-hydroxy-acrylamid |
| 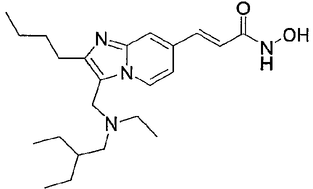 | 3-(2-Butyl-3-{[ethyl-(2-ethyl-butyl)-amino]-methyl}-imidazo[1,2-a]pyridin-7-yl)-N-hydroxy-acrylamid |
| 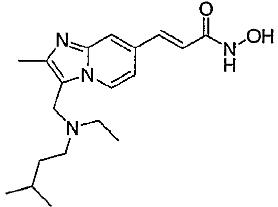 | 3-(3-{[Ethyl-(3-methyl-butyl)-amino]-methyl}-2-methyl-imidazo[1,2-a]pyridin-7-yl)-N-hydroxy-acrylamid |
| 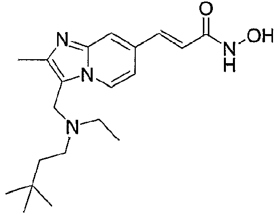 | 3-(3-{[Ethyl-(3,3-dimethyl-butyl)-amino]-methyl}-2-methyl-imidazo[1,2-a]pyridin-7-yl)-N-hydroxy-acrylamid |
| 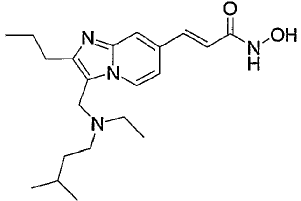 | 3-(3-{[Ethyl-(3-methyl-butyl)-amino]-methyl}-2-propyl-imidazo[1,2-a]pyridin-7-yl)-N-hydroxy-acrylamid |
| 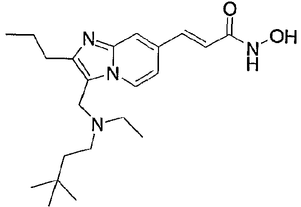 | 3-(3-{[(3,3-Dimethyl-butyl)-ethyl-amino]-methyl}-2-propyl-imidazo[1,2-a]pyridin-7-yl)-N-hydroxy-acrylamid |
| 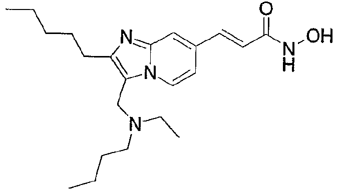 | 3-{3-[(Butyl-ethyl-amino)-methyl]-2-pentyl-imidazo[1,2-a]pyridin-7-yl}-N-hydroxy-acrylamid |
| 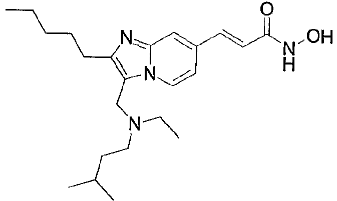 | 3-(3-{[Ethyl-(3-methyl-butyl)-amino]-methyl}-2-pentyl-imidazo[1,2-a]pyridin-7-yl)-N-hydroxy-acrylamid |
| 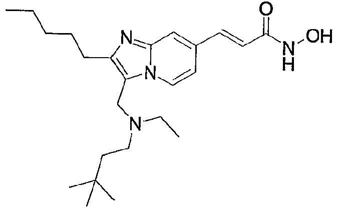 | 3-(3-{[(3,3-Dimethyl-butyl)-ethyl-amino]-methyl}-2-pentyl-imidazo[1,2-a]pyridin-7-yl)-N-hydroxy-acrylamid |
| 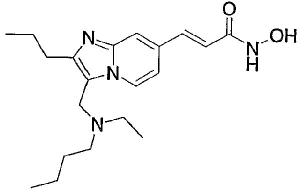 | 3-{3-[(Butyl-ethyl-amino)-methyl]-2-propyl-imidazo[1,2-a]pyridin-7-yl}-N-hydroxy-acrylamid |
| 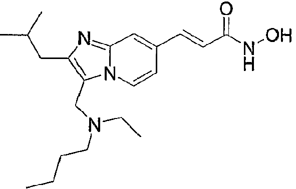 | 3-{3-[(Butyl-ethyl-amino)-methyl]-2-isobutyl-imidazo[1,2-a]pyridin-7-yl-N-hydroxy-acrylamid |
| 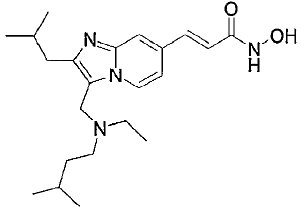 | 3-(3-{[Ethyl-(3-methyl-butyl)-amino]-methyl}-2-isobutyl-imidazo[1,2-a]pyridin-7-yl)-N-hydroxy-acrylamid |
| 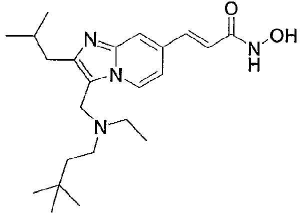 | 3-(3-{[(3,3-Dimethyl-butyl)-ethyl-amino]-methyl}-2-isobutyl-imidazo[1,2-a] pyridin-7-yl)-N-hydroxy-acrylamid |
| 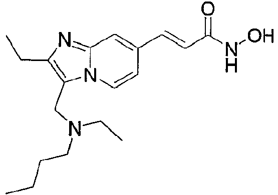 | 3-{3-[(Butyl-ethyl-amino)-methyl]-2-ethyl-imidazo[1,2-a]pyridin-7-yl}-N-hydroxy-acrylamid |
| 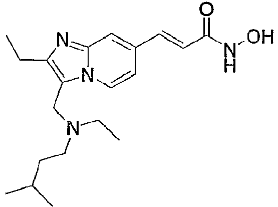 | 3-(2-Ethyl-3-{[ethyl-(3-methyl-butyl)-amino]-methyl}-imidazo[1,2-a]pyridin-7-yl)-N-hydroxy-acrylamid |
| 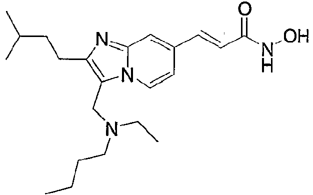 | 3-[3-[(Butyl-ethyl-amino)-methyl]-2-(3-methyl-butyl)-imidazo[1,2-a]pyridin-7-yl]-N-hydroxy-acrylamid |
| 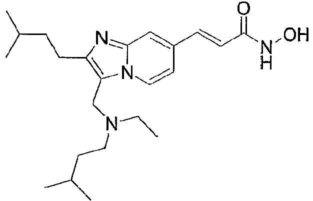 | 3-[3-{[Ethyl-(3-methyl-butyl)-amino]-methyl}-2-(3-methyl-butyl)-imidazo[1,2-a]pyridin-7-yl]-N-hydroxy-acrylamid |
| 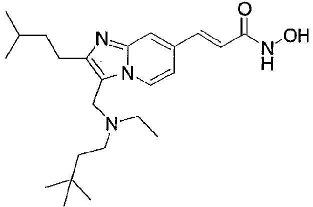 | 3-[3-{[(3,3-Dimethyl-butyl)-ethyl-amino]-methyl}-2-(3-methyl-butyl)-imidazo[1,2-a]pyridin-7-yl]-N-hydroxy-acrylamid |
besteht.

11. Pharmazeutische Zusammensetzung, die eine Verbindung nach einem der Ansprüche 1 bis 10 und ein pharmazeutisch geeignetes Verdünnungsmittel oder einen pharmazeutisch geeigneten Hilfsstoff oder Träger beinhaltet.

12. Verbindung nach einem der Ansprüche 1 bis 10 zur Anwendung bei der Behandlung einer Störung, die durch Störungen der Zellproliferation und/oder der Angiogenese verursacht wird oder damit verbunden ist oder davon begleitet wird.

13. Verbindung zur Anwendung nach Anspruch 12, wobei es sich bei der Störung um eine Krebserkrankung handelt.

14. Verbindung nach einem der Ansprüche 1 bis 10 zur Anwendung bei der Behandlung einer Störung, die durch Hemmung der Histon-Deacetylase behandelt werden kann.

15. Verbindung nach Anspruch 14, wobei die Störung ausgewählt ist aus der Gruppe bestehend aus proliferativen Störungen (z.B. Krebs); neurodegenerativen Erkrankungen einschließlich Huntington Krankheit, Polyglutamin-Erkrankungen, Parkinson'sche Krankheit, Alzheimer'sche Krankheit, Krämpfe, striatonigraler Degeneration, progressiver supranukleärer Blickparese, Torsionsdystonie, Torticollis spasmodicus und Dyskinesie, familiärer Tremor, Tourette-Syndrome, diffuse Lewy-Körperchen-Krankheit, Pick'sche Krankheit, intrazerebrale Blutung, primäre Lateralsklerose, spinale Muskelatrophie, amyotrophe Lateralsklerose, hypertrophe interstitielle Polyneuropathy, Retinitis pigmentosa, hereditäre Optikusatrophie, hereditäre spastische Paraplegie, progressive Ataxie und Shy-Drager-Syndrom; Stoffwechselerkrankungen einschließlich Typ 2-Diabetes; degenerative Erkrankungen des Auges einschließlich Glaukom, altersbedingte Makuladegeneration, myopische Makuladegeneration, rubeotisches Glaukom, interstitielle Keratitis, diabetische Retinopathie, Peters-Anomalie, Netzhautdegeneration, Zellophan-Retinopathie; Cogan-Dystrophie; Hornhautdystrophie; Neovaskularisierung der Iris (Rubeosis); Neovaskularisierung der Hornhaut; Frühgeborenen-Retinopathia; Makulaödem; Makulaloch; Makula Pucker; marginale Blepharitis, Myopie, nicht-bösartiges Wachstum der Augenbindehaut; Entzündungskrankheiten und/oder Störungen des Immunsystems einschließlich rheumatoide Arthritis (RA), Osteoarthritis, juvenile chronische Arthritis, Graft versus Host-Krankheit, Psoriasis, Asthma, Spondylarthropathie, Morbus Crohn, entzündliche Darmerkrankung, Colitis ulcerosa, Alkoholhepatitis, Diabetes, Sjögrens'sches Syndrom, multiple Sklerose, Spondylitis ankylosans, membranöse Glomerulopathie, diskogener Schmerz, systemischer Lupus erythematodes, allergische Kontaktdermatitis; Erkrankungen, die mit Angiogenese einhergeghen, einschließlich Krebs, Psoriasis, rheumatoide Arthritis; Psychologische Störungen einschließlich bipolar Störung, Schizophrenie, Depressionen und Demenz; Herz-Kreislauf-Erkrankungen einschließlich Herzversagen, Restenose, Hypertrophie des Herzens und Arteriosklerose; Fibrotische Erkrankungen einschließlich Leberfibrose, Lungenfibrose, Mukoviszidose und Angiofibrom; Infektionskrankheiten einschließlich Pilzinfektionen, wie Candida albicans, bakterielle Infektionen, Virusinfektionen, wie Herpes simplex, Protozoeninfektionen, wie Malaria, Leishmania-Infektion, Trypanosoma brucei-Infektion, Toxoplasmose und Coccidiose und hämatopoetische Störungen einschließlich Thalassämie, Anämie und Sichelzellanämie besteht.

16. Verbindung zur Anwendung nach Anspruch 13, wobei die Krebserkrankung aus der Gruppe ausgewählt ist, die aus einer hämatologischen bösartigen Erkrankung und einem soliden Tumor besteht.

17. Verbindung zur Anwendung nach Anspruch 13, wobei die Krebserkrankung aus einer Gruppe ausgewählt ist, die aus B-Zelllymphom, T-Zelllymphom, Leukämie, Brustkrebs, Lungenkarzinom, Eierstockkrebs, Prostatakarzinom, Kopf-Hals-Karzinom, Nierenkarzinom, Magenkrebs, Dickdarmkrebs, Bauchspeicheldrüsenkarzinom und Gehirntumor besteht.

## Revendications

1. Composé de la formule (I) : 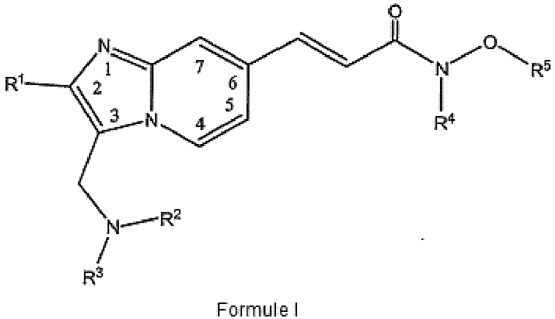 dans laquelle :
R¹ est alkyle ;
R² est alkyle ;
R³ est alkyle ;
dans laquelle si le nombre total d'atomes de carbone dans R² est X et le nombre total d'atomes de carbone dans R³ est Y, alors R² et R³ sont sélectionnés de sorte que la somme de X + Y est un entier sélectionné parmi le groupe constitué de 5, 6, 7 et 8 ;
R⁴ est H ;
R⁵ est H ;
ou un sel pharmaceutiquement acceptable de celui-ci.

2. Composé selon la revendication 1, dans lequel R¹ est alkyle en C₁-C₅.

3. Composé selon l'une quelconque des revendications 1 à 2, dans lequel R¹ est sélectionné parmi le groupe constitué de méthyle, éthyle, propyle, isopropyle, 2-méthyl-propyle, 2,2-diméthyl-propyle, butyle, tert-butyle, 3-méthyl-butyle et pentyle.

4. Composé selon l'une quelconque des revendications 1 à 3, dans lequel R² est alkyle en C₁-C₆.

5. Composé selon l'une quelconque des revendications 1 à 4, dans lequel R² est sélectionné parmi le groupe constitué de méthyle, éthyle, propyle et butyle.

6. Composé selon l'une quelconque des revendications 1 à 5, dans lequel R³ est alkyle en C₁-C₆.

7. Composé selon l'une quelconque des revendications 1 à 6, dans lequel R³ est sélectionné parmi le groupe constitué de méthyle, éthyle, propyle, isopropyle, 2-méthyl-propyle, 2,2-diméthyl-propyle, butyle, isobutyle, tert-butyle, 2-éthyl-butyle, 3-méthyl-butyle, 3,3-diméthyl-butyle, pentyle, 2-méthyl-pentyle, 3-méthyl-pentyle, 4-méthyl-pentyle et hexyle.

8. Composé selon l'une quelconque des revendications 1 à 7, dans lequel la somme de X + Y est de 6, 7 ou 8.

9. Composé selon l'une quelconque des revendications 1 à 8, dans lequel la somme de X + Y est de 7 ou 8.

10. Composé selon l'une quelconque des revendications 1 à 9, dans lequel le composé est sélectionné parmi le groupe constitué de :
| | |
|---|---|
| 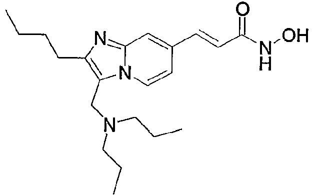 | 3-(2-butyl-3-dipropylaminométhyl-imidazo[1,2-a]pyridin-7-yl)-N-hydroxy-acrylamide |
| 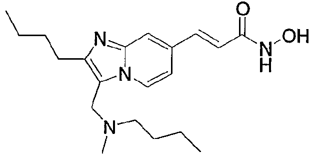 | 3-{2-butyl-3-[(butyl-méthyl-amino)-méthyl]-imidazo[1,2-a]pyridin-7-yl}-N-hydroxy-acrylamide |
| 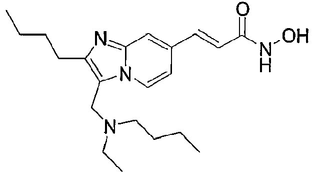 | 3-{2-butyl-3-[(butyl-éthyl-amino)-méthyl]-imidazo[1,2-a]pyridin-7-yl}-N-hydroxy-acrylamide |
| 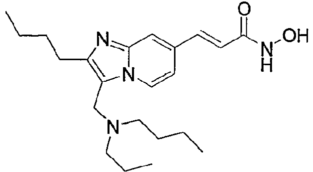 | 3-{2-butyl-3-[(butyl-propyl-amino)-méthyl]-imidazo[1,2-a]pyridin-7-yl}-N-hydroxy-acrylamide |
| 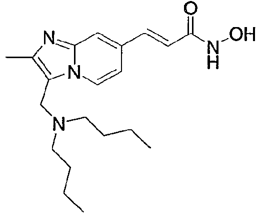 | 3-(3-dibutylaminométhyl-2-méthyl-imidazo[1,2-a]pyridin-7-yl)-N-hydroxy-acrylamide |
| 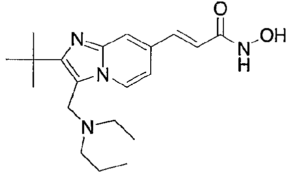 | 3-{2-tert-butyl-3-[(éthyl-propyl-amino)-méthyl]-imidazo[1,2-a]pyridin-7-yl}-N-hydroxy-acrylamide |
| 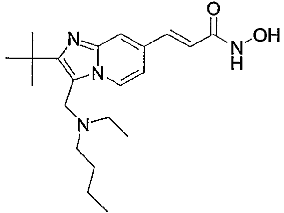 | 3-{2-tert-butyl-3-[(butyl-éthyl-amino)-méthyl]-imidazo[1,2-a]pyridin-7-yl}-N-hydroxy-acrylamide |
| 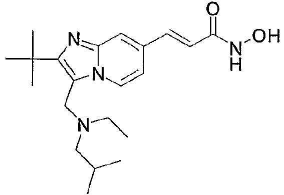 | 3-{2-tert-butyl-3-[(éthyl-isobutyl-amino)-méthyl]-imidazo[1,2-a]pyridin-7-yl}-N-hydroxy-acrylamide |
| 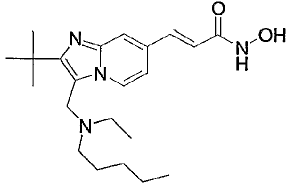 | 3-2-tert-butyl-3-[(éthyl-pentyl-amino)-méthyl]-imidazo[1,2-a]pyridin-7-yl}-N-hydroxy-acrylamide |
| 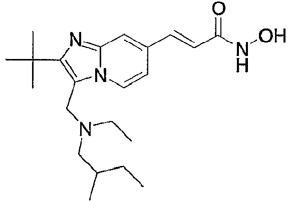 | 3-(2-tert-butyl-3-{[éthyl-(2-méthyl-butyl)-amino]-méthyl}-imidazo[1,2-a]pyridin-7-yl)-N-hydroxy-acrylamide |
| 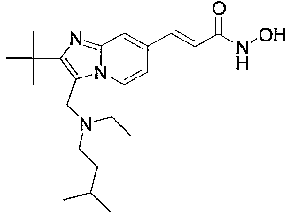 | 3-(2-tert-butyl-3-{[éthyl-(3-méthyl-butyl)-amino]-méthyl}-imidazo[1,2-a]pyridin-7-yl)-N-hydroxy-acrylamide |
| 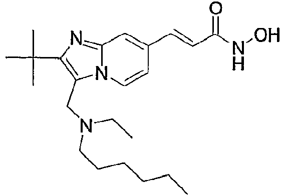 | 3-{2-tert-butyl-3-[(éthyl-hexyl-amino)-méthyl]-imidazo[1,2-a]pyridin-7-yl}-N-hydroxy-acrylamide |
| 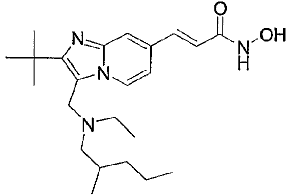 | 3-(2-tert-butyl-3-{[éthyl-(2-méthyl-pentyl)-amino]-méthyl}-imidazo[1,2-a]pyridin-7-yl)-N-hydroxy-acrylamide |
| 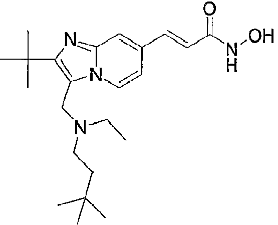 | 3-(2-tert-butyl-3-{[(3,3-diméthyl-butyl)-éthyl-amino]-méthyl}-imidazo[1,2-a]pyridin-7-yl)-N-hydroxy-acrylamide |
| 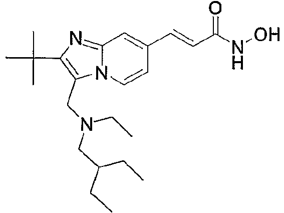 | 3-(2-tert-butyl-3-{[éthyl-(2-éthyl-butyl)-amino]-méthyl}-imidazo[1,2-a]pyridin-7-yl)-N-hydroxy-acrylamide |
| 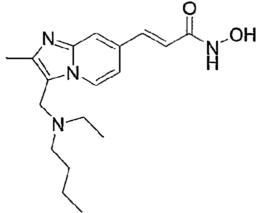 | 3-{3-[(butyl-éthyl-amino)-méthyl]-2-méthyl-imidazo[1,2-a]pyridin-7-yl}-N-hydroxy-acrylamide |
| 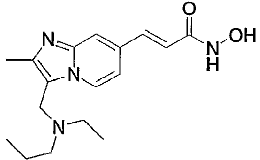 | 3-{3-[(éthyl-propyl-amino)-méthyl]-2-méthyl-imidazo[1,2-a]pyridin-7-yl}-N-hydroxy-acrylamide |
| 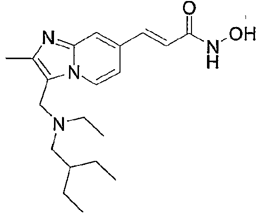 | 3-(3-{[éthyl-(2-éthyl-butyl)-amino]-méthyl}-2-méthyl-imidazo [1,2-a]pyridin-7-yl)-N-hydroxy-acrylamide |
| 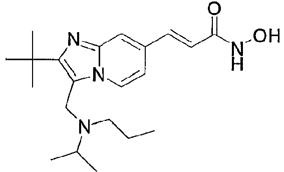 | 3-{2-tert-butyl-3-[(isopropyl-propyl-amino)-méthyl]-imidazo[1,2-a]pyridin-7-yl}-N-hydroxy-acrylamide |
| 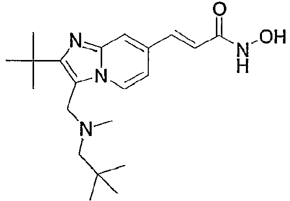 | 3-(2-tert-butyl-3-{[(2,2-diméthyl-propyl)-méthyl-amino]-méthyl}-imidazo[1,2-a]pyridin-7-yl)-N-hydroxy-acrylamide |
| 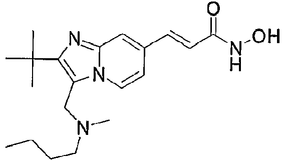 | 3-{2-tert-butyl-3-[(butyl-méthyl-amino)-méthyl]-imidazo[1,2-a]pyridin-7-yl}-N-hydroxy-acrylamide |
| 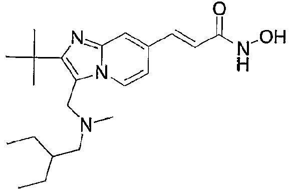 | 3-(2-tert-butyl-3-{[(2-éthyl-butyl)-méthyl-amino]-méthyl}-imidazo[1,2-a]pyridin-7-yl)-N-hydroxy-acrylamide |
| 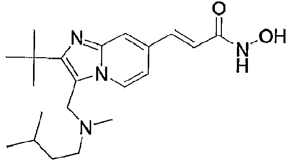 | 3-(2-tert-butyl-3-{[méthyl-(3-méthyl-butyl)-amino]-méthyl}-imidazo[1,2-a]pyridin-7-yl)-N-hydroxy-acrylamide |
| 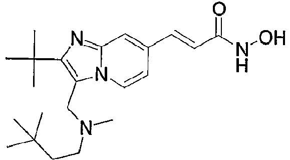 | 3-(2-tert-butyl-3-{[(3,3-diméthyl-butyl)-méthyl-amino] -méthyl}1-imidazo[1,2-a]pyudin-7-yl)-N-hydroxy-acrylamide |
| 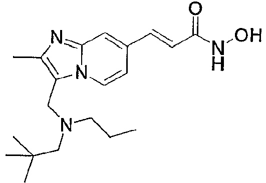 | 3-(3- {[(2,2-diméthyl-propyl)-propyl-amino]-méthyl}-2-méthyl-imidazo[1,2-a]pyridin-7-yl)-N-hydroxy-acrylamide |
| 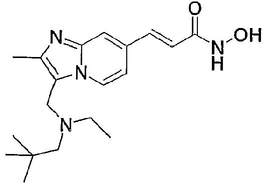 | 3-(3- {[(2,2-diméthyl-propyl)-éthyl-amino]-méthyl}-2-méthyl-imidazo[1,2-a]pyridin-7-yl)-N-hydroxy-acrylamide |
| 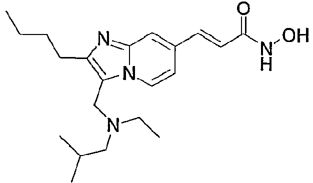 | 3-{2-butyl-3-[(éthyl-isobutyl-amino)-méthyl]-imidazo[1,2-a]pyridin-7-yl}-N-hydroxy-acrylamide |
| 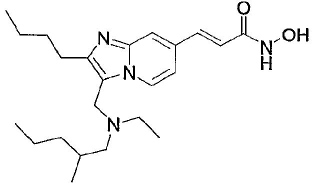 | 3-(2-butyl-3-[éthyl-(2-méthyl-pentyl)-amino]-méthyl}-imidazo[1,2-a]pyridin-7-yl)-N-hydroxy-acrylamide |
| 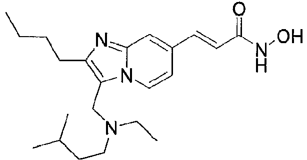 | 3-(2-butyl-3- [éthyl-(3-méthyl-butyl)-amino]-méthyl}-imidazo[1,2-a]pyridin-7-yl)-N-hydroxy-acrylamide |
| 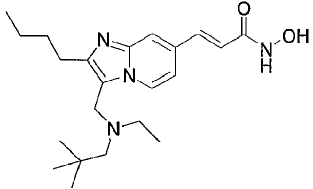 | 3-(2-butyl-3-{[(2,2-diméthyl-propyl)-éthyl-amino]-méthyl}-imidazo[1,2-a]pyridin-7-yl)-N-hydroxy-acrylamide |
| 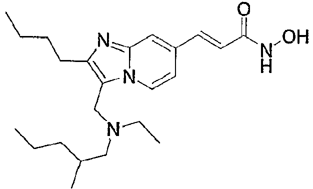 | 3-(2-butyl-3- {[éthyl-(2-méthyl-pentyl)-amino]-méthyl}-imidazo[1,2-a]pyridin-7-yl)-N-hydroxy-acrylamide |
| 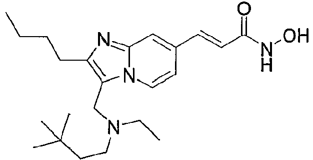 | 3-(2-butyl-3-{[(3,3-diméthyl-butyl)-éthyl-amino]-méthyl}-imidazo[[1,2-a]pyridin-7-yl)-N-hydroxy-acrylamide |
| 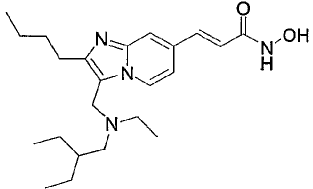 | 3-(2-butyl-3-{[éthyl-(2-éthyl-butyl)-amino]-méthyl}-imidazo[1,2-a]pyridin-7-yl)-N-hydroxy-acrylamide |
| 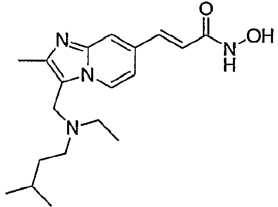 | 3-(3-{[éthyl-(3-méthyl-butyl)-amino]-méthyl}-2-méthyl-imidazo[1,2-a]pyridin-7-yl)-N-hydroxy-acrylamide |
| 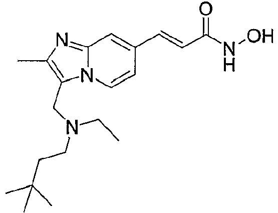 | 3-(3-{[éthyl-(3,3-diméthyl-butyl)-amino]-méthyl}-2-méthyl-imidazo[1,2-a]pyridin-7-yl)-N-hydroxy-acrylamide |
| 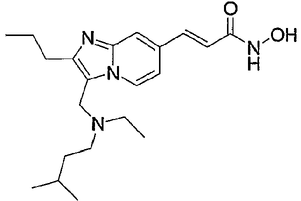 | 3-(3-{[éthyl-(3-méthyl-butyl)-amino]-méthyl}-2-propyl-imidazo[1,2-a]pyridin-7-yl)-N-hydroxy-acrylamide |
| 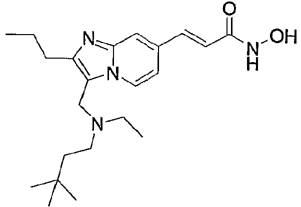 | 3-(3-{[(3,3-diméthyl-butyl)-éthyl-amino]-méthyl}-2-propyl-imidazo[1,2-a]pyridin-7-yl)-N-hydroxy-acrylamide |
| 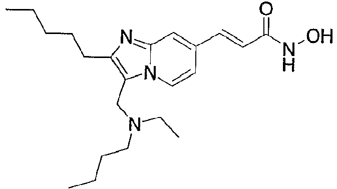 | 3-{3-[(butyl-éthyl-amino)-méthyl]-2-pentyl-imidazo[1,2-a]pyridin-7-yl}-N-hydroxy-acrylamide |
| 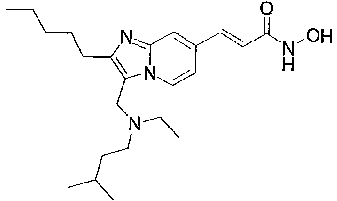 | 3-(3-{[éthyl-(3-méthyl-butyl)-amino]-méthyl}-2-pentyl-imidazo[1,2-a]pyridin-7-yl)-N-hydroxy-acrylamide |
| 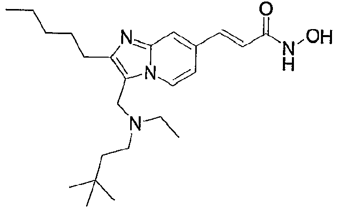 | 3-(3-{[(3,3-diméthyl-butyl)-éthyl-amino]-méthyl}-2-pentyl-imidazo[1,2-a]pyridin-7-yl)-N-hydroxy-acrylamide |
| 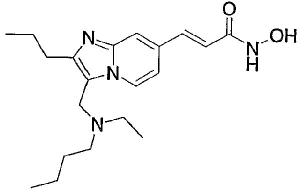 | 3-{3-[(butyl-éthyl-amino)-méthyl]-2-propyl-imidazo[1,2-a]pyridin-7-yl}-N-hydroxy-acrylamide |
| 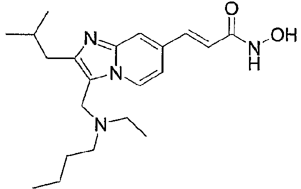 | 3-{3-[(butyl-éthyl-amino)-méthyl]-2-isobutyl-imidazo[1,2-a]pyridin-7-yl}-N-hydroxy-acrylamide |
| 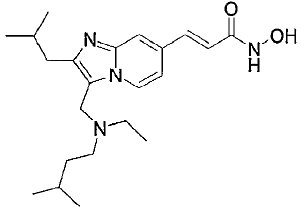 | 3-(3-{[éthyl-(3-méthyl-butyl)-amino]-méthyl}-2-isobutyl-imidazo[1,2-a]pyridin-7-yl)-N-hydroxy-acrylamide |
| 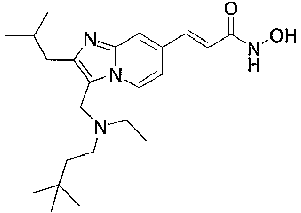 | 3-(3-{[(3,3-diméthyl-butyl)-éthyl-amino]-méthyl}-2-isobutyl-imidazo[1,2-a]pyridin-7-yl)-N-hydroxy-acrylamide |
| 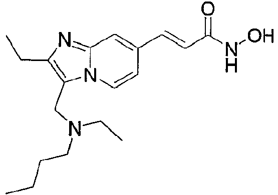 | 3-{3-[(butyl-éthyl-amino)-méthyl]-2-éthyl-imidazo[1,2-a]pyridin-7-yl}-N-hydroxy-acrylamide |
| 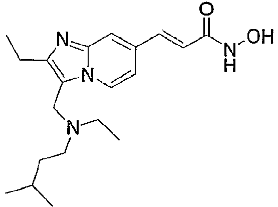 | 3-(2-éthyl-3-{[éthyl-(3-méthyl-butyl)-amino]-méthyl}-imidazo[1,2-a]pyridin-7-yl)-N-hydroxy-acrylamide |
| 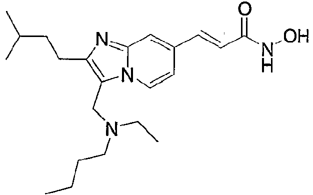 | 3-[3-[(butyl-éthyl-amino)-méthyl]-2-(3-méthyl-butyl)-imidazo[1,2-a]pyridin-7-yl]-N-hydroxy-acrylamide |
| 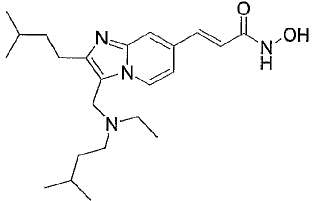 | 3-[3-{[éthyl-(3-méthyl-butyl)-amino]-méthyl}-2-(3-méthyl-butyl)-imidazo[1,2-a]pyridin-7-yl]-N-hydroxy-acrylamide |
| 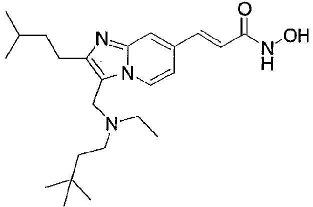 | 3-[3-{[(3,3-diméthyl-butyl)-éthyl-amino]-méthyl}-2-(3-méthyl-butyl)-imidazo[1,2-a]pyridin-7-yl]-N-hydroxy-acrylamide. |

11. Composition pharmaceutique incluant un composé selon l'une quelconque des revendications 1 à 10 et un diluant, excipient ou vecteur pharmaceutiquement acceptable.

12. Composé selon l'une quelconque des revendications 1 à 10 destiné à être utilisé dans le traitement d'un trouble provoqué par, associé à ou accompagné de perturbations de la prolifération cellulaire et/ou de l'angiogenèse.

13. Composé destiné à être utilisé selon la revendication 12, dans lequel le trouble est le cancer.

14. Composé selon l'une quelconque des revendications 1 à 10 destiné à être utilisé dans le traitement d'un trouble qui peut être traité par l'inhibition de l'histone désacétylase.

15. Composé destiné à être utilisé selon la revendication 14, dans lequel le trouble est sélectionné parmi le groupe constitué de troubles prolifératifs (par exemple cancer) ; de maladies neurodégénératives incluant la maladie de Huntington, des maladies à polyglutamine, la maladie de Parkinson, la maladie d'Alzheimer, des attaques, la dégénérescence striatonigrique, la paralysie supranucléaire progressive, la dystonie de torsion, le torticolis spasmodique et la dyskinésie, le tremblement familial, le syndrome de Gilles de la Tourette, la maladie diffuse à corps de Lewy, la maladie de Pick, l'hémorragie intracérébrale, la sclérose latérale primaire, l'atrophie musculaire spinale, la sclérose latérale amyotrophique, la polyneuropathie interstitielle hypertrophique, la rétinite pigmentaire, l'atrophie optique héréditaire, la paraplégie spastique héréditaire, l'ataxie progressive et le syndrome de Shy-Drager ; de maladies métaboliques incluant le diabète de type 2 ; de maladies dégénératives de l'oeil incluant le glaucome, la dégénérescence maculaire liée à l'âge, la dégénérescence myopique maculaire, le glaucome rubéotique, la kératite interstitielle, la rétinopathie diabétique, l'anomalie de Peters, la dégénérescence rétinienne, la rétinopathie de la membrane cellophane ; la dystrophie de Cogan ; la dystrophie cornéenne ; la néovascularisation de l'iris (rubéose) ; la néovascularisation de la cornée ; la rétinopathie de prématurité ; l'oedème maculaire ; le trou maculaire ; la membrane épirétinienne ; la blépharite marginale, la myopie, la croissance non maligne de la conjonctive ; de maladies inflammatoires et/ou de troubles du système immunitaire incluant la polyarthrite rhumatoïde (PR), l'ostéoarthrite, l'arthrite juvénile chronique, la maladie de greffe contre hôte, le psoriasis, l'asthme, la spondylarthropathie, la maladie de Crohn, la maladie du côlon inflammatoire, la rectocolite hémorragique, l'hépatite alcoolique, le diabète, le syndrome de Sjogren, la sclérose en plaques, la spondylarthrite ankylosante, la glomérulopathie membraneuse, la douleur d'origine discale, le lupus érythémateux systémique, la dermatite de contact allergique ; une maladie impliquant l'angiogenèse incluant le cancer, le psoriasis, la polyarthrite rhumatoïde ; de troubles psychologiques incluant la maladie bipolaire, la schizophrénie, la dépression et la démence ; de maladies cardiovasculaires incluant l'insuffisance cardiaque, la resténose, l'hypertrophie cardiaque et l'artériosclérose ; de maladies fibrotiques incluant la fibrose du foie, la fibrose du poumon, la mucoviscidose et l'angiofibrome ; de maladies infectieuses incluant des infections fongiques telles que Candida Albicans, des infections bactériennes, des infections virales, telles que Herpès Simplex, des infections protozoaires, telles que la malaria, une infection à Leishmania, une infection à Trypanosoma brucei, la toxoplasmose et la coccidiose et de troubles hématopoïétiques incluant la thalassémie, l'anémie et la drépanocytose.

16. Composé destiné à être utilisé selon la revendication 13, dans lequel le cancer est sélectionné parmi le groupe constitué d'une malignité hématologique et d'une tumeur solide.

17. Composé destiné à être utilisé selon la revendication 13, dans lequel le cancer est sélectionné parmi un groupe constitué d'un lymphome à lymphocytes B, lymphome à lymphocytes T, leucémie, cancer du sein, cancer du poumon, cancer ovarien, cancer de la prostate, cancer de la tête et du cou, cancer rénal, cancer gastrique, cancer du côlon, cancer pancréatique et cancer du cerveau.
